# EUROPEAN PATENT APPLICATION

(11) **EP 3 326 630 A2**
(43) Date of publication of application: **30.05.2018**
(21) Application number: 17191332.0
(22) Date of filing: 05.05.2008
(51) Int. Cl.: A61K 31/436, A61K 31/337, A61P 9/14

(54) **METHODS AND COMPOSITIONS FOR TREATING PULMONARY HYPERTENSION**

(30) Priority: 03.05.2007 US 927729 P
(62) Divisional of application: 08767585.6
(71) Applicant: Abraxis BioScience, LLC, Summit, NJ 07901 (US)
(72) Inventor: DESAI, Neil, P., Pacific Palisades, CA 90272 (US); SOON-SHIONG, Patrick, Culver City, CA 90232 (US)
(74) Representative: Jones Day

(57) **Abstract**

The present invention relates to a composition comprising nanoparticles that comprise rapamycin or a derivative thereof and a carrier protein, for use in a method of treating pulmonary hypertension in an individual. Said method may further comprise administering a composition comprising nanoparticles that comprise a taxane or a derivative thereof and a carrier protein. The present invention also relates to unit dosage forms of the compositions for use of the invention.

## Description

### CROSS-REFERENCE TO RELATED APPLICATIONS

This application claims priority benefit to the provisional application 60/927,729, filed on May 3, 2007, the contents of which are incorporated by reference herein in its entirety.

### TECHNICAL FIELD

This application pertains to methods and compositions for treating, stabilizing, preventing, and/or delaying pulmonary hypertension using nanoparticles that comprise a taxane (e.g., paclitaxel) or a derivative thereof and/or nanoparticles that comprise rapamycin or a derivative thereof.

### BACKGROUND

Pulmonary hypertension (PH) is a syndrome characterized by increased pulmonary artery pressure. PH is defined hemodynamically as a systolic pulmonary artery pressure greater than 30 mm Hg or evaluation of mean pulmonary artery pressure greater than 25 mm Hg. *See* Zaiman et al., Am. J. Respir. Cell Mol. Biol. 33:425-31 (2005). Further, PH, as a result of the increased pressure, damages both the large and small pulmonary arteries. The walls of the smallest blood vessels thicken and are no longer able to transfer oxygen and carbon dioxide normally between the blood and the lungs. In time, pulmonary hypertension leads to thickening of the pulmonary arteries and narrowing of the passageways through which blood flows. Once pulmonary hypertension develops, the right side of the heart works harder to compensate; however, the increased effort causes it to become enlarged and thickened. Proliferation of smooth muscle and endothelial cells which normally exist in a quiescent state leads to remodeling of the vessels with obliteration of the lumen of the pulmonary vasculature. This causes a progressive rise in pulmonary pressures as blood is pumped through decreased lumen area. The enlarged right ventricle places a person at risk for pulmonary embolism because blood tends to pool in the ventricle and in the legs. If clots form in the pooled blood, they may eventually travel and lodge in the lungs with disastrous consequences. The progressive rise in pressure also places an additional workload on the right ventricle which eventually fails and leads to premature death in these patients.

Various pathologic changes occur in pulmonary arteries as a result of PH. Persistent vasoconstriction and structural remodeling of the pulmonary vessels are cardinal features of PH. Pulmonary vascular smooth muscle cells undergo a phenotypic switch from contracticle normal phenotype to a synthetic phenotype leading to cell growth and matrix deposition. Histological examination of tissue samples from patients with pulmonary hypertension shows intimal thickening, as well as smooth muscle cell hypertrophy, especially for those vessels <100 µm diameter. Further, abnormal smooth muscle cells often overexpress endotheline and serotonin transporters, which likely play a role in the development of PH.

The most common symptom of pulmonary hypertension initially is shortness of breath upon exertion. Some people feel light-headed or fatigued upon exertion, and an angina-like chest pain is common. Because body tissues are not receiving enough oxygen, general weakness is another problem. Other symptoms, such as coughing and wheezing, may be caused by an underlying lung disease. Edema, particularly of the legs, may occur because fluid may leak out of the veins and into the tissues, signaling that cor pulmonale has developed. Some people with pulmonary hypertension have connective tissue disorders, especially scleroderma. When people have both conditions, pulmonary hypertension and connective tissue disorders, Raynaud's phenomenon often develops before symptoms of pulmonary hypertension appear, sometimes as long as years earlier.

Treatment of some types of pulmonary hypertension is often directed at the underlying lung disease. Currently, the treatment options available for those suffering from PH target cellular dysfunction that leads to constriction of the vasculature. Therapies such as prostanoids, phosphodiesterase-5 inhibitors and endothelin receptor antagonists primarily work by causing dilation of the pulmonary vessels. Vasodilators, such as calcium channel blockers, nitric oxide, and prostacyclin, are often helpful for pulmonary hypertension associated with scleroderma, chronic liver disease, and HIV infection. In contrast, these drugs have not been proven effective for people with pulmonary hypertension due to an underlying lung disease. For most people with pulmonary hypertension due to an unknown cause, vasodilators, such as prostacyclin, drastically reduce blood pressure in the pulmonary arteries. Prostacyclin given intravenously through a catheter surgically implanted in the skin improves the quality of life, increases survival, and reduces the urgency of lung transplantation. Unfortunately, many patients respond poorly to these therapies or stop responding to them over time. The only remaining option at that point in time is a single or double lung transplantation to treat PH. Although there is some evidence that available therapies have secondary effects on vascular remodeling, there are currently no therapies that target abnormal cell proliferation in PAH.

Many anti-proliferative agents are dissolved in a solvent/surfactant which produces hypersensitivity reactions. Great efforts have been invested on the development of water soluble prodrugs and derivatives of anti-proliferative agents with higher hydrophilic groups to enhance water solubility and thus obviate the need for potentially toxic solvents/surfactants. Another approach to address the problem associated with the poor water solubility of anti-proliferative agents is the development of various formulations such as nanoparticles, oil-in-water emulsions, and liposomes. For example, Abraxane® is a nanoparticle composition of paclitaxel and albumin. Nanoparticle compositions of substantially poorly water soluble drugs and uses thereof have been disclosed, for example, in U.S. Patent Nos. 5,916,596; 6,096,331; 6,749,868; and 6,537,579; U.S. Patent Appln. No. 09/847,945; and PCT Application Pub. Nos. WO98/14174, WO99/00113, WO07/027941 and WO07/027819.

Due to the limitations of current treatments for pulmonary hypertension, there remains a significant interest in and need for additional or alternative therapies for treating, stabilizing, preventing, and/or delaying pulmonary hypertension. Preferably, the treatments overcome the shortcomings of current drug and transplant treatments, such as hypersensitivity reactions due to the solvent/surfactant in which drugs are dissolved.

The specification is most thoroughly understood in light of the references cited herein. The disclosures of all publications, patents, patent applications, and published patent applications referred to herein are each hereby incorporated herein by reference in their entireties.

### BRIEF SUMMARY OF THE INVENTION

The present invention provides methods for the treatment of pulmonary hypertension using nanoparticles that comprise rapamycin or a derivative thereof or nanoparticles that comprise a taxane (*e.g*., paclitaxel) or a derivative thereof. Accordingly, the invention in some variations provides a method of treating pulmonary hypertension in an individual by administering to the individual (*e.g*., a human) an effective amount of a composition comprising nanoparticles that comprise rapamycin or a derivative thereof and a carrier protein or nanoparticles that comprise a taxane (*e.g*., paclitaxel) or a derivative thereof and a carrier protein. In some variations, the invention provides a method of treating pulmonary hypertension in an individual by administering to the individual (*e.g*., a human) an effective amount of a composition comprising nanoparticles that comprise rapamycin or a derivative thereof and a carrier protein. In some variations, the invention provides a method of treating pulmonary hypertension in an individual by administering to the individual (*e.g*., a human) an effective amount of a composition comprising nanoparticles that comprise a taxane (*e.g*., paclitaxel) or a derivative thereof and a carrier protein.

In some variations, the invention provides a method of treating pulmonary hypertension in an individual by administering to the individual an effective amount of a composition comprising nanoparticles that comprise rapamycin and albumin. In some variations, the invention provides a method of treating pulmonary hypertension in an individual by administering to the individual an effective amount of a composition comprising nanoparticles that comprise paclitaxel and albumin. In some variations, the pulmonary hypertension is pulmonary arterial hypertension (PAH). In some variations, the PAH is idiopathic PAH. In some variations, the PAH is familial PAH. In some variations, the PAH is associated with persistent pulmonary hypertension of a newborn. In some variations, the PAH is associated with pulmonary veno-occusive disease. In some variations, the PAH is associated with pulmonary capillary hemangiomatosis. In some variations, the pulmonary hypertension is pulmonary venous hypertension. In some variations, the pulmonary hypertension is pulmonary hypertension associated with disorders of the respiratory system and/or hypoxia. In some variations, the pulmonary hypertension is pulmonary hypertension due to chronic thrombotic and/or embolic disease. In some variations, the pulmonary hypertension is miscellaneous pulmonary hypertension. In some variations, the miscellaneous pulmonary hypertension is associated with sarcoidosis, eosiniphilic granuloma, histicytosis X, lymphangiolomyiomatosis, or compression of pulmonary vessels (*e.g*., adenopath, tumor, or fibrosing medianstinitis). In some variations, the pulmonary hypertension is associated with chronic obstructive pulmonary disease (COPD). In some variations, the pulmonary hypertension is associated with pulmonary fibrosis. In some variations, the pulmonary hypertension is early stage pulmonary hypertension or advanced pulmonary hypertension. In some variations, one or more symptoms of the pulmonary hypertension are ameliorated. In some variations, the pulmonary hypertension is delayed. In some variations, the methods of treatment provided herein reduce pulmonary pressure. In some variations, the methods of treatment provided herein inhibit and/or reduce abnormal cell proliferation in the pulmonary artery.

In some variations, the invention provides a method of preventing pulmonary hypertension in an individual by administering to the individual (*e.g*., a human) an effective amount of a composition comprising nanoparticles that comprise rapamycin or a derivative thereof and a carrier protein or nanoparticles that comprise a taxane (*e.g*., paclitaxel) or a derivative thereof and a carrier protein. In some variations, the invention provides a method of preventing pulmonary hypertension in an individual by administering to the individual an effective amount of a composition comprising nanoparticles that comprise rapamycin and albumin. In some variations, the invention provides a method of preventing pulmonary hypertension in an individual by administering to the individual an effective amount of a composition comprising nanoparticles that comprise paclitaxel and albumin.

In some variations, the amount of the rapamycin or derivative thereof or the taxane (*e.g*., paclitaxel) or a derivative thereof in the effective amount of the composition is in the range of about 5 mg to about 500 mg, such as about 30 mg to about 300 mg or about 50 mg to about 200 mg. In some variations, rapamycin or derivative thereof or a taxane (*e.g*., paclitaxel) or a derivative thereof in the effective amount of the composition is about 5 to about 300 mg/m². In some variations, rapamycin or derivative thereof or a taxane (*e.g*., paclitaxel) or a derivative thereof in the effective amount of the composition is about 30 to about 100 mg/m². In some variations, the rapamycin or derivative thereof or the taxane (*e.g*., paclitaxel) or a derivative thereof is administered parenterally (*e.g*., intravenously). In some variations, the rapamycin or derivative thereof or the taxane (*e.g*., paclitaxel) or a derivative thereof is administered by inhalation. In some variations, the rapamycin or derivative thereof or the taxane (*e.g*., paclitaxel) or a derivative thereof is the only pharmaceutically active agent for the treatment of pulmonary hypertension that is administered to the individual. In some variations, the composition comprises more than about 50% of the rapamycin or derivative thereof or the taxane (*e.g*., paclitaxel) or a derivative thereof in nanoparticle form. In some variations, the composition comprises more than about 90% of the rapamycin or derivative thereof or the taxane (*e.g*., paclitaxel) or a derivative thereof in nanoparticle form. In some variations, the carrier protein is albumin, such as human serum albumin. In some variations, the average diameter of the nanoparticles in the composition is no greater than about 200 nm. In some variations, the weight ratio of the carrier protein to the rapamycin or derivative thereof or a taxane (*e.g*., paclitaxel) or a derivative thereof in the nanoparticles is less than about 18:1. In some variations, the nanoparticles in the composition have a solid core. In some variations, the nanoparticles in the composition have a core that is not aqueous (*i.e.*, other than aqueous core). In some variations, the nanoparticles in the composition are substantially free of lipids. In some variations, the nanoparticles in the composition are free of lipids. In some variations, the nanoparticles of the composition lack a polymeric matrix. In some variations, the nanoparticles of the composition are filter sterilizable. In some variations, the nanoparticles of the composition comprise at least one cross-linked (*e.g*., disulfide bonds) carrier protein. In some variations, the nanoparticles of the composition comprise at least ten-percent of carrier protein that is cross-linked (*e.g*., disulfide bonds).

The invention also provides a method of treating pulmonary hypertension in an individual (*e.g*., human) comprising administering to the individual an effective amount of a composition comprising nanoparticles that comprise rapamycin or a derivative thereof and a carrier protein (*e.g*., albumin) in combination with one or more other therapeutic agents and/or therapies. In some variations, the present invention provides a method of treating pulmonary hypertension in an individual (*e.g*., human) comprising administering to the individual an effective amount of a composition comprising nanoparticles that comprise rapamycin and an albumin in combination with one or more other therapeutic agents and/or therapies. In some variations, the other therapeutic agent is a taxane or a derivative thereof. In some variations, the taxane or a derivative thereof is provided in a composition comprising nanoparticles that comprise a taxane or a derivative thereof and a carrier protein (*e.g*., albumin). In some variations, the taxane or a derivative thereof is paclitaxel. In some variations, the paclitaxel is provided in a composition comprising nanoparticles that comprise paclitaxel and albumin.

The invention further provides a method of treating pulmonary hypertension in an individual (*e.g*., human) comprising administering to the individual an effective amount of a composition comprising nanoparticles that comprise a taxane or a derivative thereof and a carrier protein (*e.g*., albumin) in combination with one or more other therapeutic agents and/or therapies. In some variations, the present invention provides a method of treating pulmonary hypertension in an individual (*e.g*., human) comprising administering to the individual an effective amount of a composition comprising nanoparticles that comprise paclitaxel and an albumin in combination with one or more other therapeutic agents and/or therapies. In some variations, the other therapeutic agent is rapamycin or a derivative thereof. In some variations, the rapamycin or a derivative thereof is provided in a composition comprising nanoparticles that comprise rapamycin or a derivative thereof and a carrier protein (*e.g*., albumin). In some variations, the rapamycin or derivative thereof is rapamycin. In some variations, the rapamycin is provided in a composition comprising nanoparticles that comprise rapamycin and albumin.

In some variations of any of the above methods of combination therapy, the pulmonary hypertension is pulmonary arterial hypertension (PAH). In some variations, the PAH is idiopathic PAH. In some variations, the PAH is familial PAH. In some variations, the PAH is associated with persistent pulmonary hypertension of a newborn. In some variations, the PAH is associated with pulmonary veno-occusive disease. In some variations, the PAH is associated with pulmonary capillary hemangiomatosis. In some variations, the pulmonary hypertension is pulmonary venous hypertension. In some variations, the pulmonary hypertension is pulmonary hypertension associated with disorders of the respiratory system and/or hypoxia. In some variations, the pulmonary hypertension is pulmonary hypertension due to chronic thrombotic and/or embolic disease. In some variations, the pulmonary hypertension is miscellaneous pulmonary hypertension. In some variations, the miscellaneous pulmonary hypertension is associated with sarcoidosis, eosiniphilic granuloma, histicytosis X, lymphangiolomyiomatosis, or compression of pulmonary vessels (*e.g*., adenopath, tumor, or fibrosing medianstinitis). In some variations, the pulmonary hypertension is associated with chronic obstructive pulmonary disease (COPD). In some variations, the pulmonary hypertension is associated with pulmonary fibrosis. In some variations, the pulmonary hypertension is early stage pulmonary hypertension or advanced pulmonary hypertension. In some variations, one or more symptoms of the pulmonary hypertension are ameliorated. In some variations, the pulmonary hypertension is delayed. In some variations, the pulmonary hypertension is prevented. In some variations, the methods of treatment provided herein reduce pulmonary pressure. In some variations, the methods of treatment provided herein inhibit and/or reduce abnormal cell proliferation in the pulmonary artery.

In some variations of any of the above methods of combination therapy, the amount of the rapamycin or derivative thereof or the taxane (*e.g*., paclitaxel) or a derivative thereof in the effective amount of the composition is in the range of about 5 mg to about 500 mg, such as about 30 mg to about 300 mg or about 50 mg to about 200 mg. In some variations, rapamycin or derivative thereof or a taxane (*e.g*., paclitaxel) or a derivative thereof in the effective amount of the composition is about 5 to about 300 mg/m². In some variations, rapamycin or derivative thereof or a taxane (*e.g*., paclitaxel) or a derivative thereof in the effective amount of the composition is about 30 to about 100 mg/m². In some variations, the rapamycin or derivative thereof or the taxane (*e.g*., paclitaxel) or a derivative thereof is administered parenterally (*e.g*., intravenously). In some variations, the rapamycin or derivative thereof or the taxane (*e.g*., paclitaxel) or a derivative thereof is administered by inhalation. In some variations, the composition comprises more than about 50% of the rapamycin or derivative thereof or the taxane (*e.g*., paclitaxel) or a derivative thereof in nanoparticle form. In some variations, the composition comprises more than about 90% of the rapamycin or derivative thereof or the taxane (*e.g*., paclitaxel) or a derivative thereof in nanoparticle form. In some variations, the carrier protein is albumin, such as human serum albumin. In some variations, the average diameter of the nanoparticles in the composition is no greater than about 200 nm. In some variations, the weight ratio of the carrier protein to the rapamycin or derivative thereof or the taxane (*e.g*., paclitaxel) or a derivative thereof in the nanoparticles is less than about 18:1. In some variations, the nanoparticles in the composition have a solid core. In some variations, the nanoparticles in the composition have a core that is not aqueous (*i.e.*, other than aqueous core). In some variation, the nanoparticles of the composition are substantially free of lipids. In some variations, the nanoparticles of the composition are free of lipids. In some variations, the nanoparticles of the composition lack a polymeric matrix. In some variations, the nanoparticles of the composition are filter sterilizable. In some variations, the nanoparticles of the composition comprise at least one cross-linked carrier protein. In some variations, the nanoparticles of the composition comprise at least ten percent of carrier protein that is cross-linked.

In some variations of the methods described herein, the nanoparticles comprise taxane, which may be paclitaxel. In some variations, the nanoparticles comprise rapamycin.

The invention also provides pharmaceutical compositions such as unit dosage forms that are useful for treating pulmonary hypertension. Accordingly, the invention in some variations provides a pharmaceutical composition (*e.g*., a unit dosage form of a pharmaceutical composition) that includes nanoparticles that comprise rapamycin or a derivative thereof and a carrier protein (*e.g.,* albumin) or nanoparticles that comprise a taxane (*e.g.,* paclitaxel) or a derivative thereof and a carrier protein (*e.g*., albumin). In some variations, the composition also includes a pharmaceutically acceptable carrier. In some variations, the pulmonary hypertension is pulmonary arterial hypertension (PAH). In some variations, the PAH is idiopathic PAH. In some variations, the PAH is familial PAH. In some variations, the PAH is associated with persistent pulmonary hypertension of a newborn. In some variations, the PAH is associated with pulmonary veno-occusive disease. In some variations, the PAH is associated with pulmonary capillary hemangiomatosis. In some variations, the pulmonary hypertension is pulmonary venous hypertension. In some variations, the pulmonary hypertension is pulmonary hypertension associated with disorders of the respiratory system and/or hypoxia. In some variations, the pulmonary hypertension is pulmonary hypertension due to chronic thrombotic and/or embolic disease. In some variations, the pulmonary hypertension is miscellaneous pulmonary hypertension. In some variations, the miscellaneous pulmonary hypertension is associated with sarcoidosis, eosiniphilic granuloma, histicytosis X, lymphangiolomyiomatosis, or compression of pulmonary vessels (*e.g*., adenopath, tumor, or fibrosing medianstinitis). In some variations, the pulmonary hypertension is associated with chronic obstructive pulmonary disease (COPD). In some variations, the pulmonary hypertension is associated with pulmonary fibrosis. In various variations, the pulmonary hypertension is early stage pulmonary hypertension or advanced pulmonary hypertension. In some variations, one or more symptoms of the pulmonary hypertension are ameliorated. In some variations, the pulmonary hypertension is delayed or prevented.

In some variations, the amount of the rapamycin or derivatives thereof or the taxane (*e.g.*, paclitaxel) or a derivative thereof in the composition (*e.g.*, a dose or a unit dosage form) is in the range of about 5 mg to about 500 mg, such as about 30 mg to about 300 mg or about 50 mg to about 200 mg. In some variations, the carrier is suitable for parenteral administration (*e.g*., intravenously). In some variations, the carrier is suitable for administration by inhalation. In some variations, the rapamycin or derivative thereof or the taxane (*e.g*., paclitaxel) or a derivative thereof is the only pharmaceutically active agent for the treatment of pulmonary hypertension that is contained in the composition. In some variations, the composition comprises rapamycin. In some variations, the composition comprises paclitaxel. In some variations, the composition comprises both rapamycin and paclitaxel. In some variations, the composition comprises more than about 50% of the rapamycin or derivative thereof or the taxane (*e.g*., paclitaxel) or a derivative thereof in nanoparticle form. In some variations, the carrier protein is albumin, such as human serum albumin. In some variations, the average diameter of the nanoparticles in the composition is no greater than about 200 nm. In some variations, the weight ratio of the carrier protein to the rapamycin or derivative thereof or the taxane (*e.g*., paclitaxel) or a derivative thereof in the nanoparticles is less than about 18:1.

The invention also contemplates kits for application in the uses described herein comprising any of the compositions described herein. In yet another aspect, the invention includes a kit with (i) a composition comprising nanoparticles that comprise rapamycin or a derivative thereof and a carrier protein (*e.g*., albumin) or nanoparticles that comprise a taxane (*e.g.*, paclitaxel) or a derivative thereof and a carrier protein (*e.g.*, albumin) and (ii) instructions for use in treating pulmonary hypertension. In some variations, the pulmonary hypertension is pulmonary arterial hypertension (PAH). In some variations, the PAH is idiopathic PAH. In some variations, the PAH is familial PAH. In some variations, the PAH is associated with persistent pulmonary hypertension of a newborn. In some variations, the PAH is associated with pulmonary veno-occusive disease. In some variations, the PAH is associated with pulmonary capillary hemangiomatosis. In some variations, the pulmonary hypertension is pulmonary venous hypertension. In some variations, the pulmonary hypertension is pulmonary hypertension associated with disorders of the respiratory system and/or hypoxia. In some variations, the pulmonary hypertension is pulmonary hypertension due to chronic thrombotic and/or embolic disease. In some variations, the pulmonary hypertension is miscellaneous pulmonary hypertension. In some variations, the miscellaneous pulmonary hypertension is associated with sarcoidosis, eosiniphilic granuloma, histicytosis X, lymphangiolomyiomatosis, or compression of pulmonary vessels (*e.g*., adenopath, tumor, or fibrosing medianstinitis). In some variations, the pulmonary hypertension is associated with chronic obstructive pulmonary disease (COPD). In some variations, the pulmonary hypertension is associated with pulmonary fibrosis. In various variations, the pulmonary hypertension is early stage pulmonary hypertension or advanced pulmonary hypertension. In some variations, one or more symptoms of the pulmonary hypertension are ameliorated. In some variations, the pulmonary hypertension is delayed or prevented.

In some variations, the amount of the rapamycin or derivative thereof or the taxane (*e.g*., paclitaxel) or a derivative thereof in the kit is in the range of about 5 mg to about 500 mg, such as about 30 mg to about 300 mg or about 50 mg to about 200 mg. In some variations, the rapamycin or derivative thereof or the taxane (*e.g*., paclitaxel) or a derivative thereof is administered parenterally (*e.g*., intravenously). In some variations, the rapamycin or derivative thereof or the taxane (*e.g*., paclitaxel) or a derivative thereof is administered by inhalation. In some variations, the rapamycin or derivative thereof or the taxane (*e.g*., paclitaxel) or a derivative thereof is the only pharmaceutically active agent for the treatment of pulmonary hypertension that is contained in the kit. In some variations, the kit comprises rapamycin. In some variations, the kit comprises paclitaxel. In some variations the kit comprises both rapamycin and paclitaxel. In some variations, the composition comprises more than about 50% of the rapamycin or derivative thereof or the taxane (*e.g*., paclitaxel) or a derivative thereof in nanoparticle form. In some variations, the carrier protein is albumin, such as human serum albumin. In some variations, the average diameter of the nanoparticles in the composition is no greater than about 200 nm. In some variations, the weight ratio of the carrier protein to the rapamycin or derivative thereof or the taxane (*e.g*., paclitaxel) or a derivative thereof in the nanoparticles is less than about 18:1.

The invention also provides any of the compositions described (*e.g*., a composition comprising nanoparticles that comprise rapamycin or a derivative thereof and a carrier protein (*e.g.,* albumin) or nanoparticles that comprise a taxane (*e.g.,* paclitaxel) or a derivative thereof and a carrier protein (*e.g*., albumin) for any use described herein whether in the context of use as a medicament and/or use for manufacture of a medicament. Also provided are unit dosage forms of compositions described herein, articles of manufacture comprising the inventive compositions or unit dosage forms in suitable packaging (*e.g*., vials or vessels including sealed vials or vessels and sterile sealed vials or vessels), and kits comprising the unit dosage forms. The invention also provides methods of making and using these compositions as described herein.

It is to be understood that one, some, or all of the properties of the various variations described herein may be combined to form other variations of the present invention.

### BRIEF DESCRIPTION OF FIGURES

Figure 1 is a table listing the intravenous pharmacokinetic parameters for the albumin-containing nanoparticle formulation of rapamycin (hereinafter referred to as *Nab-*rapamycin).
Figure 2A is a graph of Cmax versus dose, showing linearity for the *Nab-*rapamycin.
Figure 2B is a graph of AUC versus dose, showing linearity for *Nab*-rapamycin.
Figure 2C is a graph of Vss versus dose, showing possible saturable volume of distribution for *Nab*-rapamycin.

### DETAILED DESCRIPTION OF THE INVENTION

The present invention provides methods, compositions, and kits for the treatment or prevention of pulmonary hypertension using nanoparticles that comprise a taxane (*e.g.,* paclitaxel) or a derivative thereof and a carrier protein (such as albumin). The present invention also provides methods, compositions, and kits for the treatment or prevention of pulmonary hypertension using nanoparticles that comprise rapamycin or a derivative thereof and a carrier protein (such as albumin). These compositions can be used to treat, stabilize, prevent, and/or delay pulmonary hypertension.

The nanoparticle formulation of albumin and rapamycin and the nanoparticles formulation of albumin and a taxane (*e.g*., paclitaxel) enhances tissue penetration through albumin receptor (gp60)-mediated binding of the SPARC protein. This increased specificity of *Nab*-rapamycin and *Nab*-paclitaxel may increase the effectiveness of rapamycin and a taxane (*e.g*., paclitaxel) and may allow lower doses of rapamycin and a taxane (*e.g.,* paclitaxel) to be used, which would minimize toxic effects from rapamycin and a taxane (*e.g*., paclitaxel) while still inhibiting, stabilizing, preventing, or delaying pulmonary hypertension. The increased specificity may also reduce toxic side-effects of rapamycin and a taxane (*e.g*., paclitaxel), such as intestinal toxicity that sometimes limits the dose of rapamycin and a taxane (*e.g*., paclitaxel) that can be given to a patient. The nanoparticle formulation of rapamycin and nanoparticles formulation of a taxane (*e.g*., paclitaxel) also increases the solubility of rapamycin and a taxane (*e.g*., paclitaxel) and allows larger doses to be used, if desired.

### Definitions

As used herein, "the composition" or "compositions" includes and is applicable to compositions of the invention. The invention also provides pharmaceutical compositions comprising the components described herein.

Reference to "taxane" herein applies to a taxane or its derivatives and accordingly the invention contemplates and includes all these variations. Reference to "taxane" is to simplify the description and is exemplary. Taxanes include, but are not limited to, compounds that are structurally similar to or are in the same general chemical class such as paclitaxel (*i.e.,* taxol), docetaxel (*i.e.,* taxotere), or ortataxel, and pharmaceutically acceptable salts, derivatives, or analogs of paclitaxel, docetaxel, and ortataxel.

Reference to "rapamycin" herein applies to rapamycin or its derivatives and accordingly the invention contemplates and includes all these variations. Rapamycin is sometimes referred to elsewhere as sirolimus, rapammune, or rapamune. Reference to "rapamycin" is to simplify the description and is exemplary. Derivatives of rapamycin include, but are not limited to, compounds that are structurally similar to rapamycin, or are in the same general chemical class as rapamycin, analogs of rapamycin, or pharmaceutically acceptable salts of rapamycin or its derivatives or analogs. In some variations, rapamycin or a derivative thereof increases basal AKT activity, increases AKT phosphorylation, increases PI3-kinase activity, increases the length of activation of AKT (*e.g*., activation induced by exogenous IGF-1), inhibits serine phosphorylation of IRS-1, inhibits IRS-1 degradation, inhibits or alters CXCR4 subcellular localization, inhibits VEGF secretion, decreases expression of cyclin D2, decreases expression of survivin, inhibits IL-6-induced multiple myeloma cell growth, inhibits pulmonary hypertension cell proliferation, increases apoptosis, increases cell cycle arrest, increases cleavage of poly(ADPribose) polymerase, increases cleavage of caspase-8/caspase-9, alters or inhibits signaling in the phosphatidylinositol 3-kinase/AKT/mTOR and/or cyclin Dl/retinoblastoma pathways, inhibits angiogenesis, and/or inhibits osteoclast formation. In some variations, the derivative of rapamycin retains one or more similar biological, pharmacological, chemical and/or physical properties (including, for example, functionality) as rapamycin. An exemplary rapamycin derivative includes benzoyl rapamycin, such as that disclosed in paragraph [0022] of WO 2006/089207, which is hereby incorporated by reference in its entirety. Other exemplary rapamycin derivatives include WY-090217, AY-22989, NSC-226080, SiiA-9268A, oxaazacyclohentriacontine, temsirolimus (CCI 779 (Wyeth)), everolimus (RAD 001 (Novartis)), pimecrolimus (ASM981), SDZ-RAD, SAR943, ABT-578, AP23573, and Biolimus A9.

Unless clearly indicated otherwise, "an individual" as used herein intends a mammal, including but not limited to a primate, human, bovine, horse, feline, canine, or rodent.

As used herein, "treatment" or "treating" is an approach for obtaining beneficial or desired results including clinical results. For purposes of this invention, beneficial or desired clinical results include, but are not limited to, one or more of the following: decreasing one more symptoms resulting from the disease, diminishing the extent of the disease, stabilizing the disease (*e.g*., preventing or delaying the worsening of the disease), preventing or delaying the occurrence of the disease, delay or slowing the progression of the disease, ameliorating the disease state, decreasing the dose of one or more other medications required to treat the disease, increasing the quality of life, and/or prolonging survival. In some variations, the composition reduces the severity of one or more symptoms associated with pulmonary hypertension by at least about any of 10%, 20%, 30%, 40%, 50%, 60%, 70%, 80%, 90%, 95%, or 100% compared to the corresponding symptom in the same subject prior to treatment or compared to the corresponding symptom in other subjects not receiving the composition. Also encompassed by "treatment" is a reduction of pathological consequence of pulmonary hypertension. The methods of the invention contemplate any one or more of these aspects of treatment.

As used herein, "delaying" the development of pulmonary hypertension means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of pulmonary hypertension is a method that reduces probability of disease development in a given time frame and/or reduces the extent of the disease in a given time frame, when compared to not using the method. Such comparisons are typically based on clinical studies, using a statistically significant number of subjects. Pulmonary hypertension development can be detectable using standard methods, such as routine physical exams, x-ray, electrocardiogram, and echocardiogram. Development may also refer to disease progression that may be initially undetectable and includes occurrence and onset.

As used herein, an "at risk" individual is an individual who is at risk of developing pulmonary hypertension. An individual "at risk" may or may not have detectable disease, and may or may not have displayed detectable disease prior to the treatment methods described herein. "At risk" denotes that an individual has one or more so-called risk factors, which are measurable parameters that correlate with development of pulmonary hypertension, which are described herein. An individual having one or more of these risk factors has a higher probability of developing pulmonary hypertension than an individual without these risk factor(s).

As used herein, by "pharmaceutically active compound" is meant a chemical compound that induces a desired effect, *e.g*., treating, stabilizing, preventing, and/or delaying pulmonary hypertension.

As used herein, by "combination therapy" is meant a first therapy that includes nanoparticles comprising rapamycin or a derivative thereof and a carrier protein and/or nanoparticles comprising a taxane (*e.g*. paclitaxel) or a derivative thereof and a carrier protein in conjunction with a second therapy (*e.g*., surgery or a therapeutic agent) useful for treating, stabilizing, preventing, and/or delaying pulmonary hypertension. Administration in "conjunction with" another compound includes administration in the same or different composition(s), either sequentially, simultaneously, or continuously. In some variations, the combination therapy optionally includes one or more pharmaceutically acceptable carriers or excipients, non-pharmaceutically active compounds, and/or inert substances.

As is understood in the art, an "effective amount" may be in one or more doses, *i.e.,* a single dose or multiple doses may be required to achieve the desired treatment endpoint. An effective amount may be considered in the context of administering one or more therapeutic agents, and a nanoparticle composition (*e.g*., a composition including rapamycin and a carrier protein and/or a composition including a taxane (*e.g*., paclitaxel) and a carrier protein) may be considered to be given in an effective amount if, in conjunction with one or more other agents, a desirable or beneficial result may be or is achieved. The components (*e.g*., the first and second therapies) in a combination therapy of the invention may be administered sequentially, simultaneously, or continuously using the same or different routes of administration for each component. Thus, an effective amount of a combination therapy includes an amount of the first therapy and an amount of the second therapy that when administered sequentially, simultaneously, or continuously produces a desired outcome.

A "therapeutically effective amount" refers to an amount of a composition (*e.g*., nanoparticles that comprise rapamycin or a derivative thereof and a carrier protein or nanoparticles that comprise a taxane (*e.g*., paclitaxel) or a derivative thereof and a carrier protein), a therapy, or a combination therapy sufficient to produce a desired therapeutic outcome (*e.g*., reducing the severity or duration of, stabilizing the severity of, or eliminating one or more symptoms of pulmonary hypertension). For therapeutic use, beneficial or desired results include, *e.g*., decreasing one or more symptoms resulting from the disease (biochemical, histologic and/or behavioral), including its complications and intermediate pathological phenotypes presenting during development of the disease, increasing the quality of life of those suffering from the disease, decreasing the dose of other medications required to treat the disease, enhancing effect of another medication, delaying the progression of the disease, and/or prolonging survival of patients.

A "prophylactically effective amount" refers to an amount of a composition (*e.g*., nanoparticles that comprise rapamycin or a derivative thereof and a carrier protein and/or nanoparticles that comprise a taxane (e.g., paclitaxel) or a derivative thereof and a carrier protein), a therapy, or a combination therapy sufficient to prevent or reduce the severity of one or more future symptoms of pulmonary hypertension when administered to an individual who is susceptible and/or who may develop pulmonary hypertension. For prophylactic use, beneficial or desired results include, *e.g*., results such as eliminating or reducing the risk, lessening the severity of future disease, or delaying the onset of the disease (*e.g*., delaying biochemical, histologic and/or behavioral symptoms of the disease, its complications, and intermediate pathological phenotypes presenting during future development of the disease).

The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention, including, for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art. The proteins described herein may be naturally-occurring, i.e., obtained or derived from a natural source (*e.g*., blood) or synthesized (*e.g*., chemically synthesized or by synthesized by recombinant DNA techniques). Exemplary carrier proteins are described herein.

The term "antimicrobial agent" used herein refers to an agent that is capable of inhibiting (*e.g*., delaying, reducing, slowing, and/or preventing) the growth of one or more microorganisms. Significant microbial growth can be measured or indicated by a number of ways known in the art, such as one or more of the following: (i) microbial growth in a composition that is enough to cause one or more adverse effects to an individual when the composition is administered to the individual; (ii) more than about 10-fold increase in microbial growth over a certain period of time (for example over a 24 hour period) upon extrinsic contamination (*e.g.,* exposure to 10-103 colony forming units at a temperature in the range of 20 to 25 °C). Other indicia of significant microbial growth are described in U.S.S.N. 11/514,030, filed 8/30/2006, which is hereby incorporated by reference in its entirety.

"Sugar" as used herein includes, but is not limited to, monosaccharides, disaccharides, polysaccharides, and derivatives or modifications thereof. Suitable sugars for compositions described herein include, for example, mannitol, sucrose, fructose, lactose, maltose, and trehalose.

As used herein, by "pharmaceutically acceptable" or "pharmacologically compatible" is meant a material that is not biologically or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

As used herein, reference to "not" a value or parameter generally means and describes "other than" a value or parameter. For example, if a taxane is not administered, it means an agent other than a taxane is administered.

Reference to "about" a value or parameter herein includes (and describes) variations that are directed to that value or parameter per se. For example, description referring to "about X" includes description of "X".'

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

### Methods of Treating Pulmonary Hypertension

The invention provides a method of treating pulmonary hypertension in an individual (*e.g*., human) comprising administering to the individual an effective amount of a composition comprising nanoparticles comprising rapamycin or a derivative thereof and a carrier protein (*e.g*., albumin). For example, the present invention provides a method of treating pulmonary hypertension in an individual (*e.g*., human) comprising administering to the individual an effective amount of a composition comprising nanoparticles comprising rapamycin and an albumin.

The invention also provides a method of treating pulmonary hypertension in an individual (*e.g*., human) comprising administering to the individual an effective amount of a composition comprising nanoparticles comprising a taxane or a derivative thereof and a carrier protein (*e.g*., albumin). For example, the present invention provides a method of treating pulmonary hypertension in an individual (*e.g*., human) comprising administering to the individual an effective amount of a composition comprising nanoparticles comprising paclitaxel and an albumin.

The invention also provides a method of treating pulmonary hypertension in an individual (*e.g*., human) comprising administering to the individual an effective amount of a composition comprising nanoparticles that comprise rapamycin or a derivative thereof and a carrier protein (*e.g.*, albumin) in combination with one or more other therapeutic agents and/or therapies. For example, the present invention provides a method of treating pulmonary hypertension in an individual (*e.g*., human) comprising administering to the individual an effective amount of a composition comprising nanoparticles that comprise rapamycin and an albumin in combination with one or more other therapeutic agents and/or therapies. In some variations, the other therapeutic agent is a taxane or a derivative thereof. In some variations, the taxane or a derivative thereof is a composition comprising nanoparticles that comprise a taxane or a derivative thereof and a carrier protein (*e.g*., albumin). In some variations, the taxane or a derivative thereof is paclitaxel. In some variations, the paclitaxel is a composition comprising nanoparticles that comprise paclitaxel and albumin.

The invention also provides a method of treating pulmonary hypertension in an individual (*e.g*., human) comprising administering to the individual an effective amount of a composition comprising nanoparticles that comprise a taxane or a derivative thereof and a carrier protein (*e.g*., albumin) in combination with one or more other therapeutic agents and/or therapies. For example, the present invention provides a method of treating pulmonary hypertension in an individual (*e.g*., human) comprising administering to the individual an effective amount of a composition that comprise nanoparticles comprising paclitaxel and an albumin in combination with one or more other therapeutic agents and/or therapies. In some variations, the other therapeutic agent is rapamycin or a derivative thereof. In some variations, the rapamycin or a derivative thereof is a composition comprising nanoparticles that comprise rapamycin or a derivative thereof and a carrier protein (*e.g*., albumin). In some variations, the rapamycin or derivative thereof is rapamycin. In some variations, the rapamycin is a composition comprising nanoparticles that comprise rapamycin and albumin.

In some variations of any of the above methods of treatment, the pulmonary hypertension (PH) is pulmonary arterial hypertension (PAH). In some variations, the PAH is idiopathic PAH (formally primary pulmonary hypertension). In some variations, the PAH is sporadic PAH (SPAH). In some variations, SPAH is associated with a mutation in the gene bone morphogenetic protein receptor 2 (BMPR2). In some variations, the PAH is familial PAH (FPAH). In some variations, FPAH is associated with a mutation in a gene in the TGF-β pathway. In some variations, FPAH is associated with a mutation in the gene bone morphogenetic protein receptor 2 (BMPR2). In some variations, FPAH is associated with a mutation in ALK-1. In some variations, the age of onset and severity of FPAH is associated with a polymorphism of the gene serotonin transporter (SERT). In some variations, the SERT polymorphism is SERT long form. In some variations, the SERT polymorphism is SERT short form. In some variations, the PAH is associated with PAH (APAH). In some variations, the APAH is associated with collagen vascular disease (*e.g*., scleroderma or lupus), congenital heart disease, congenital systemic-to-pulmonary shunts, portal hypertension, chronic liver disease, human immunodeficiency virus, drugs and toxins (*e.g.,* diet drugs (*e.g.,* fenfluramine or dexfenfluramine), cocaine, or methamphetamine), hemorrhagic hereditary telangectasis, glycogen storage disease, thyroid disorders, Gaucher's disease, haemoglobinopathies, myeloproliferative disorders, or splenectomy. In some variations, the PAH is associated with persistent pulmonary hypertension of a newborn. In some variations, the PAH is associated with significant venous or capillary involvement. In some variations, the PAH is associated with pulmonary veno-occusive disease (*i.e.,* PVOD). In some variations, the PAH is associated with pulmonary capillary hemangiomatosis (*i*.*e*., PCH).

In some variations of any of the above methods of treatment, the PH is PH associated with left heart disease. In some variations, the PH associated with left heart disease is PH associated with left sided atrial or ventricular heart disease. In some variations, the PH associated with left heart disease is left sided valvular heart disease.

In some variations of any of the above methods of treatment, the PH is PH associated with disorders of the respiratory system and/or hypoxia. In some variations, the PH associated with disorders of the respiratory system and/or hypoxia is associated with chronic obstructive lung disease, interstitial lung disease, sleep disordered breathing, alveolar hypoventilation disorders, chronic exposure to high altitude, or developmental abnormalities.

In some variations of any of the above methods of treatment, the PH is PH due to chronic thrombotic and/or embolic disease. In some variations, the PH due to chronic thrombotic and/or embolic disease is thromboembolic obstruction of proximal pulmonary arteries, thromboembolic obstruction of distal pulmonary arteries, or non-thrombotic pulmonary embolism.

In some variations of any of the above methods of treatment, the PH is miscellaneous PH. In some variations, the miscellaneous PH is associated with sarcoidosis, eosiniphilic granuloma, histicytosis X, lymphangiolomyiomatosis, pulmonary Langerhans cell histiocytosis, or compression of pulmonary vessels (*e.g*., adenopath, tumor, or fibrosing medianstinitis). In some variations, the PH is associated with chronic obstructive pulmonary disease (COPD). In some variations, the PH is associated with pulmonary fibrosis. In some variations, the PH is pulmonary venous hypertension.

In some variations of any of the above methods of treatment, the PAH is class 1, class 2, class 3, or class 4 PH according to the NYHA/WHO classification of functional status of patients with pulmonary hypertension. In some variations, class 1 PAH is characterized by, but not limited to, pulmonary hypertension but without resulting limitation of physical activity, such that ordinary physical activity does not cause dyspnoea or fatigue, chest pain or near syncope. In some variations, class 2 PAH is characterized by, but not limited to, pulmonary hypertension resulting in slight limitation of physical activity, such that patients are comfortable at rest and ordinary physical activity causes undue dyspnoea or fatigue, chest pain or near syncope. In some variations, class 3 PAH is characterized by, but not limited to, pulmonary hypertension resulting in marked limitation of physical activity, such that patients are comfortable at rest and less than ordinary activity causes undue dyspnoea or fatigue, chest pain or near syncope. In some variations, class 4 PAH is characterized by, but not limited to, pulmonary hypertension with inability to carry out any physical activity without symptoms, such that patients manifest signs of right heart failure, dyspnoea and/or fatigue may even be present at rest, and discomfort is increased by any physical activity. In some variations, the individual may be NYHA class II or IV despite three months of stable therapy (*e.g*., prostacylin, phosphodiesterase type 5 inhibitor (sildenafil), endothelin receptor antagonist (ambrisenten), or combinations thereof).

In some variations, any of the methods of treatment provided herein may be used to treat and individual (*e.g*., human) who has been diagnosed with or is suspected of having pulmonary hypertension. In some variations, the individual may be a human who exhibits one or more symptoms associated with pulmonary hypertension. In some variations, the methods of treatment provided herein reduce pulmonary pressure. In some variations, the methods of treatment provided herein inhibit and/or reduce abnormal cell proliferation in the pulmonary artery. In some variations, the abnormal cell proliferation is abnormal cell proliferation of smooth muscle and endothelial cells. In some variations, the methods of treatment provided herein inhibit and/or reduce angiogenesis in the pulmonary artery. In some variations, angiogenesis in the pulmonary artery is inhibited and/or reduced by suppression of production of matrix metalloproteinases. In some variations, the methods of treatment provided herein inhibit and/or reduce inflammation in the pulmonary artery. In some variations, inflammation is inhibited and/or reduced by effecting neutrophil and T cell function. In some variations, the methods of treatment provided herein are effective in reducing symptoms of pulmonary hypertension in an animal model system where pulmonary hypertension is induced by either chronic hypoxia or monocrotaline. In some variations, the individual may have advanced disease or a lesser extent of disease. In some variations, the individual is at an early stage of a pulmonary hypertension. In some variations, the individual is at an advanced stage of pulmonary hypertension. In some variations, the individual has severe pulmonary hypertension. In some variations, the individual does not tolerate the toxicities of paclitaxel at traditional chemotherapeutic doses or formulations. In some variations, the individual may have echocardiographic evidence of right ventricular dysfunction. In some variations, the individual may have a 6-Minute Walk Distance of less than or equal to 380m. In some of the variations, the methods of treatment provided herein, the individual may be a human who is genetically or otherwise predisposed (e.g., at risk) to developing pulmonary hypertension who has or has not been diagnosed with pulmonary hypertension, as described herein. In some variations, these risk factors include, but are not limited to, age, sex, race, diet, history of previous disease, presence of precursor disease, genetic (*e.g*., hereditary) considerations, and environmental exposure. In some variations, the individuals at risk for pulmonary hypertension include, *e.g*., those having relatives who have experienced this disease and those whose risk is determined by analysis of genetic or biochemical markers.

In some variations, any of the method of treatment provided herein may improve one or more indicia of pulmonary hypertension, *e.g.,* pressure in the pulmonary artery (*e.g.,* pulmonary artery systolic and/or diastolic pressure), right atrial pressure, pulmonary capillary wedge pressure, systemic artery systolic and diastolic pressure, calculated systemic and pulmonary vascular resistance, heart rate, chest x-ray, cardiac output, vasoreactivity, 6-Minute Walk Test, MRI of the cardiac muscle, NT-pro-BNP levels, and/or C-reactive protein levels.

The invention provides methods of inhibiting abnormal cell proliferation in a patient having pulmonary hypertension comprising administering to the individual an effective amount of a composition comprising nanoparticles comprising rapamycin or a derivative thereof and a carrier protein (*e.g*., albumin). The invention also provides methods of inhibiting abnormal cell proliferation in a patient having pulmonary hypertension comprising administering to the individual an effective amount of a composition comprising nanoparticles comprising taxane or a derivative thereof and a carrier protein (*e.g*., albumin). In some variations of any of the methods, the abnormal cell proliferation is abnormal cell proliferation of smooth muscle and endothelial cells.

The invention provides methods of inhibiting and/or reducing angiogenesis in the pulmonary artery of a patient having pulmonary hypertension comprising administering to the individual an effective amount of a composition comprising nanoparticles comprising rapamycin or a derivative thereof and a carrier protein (*e.g*., albumin). The invention also provides methods of inhibiting and/or reducing angiogenesis in the pulmonary artery of a patient having pulmonary hypertension comprising administering to the individual an effective amount of a composition comprising nanoparticles comprising taxane or a derivative thereof and a carrier protein (*e.g*., albumin). In some variations of any of the methods, angiogenesis in the pulmonary artery is inhibited and/or reduced by suppression of production of matrix metalloproteinases.

The invention provides methods of inhibiting and/or reducing inflammation in the pulmonary artery of a patient having pulmonary hypertension comprising administering to the individual an effective amount of a composition comprising nanoparticles comprising rapamycin or a derivative thereof and a carrier protein (*e.g*., albumin). The invention also provides methods of inhibiting and/or reducing inflammation in the pulmonary artery of a patient having pulmonary hypertension comprising administering to the individual an effective amount of a composition comprising nanoparticles comprising taxane or a derivative thereof and a carrier protein (*e.g*., albumin). In some variations of any of the methods, inflammation is inhibited and/or reduced by effecting neutrophil and T cell function.

In some variations, any of the method of treatment provided herein may be used to treat an individual who has previously been treated. The methods of treatment provided herein may be used to treat an individual who has not previously been treated. In some variations, the methods of treatment provided herein, a taxane or a derivative thereof is the only pharmaceutical active agent administered to the individual. In some variations, the methods of treatment provided herein, a rapamycin or a derivative thereof is the only pharmaceutical active agent administered to the individual. In some variations, the first and/or second therapies do comprise a SPARC polypeptide or anti-SPARC antibody (*i.e.*, other than SPARC polypeptide or anti-SPARC antibody). In some variations, the first and/or second therapies do not comprise a SPARC polypeptide or anti-SPARC antibody (*i.e.*, other than SPARC polypeptide or anti-SPARC antibody).

In some variations, any of the methods of treatment provided herein may be used to treat, stabilize, prevent, and/or delay any type or stage of pulmonary hypertension. In some variations, the individual is at least about any of 40, 45, 50, 55, 60, 65, 70, 75, 80, or 85 years old. In some variations, one or more symptoms of the pulmonary hypertension are ameliorated or eliminated. In some variations, the pulmonary hypertension is delayed or prevented.

In some variations of any of the methods of treatment provided herein, the individual is a human

### Dosing and Method of Administration

The dose of the inventive composition administered to an individual (such as a human) may vary with the particular composition, the method of administration, and the particular stage of pulmonary hypertension being treated. The amount should be sufficient to produce a desirable response, such as a therapeutic or prophylactic response against pulmonary hypertension. In some variations, the amount of the composition is a therapeutically effective amount. In some variations, that amount of the composition is a prophylactically effective amount. In some variations, the amount of rapamycin or a derivative thereof and/or paclitaxel or a derivative thereof in the composition is below the level that induces a toxicological effect (*i.e.,* an effect above a clinically acceptable level of toxicity) or is at a level where a potential side effect can be controlled or tolerated when the composition is administered to the individual.

In some variations, the amount of rapamycin or a derivative thereof in the composition is an amount sufficient to increase basal AKT activity, increase AKT phosphorylation, increase PI3-kinase activity, increase the length of activation of AKT (*e.g.,* activation induced by exogenous IGF-1), inhibit serine phosphorylation of IRS-1, inhibit IRS-1 degradation, inhibit or alter CXCR4 subcellular localization, inhibit VEGF secretion, decrease expression of cyclin D2, decrease expression of survivin, inhibit IL-6-induced multiple myeloma cell growth, inhibit cell proliferation, increase apoptosis, increase cell cycle arrest, increase cleavage of poly(ADPribose) polymerase, increase cleavage of caspase-8/caspase-9, alter or inhibit signaling in the phosphatidylinositol 3-kinase/AKT/mTOR and/or cyclin D1/retinoblastoma pathways, inhibit angiogenesis, and/or inhibit osteoclast formation.

In some variations, the invention provides a method of treating pulmonary hypertension in an individual by administering to the individual (*e.g*., a human) an effective amount of a composition comprising nanoparticles that comprise rapamycin or a derivative thereof and a carrier protein (*e.g*., albumin such as human serum albumin). In some variations, the amount of rapamycin or a derivative thereof in the composition is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of rapamycin or derivative thereof in the effective amount of the composition (*e.g*., a unit dosage form) is in the range of about 5 mg to about 500 mg, such as about 30 mg to about 300 mg or about 50 mg to about 200 mg. In some variations, the concentration of the rapamycin in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some variations, the concentration of the rapamycin is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of rapamycin or a derivative thereof in the nanoparticle composition include, but are not limited to, about any of 25 mg/m², 30 mg/m², 50 mg/m², 60 mg/m², 75 mg/m², 80 mg/m², 90 mg/m², 100 mg/m², 120 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² rapamycin. In various variations, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² rapamycin or a derivative thereof. In some variations, the amount of the rapamycin or a derivative thereof per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some variations, the effective amount of rapamycin or a derivative thereof in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some variations, the effective amount of rapamycin or a derivative thereof in the composition is about 5 to about 300 mg/m², such as about 100 to about 150 mg/m², about 120 mg/m², about 130 mg/m², or about 140 mg/m². In some variations, the effective amount of rapamycin or a derivative thereof in the composition is about 5 to about 100 mg/m². In some variations, the effective amount of rapamycin or a derivative thereof in the composition is about any of 30mg/m², 60 mg/m², 80 mg/m², or 100 mg/m².

In some variations of any of the above aspects, the effective amount of rapamycin or a derivative thereof in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various variations, the effective amount of rapamycin or a derivative thereof in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg rapamycin or a derivative thereof.

Exemplary dosing frequencies include, but are not limited to, weekly without break; weekly, three out of four weeks; once every three weeks; once every two weeks; weekly, two out of three weeks. In some variations, the composition is administered about once every 2 weeks, once every 3 weeks, once every 4 weeks, once every 6 weeks, or once every 8 weeks. In some variations, the composition is administered at least about any of 1x, 2x, 3x, 4x, 5x, 6x, or 7x (*i*.*e*., daily) a week. In some variations, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some variations, the intervals between each administration are more than about any of 1 month, 2 months, 3 months, 4 months, 5 months, 6 months, 8 months, or 12 months. In some variations, there is no break in the dosing schedule. In some variations, the interval between each administration is no more than about a week.

The administration of the composition can be extended over an extended period of time, such as from about a month up to about seven years. In some variations, the composition is administered over a period of at least about any of 2, 3, 4, 5, 6, 7, 8, 9, 10, 11, 12, 18, 24, 30, 36, 48, 60, 72, or 84 months. In some variations, the rapamycin or derivative thereof is administered over a period of at least one month, wherein the interval between each administration is no more than about a week, and wherein the dose of the rapamycin or a derivative thereof at each administration is about 0.25 mg/m² to about 75 mg/m², such as about 0.25 mg/m² to about 25 mg/m² or about 25 mg/m² to about 50 mg/m².

In some variations, the dosage of rapamycin in a nanoparticle composition can be in the range of 5-400 mg/m² when given on a 3 week schedule, or 5-250 mg/m² when given on a weekly schedule. Preferably, the amount of rapamycin is about 80 to about 180 mg/m² (e.g., about 100 mg/m² to about 150 mg/m², such as about 120 mg/m²).

Other exemplary dosing schedules for the administration of the nanoparticle composition (*e.g*., rapamycin/albumin nanoparticle composition) include, but are not limited to, 100 mg/m², weekly, without break; 30 mg/m² weekly, 3 out of four weeks; 60 mg/m² weekly, 3 out of four weeks; 75 mg/m² weekly, 3 out of four weeks; 80 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; and 150-250 mg/m² twice a week. The dosing frequency of the composition may be adjusted over the course of the treatment based on the judgment of the administering physician.

In yet another aspect, the invention provides a method of treating pulmonary hypertension in an individual by parenterally administering to the individual (*e.g*., a human) an effective amount of a composition comprising nanoparticles that comprise rapamycin or a derivative thereof and a carrier protein (*e.g*., albumin such as human serum albumin). The invention also provides a method of treating pulmonary hypertension in an individual by intravenous, intra-arterial, intramuscular, subcutaneous, inhalation, intraperitoneal, nasally, or intra-tracheal administering to the individual (*e.g*., a human) an effective amount of a composition comprising nanoparticles that comprise rapamycin or a derivative thereof and a carrier protein (*e.g*., albumin such as human serum albumin). In some variations, the route of administration is intravenous, intra-arterial, intramuscular, or subcutaneous. In some variations, an effective amount of the composition is administered systemically (*e.g*., intravenously) over a period of less than 30 minutes. In some variations, an effective amount of the composition is administered systemically (*e.g*., intravenously) over a period of about any of 30 minutes, 20 minutes, 15 minutes, 10 minutes, 5 minutes, or 1 minute. In various variations, about 5 mg to about 500 mg, such as about 30 mg to about 300 mg or about 50 mg to about 200 mg, of the rapamycin or derivative thereof is administered per dose. In some variations, a taxane is not contained in the composition. In some variations, the rapamycin or derivative thereof is the only pharmaceutically active agent for the treatment of pulmonary hypertension that is contained in the composition.

In some variations, the invention provides a method of treating pulmonary hypertension in an individual by administering to the individual (*e.g*., a human) an effective amount of a composition comprising nanoparticles that comprise a taxane or a derivative thereof and a carrier protein (*e.g*., albumin such as human serum albumin). In some variations, the amount of a taxane (*e.g*., paclitaxel) or a derivative thereof in the composition is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of a taxane (*e.g*., paclitaxel) or derivative thereof in the effective amount of the composition (*e.g*., a unit dosage form) is in the range of about 5 mg to about 500 mg, such as about 30 mg to about 300 mg or about 50 mg to about 200 mg. In some variations, the concentration of the taxane (*e.g*., paclitaxel) in the composition is dilute (about 0.1 mg/ml) or concentrated (about 100 mg/ml), including example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some variations, the concentration of the taxane (*e.g*., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (*e.g*., paclitaxel) or a derivative thereof in the nanoparticle composition include, but are not limited to, about any of 25 mg/m², 30 mg/m², 50 mg/m², 60 mg/m², 75 mg/m², 80 mg/m², 90 mg/m², 100 mg/m², 120 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of a taxane (*e.g*., paclitaxel). In various variations, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of a taxane (*e.g*., paclitaxel) or a derivative thereof. In some variations, the amount of the taxane (*e.g*., paclitaxel) or a derivative thereof per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some variations, the effective amount of a taxane (*e.g*., paclitaxel) or a derivative thereof in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some variations, the effective amount of a taxane (*e.g*., paclitaxel) or a derivative thereof in the composition is about 5 to about 300 mg/m², such as about 100 to about 150 mg/m², about 120 mg/m², about 130 mg/m², or about 140 mg/m². In some variations, the effective amount of taxane (*e.g.,* paclitaxel) or a derivative thereof in the composition is about 5 to about 100 mg/m². In some variations, the effective amount of taxane (*e.g*., paclitaxel) or a derivative thereof in the composition is about any of 30mg/m², 60 mg/m², 80 mg/m², or 100 mg/m2.

In some variations of any of the above aspects, the effective amount of a taxane (*e.g*., paclitaxel) or a derivative thereof in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various variations, the effective amount of a taxane (*e.g*., paclitaxel) or a derivative thereof in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (*e.g*., paclitaxel) or a derivative thereof.

Exemplary dosing frequencies include, but are not limited to, weekly without break; weekly, three out of four weeks; once every three weeks; once every two weeks; weekly, two out of three weeks. In some variations, the composition is administered about once every 2 weeks, once every 3 weeks, once every 4 weeks, once every 6 weeks, or once every 8 weeks. In some variations, the composition is administered at least about any of 1x, 2x, 3x, 4x, 5x, 6x, or 7x (*i.e.*, daily) a week. In some variations, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some variations, the intervals between each administration are more than about any of 1 month, 2 months, 3 months, 4 months, 5 months, 6 months, 8 months, or 12 months. In some variations, there is no break in the dosing schedule. In some variations, the interval between each administration is no more than about a week.

The administration of the composition can be extended over an extended period of time, such as from about a month up to about seven years. In some variations, the composition is administered over a period of at least about any of 2, 3, 4, 5, 6, 7, 8, 9, 10, 11, 12, 18, 24, 30, 36, 48, 60, 72, or 84 months. In some variations, the taxane (*e.g*., paclitaxel) or derivative thereof is administered over a period of at least one month, wherein the interval between each administration is no more than about a week, and wherein the dose of the taxane (*e.g*., paclitaxel) or a derivative thereof at each administration is about 0.25 mg/m² to about 75 mg/m², such as about 0.25 mg/m² to about 25 mg/m² or about 25 mg/m² to about 50 mg/m².

In some variations, the dosage of a taxane (*e.g*., paclitaxel) in a nanoparticle composition can be in the range of 5-400 mg/m² when given on a 3 week schedule, or 5-250 mg/m² when given on a weekly schedule. Preferably, the amount of a taxane (*e.g*., paclitaxel) is about 80 to about 180 mg/m² (*e.g.,* about 100 mg/m² to about 150 mg/m², such as about 120 mg/m²).

Other exemplary dosing schedules for the administration of the nanoparticle composition (*e.g*., paclitaxel/albumin nanoparticle composition) include, but are not limited to, 100 mg/m², weekly, without break; 30 mg/m² weekly, 3 out of four weeks; 60 mg/m² weekly, 3 out of four weeks; 75 mg/m² weekly, 3 out of four weeks; 80 mg/m² weekly, 3 out of four weeks; 100 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; and 150-250 mg/m² twice a week. The dosing frequency of the composition may be adjusted over the course of the treatment based on the judgment of the administering physician.

In yet another aspect, the invention provides a method of treating pulmonary hypertension in an individual by parenterally administering to the individual (*e.g*., a human) an effective amount of a composition comprising nanoparticles that comprise a taxane (*e.g*., paclitaxel) or a derivative thereof and a carrier protein (*e.g*., albumin such as human serum albumin). The invention also provides a method of treating pulmonary hypertension in an individual by intravenous, intra-arterial, intramuscular, subcutaneous, inhalation, intraperitoneal, nasally, or intra-tracheal administering to the individual (*e.g*., a human) an effective amount of a composition comprising nanoparticles that comprise a taxane (*e.g*., paclitaxel) or a derivative thereof and a carrier protein (*e.g*., albumin such as human serum albumin). In some variations, the route of administration is intravenous, intra-arterial, intramuscular, or subcutaneous. In some variations, an effective amount of the composition is administered systemically (*e.g*., intravenously) over a period of less than 30 minutes. In some variations, an effective amount of the composition is administered systemically (*e.g*., intravenously) over a period of about any of 30 minutes, 20 minutes, 15 minutes, 10 minutes, 5 minutes, or 1 minute. In various variations, about 5 mg to about 500 mg, such as about 30 mg to about 300 mg or about 50 to about 500 mg, of the taxane (*e.g*., paclitaxel) or derivative thereof is administered per dose. In some variations, the taxane (*e.g*., paclitaxel) or derivative thereof is the only pharmaceutically active agent for the treatment of pulmonary hypertension that is contained in the composition.

Any of the compositions described herein can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some variations, sustained continuous release formulation of the composition may be used. In one variation of the invention, nanoparticles (such as albumin nanoparticles) of the inventive compounds can be administered by any acceptable route including, but not limited to, orally, intramuscularly, transdermally, intravenously, through an inhaler or other air borne delivery systems and the like. In some variations, the rapamycin or derivative thereof and/or a taxane (*e.g*. paclitaxel) or derivative thereof is coating a stent or is administered using a stent. In some variations, the rapamycin or derivative thereof and/or a taxane (*e.g*. paclitaxel) or derivative thereof is not coating a stent or is not administered using a stent.

In some variations, the inventive composition can be administered by inhalation to treat PH. In some variations, the inventive composition can be administered by inhalation using an aerosol to treat PH. Formulations suitable for aerosol administration comprise the inventive composition include aqueous and non-aqueous, isotonic sterile solutions, which can contain anti-oxidants, buffers, bacteriostats, and solutes, as well as aqueous and non-aqueous sterile suspensions that can include suspending agents, solubilizers, thickening agents, stabilizers, and preservatives, alone or in combination with other suitable components, which can be made into aerosol formulations to be administered via inhalation. In some variations, the aerosol carrier may include, but is not limited to, lactose, trehalose, Pharmatose 325 M, sucrose, mannitol, and the like. The size of the aerosol carrier powder is significantly larger than that of the formulated drug particles (∼63-90 µm for lactose, 40-100 µm for Pharmatose). These aerosol formulations can be placed into pressurized acceptable propellants, such as dichlorodifluoromethane, propane, nitrogen, and the like. They also can be formulated as pharmaceuticals for non pressured preparations, such as in a nebulizer or an atomizer.

In some variations, rapamycin-containing nanoparticle composition and/or paclitaxel-containing nanoparticles composition may be administered with a second therapeutic compound and/or a second therapy. The dosing frequency of the rapamycin-containing nanoparticle composition and/or paclitaxel-containing nanoparticles composition and the second compound may be adjusted over the course of the treatment based on the judgment of the administering physician. In some variations, the first and second therapies are administered simultaneously, sequentially, or concurrently. When administered separately, the rapamycin-containing nanoparticle composition and/or a taxane (*e.g*., paclitaxel)-containing nanoparticles composition and the second compound can be administered at different dosing frequency or intervals. For example, the rapamycin-containing nanoparticle composition and/or a taxane (*e.g*., paclitaxel)-containing nanoparticle composition can be administered weekly, while a second compound can be administered more or less frequently. In some variations, sustained continuous release formulation of the rapamycin-containing nanoparticle and/or second compound may be used. In some variations, sustained continuous release formulation of the taxane (*e.g*., paclitaxel)-containing nanoparticle and/or second compound may be used. Various formulations and devices for achieving sustained release are known in the art. A combination of the administration configurations described herein can be used.

### Metronomic Therapy Regimes

The present invention also provides metronomic therapy regimes for any of the methods of treatment and methods of administration described herein. Exemplary metronomic therapy regimes and variations for the use of metronomic therapy regimes are discussed below and disclosed in U.S.S.N. 11/359,286, filed 2/21/2006, published as U.S. Pub. No. 2006/0263434 (such as those described in paragraphs [0138] to [0157]), which is hereby incorporated by reference in its entirety. In some variations, the nanoparticle composition is administered over a period of at least one month, wherein the interval between each administration is no more than about a week, and wherein the dose of rapamycin or a derivative thereof and/or a taxane (*e.g*., paclitaxel) at each administration is about 0.25% to about 25% of its maximum tolerated dose following a traditional dosing regime. In some variations, the nanoparticle composition is administered over a period of at least two months, wherein the interval between each administration is no more than about a week, and wherein the dose of rapamycin or a derivative thereof and/or a taxane (*e.g*., paclitaxel) at each administration is about 1% to about 20% of its maximum tolerated dose following a traditional dosing regime. In some variations, the dose of rapamycin or a derivative thereof per administration is less than about any of 25%, 24%, 23%, 22%, 20%, 18%, 15%, 14%, 13%, 12%, 11%, 10%, 9%, 8%, 7%, 6%, 5%, 4%, 3%, 2%, or 1% of the maximum tolerated dose. In some variations, the dose of a taxane (e.g., paclitaxel) or a derivative thereof per administration is less than about any of 25%, 24%, 23%, 22%, 20%, 18%, 15%, 14%, 13%, 12%, 11%, 10%, 9%, 8%, 7%, 6%, 5%, 4%, 3%, 2%, or 1% of the maximum tolerated dose. In some variations, any nanoparticle composition is administered at least about any of 1x, 2x, 3x, 4x, 5x, 6x, or 7x (*i.e.*, daily) a week. In some variations, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some variations, the intervals between each administration are more than about any of 1 month, 2 months, 3 months, 4 months, 5 months, 6 months, 8 months, or 12 months. In some variations, the composition is administered over a period of at least about any of 2, 3, 4, 5, 6, 7, 8, 9, 10, 11, 12, 18, 24, 30, 36, 48, 60, 72, or 84 months.

### Pharmaceutical agents

Provided herein are compositions comprising nanoparticles that comprise rapamycin for use in the methods of treatment of pulmonary hypertension, methods of administration, and dosing regimes described herein. In some variations, rapamycin may be rapamycin or its derivatives or pharmaceutically acceptable salts and accordingly the invention contemplates and includes all these variations. Rapamycin is sometimes referred to elsewhere as sirolimus, rapammune, or rapamune. Derivatives of rapamycin include, but are not limited to, compounds that are structurally similar to rapamycin or are in the same general chemical class as rapamycin.

In some embodiments, rapamycin or a derivative thereof increases basal AKT activity, increases AKT phosphorylation, increases PI3-kinase activity, increases the length of activation of AKT (*e.g*., activation induced by exogenous IGF-1), inhibits serine phosphorylation of IRS-1, inhibits IRS-1 degradation, inhibits or alters CXCR4 subcellular localization, inhibits VEGF secretion, decreases expression of cyclin D2, decreases expression of survivin, inhibits IL-6-induced multiple myeloma cell growth, inhibits cancer cell proliferation, increases apoptosis, increases cell cycle arrest, increases cleavage of poly(ADPribose) polymerase, increases cleavage of caspase-8/caspase-9, alters or inhibits signaling in the phosphatidylinositol 3-kinase/AKT/mTOR and/or cyclin D1/retinoblastoma pathways, inhibits angiogenesis, and/or inhibits osteoclast formation. I

In some variations, the derivative of rapamycin retains one or more similar biological, pharmacological, chemical and/or physical properties (including, for example, functionality) as rapamycin. In some variations, the rapamycin derivative has at least about any of 10%, 20%, 30%, 40%, 50%, 60%, 70%, 80%, 90%, 95% or 100% of an activity of rapamycin. An exemplary rapamycin derivative includes benzoyl rapamycin, such as that disclosed in paragraph [0022] of WO 2006/089207, which is hereby incorporated by reference in its entirety. Other exemplary rapamycin derivatives include WY-090217, AY-22989, NSC-226080, SiiA-9268A, oxaazacyclohentriacontine, temsirolimus (CCI-779 (Wyeth)), everolimus (RAD001 (Novartis)), pimecrolimus (ASM981), SDZ-RAD, SAR943, ABT-578, AP23573, and Biolimus A9.

In some variations of any of the compositions, the composition may be used to treat pulmonary hypertension. In some variations, the pulmonary hypertension is IPAH, FPAH, or APAH. In some variations, the compositions provided herein inhibit and/or reduce abnormal cell proliferation in the pulmonary artery. In some variations, the abnormal cell proliferation is abnormal cell proliferation of smooth muscle and endothelial cells. In some variations, the compositions provided herein inhibit and/or reduce angiogenesis in the pulmonary artery. In some variations, angiogenesis in the pulmonary artery is inhibited and/or reduced by suppression of production of matrix metalloproteinases. In some variations, the compositions provided herein inhibit and/or reduce inflammation in the pulmonary artery. In some variations, inflammation is inhibited and/or reduced by effecting neutrophil and T cell function. In some variations, the amount of a taxane (*e*.*g*., paclitaxel) or rapamycin administered is between about 30 mg/m² and about 250 mg/m².

Provided herein are compositions comprising nanoparticles that comprise a taxane for use in the methods of treatment of pulmonary hypertension, methods of administration, and dosing regimes described herein. Taxanes include, but are not limited to, compounds that are structurally similar to or are in the same general chemical class such as paclitaxel (*i.e.*, taxol), docetaxel (*i.e.*, taxotere), or ortataxel, and pharmaceutically acceptable salts, derivatives, or analogs of paclitaxel, docetaxel, and ortataxel. In some variations, the compositions provided herein inhibit and/or reduce abnormal cell proliferation in the pulmonary artery. In some variations, the abnormal cell proliferation is abnormal cell proliferation of smooth muscle and endothelial cells. In some variations, the compositions provided herein inhibit and/or reduce angiogenesis in the pulmonary artery. In some variations, angiogenesis in the pulmonary artery is inhibited and/or reduced by suppression of production of matrix metalloproteinases. In some variations, the compositions provided herein inhibit and/or reduce inflammation in the pulmonary artery. In some variations, inflammation is inhibited and/or reduced by effecting neutrophil and T cell function.

Provided herein are compositions for use in inhibiting abnormal cell proliferation in a patient having pulmonary hypertension comprising administering to the individual an effective amount of a composition comprising nanoparticles comprising rapamycin or a derivative thereof and a carrier protein (*e.g*., albumin). The invention also provides compositions for use in inhibiting abnormal cell proliferation in a patient having pulmonary hypertension comprising administering to the individual an effective amount of a composition comprising nanoparticles comprising taxane or a derivative thereof and a carrier protein (*e.g*., albumin). In some variations of any of the compositions for use, the abnormal cell proliferation is abnormal cell proliferation of smooth muscle and endothelial cells. In some variations, the amount of a taxane (*e.g*., paclitaxel) or rapamycin administered is between about 30 mg/m² and about 250 mg/m².

The invention provides compositions for use in inhibiting and/or reducing angiogenesis in the pulmonary artery of a patient having pulmonary hypertension comprising administering to the individual an effective amount of a composition comprising nanoparticles comprising rapamycin or a derivative thereof and a carrier protein (*e.g*., albumin). The invention also provides compositions for use in inhibiting and/or reducing angiogenesis in the pulmonary artery of a patient having pulmonary hypertension comprising administering to the individual an effective amount of a composition comprising nanoparticles comprising taxane or a derivative thereof and a carrier protein (*e.g*., albumin). In some variations of any of the compositions for use, angiogenesis in the pulmonary artery is inhibited and/or reduced by suppression of production of matrix metalloproteinases. In some variations, the amount of a taxane (*e.g*., paclitaxel) or rapamycin administered is between about 30 mg/m² and about 250 mg/m².

The invention provides compositions for use in inhibiting and/or reducing inflammation in the pulmonary artery of a patient having pulmonary hypertension comprising administering to the individual an effective amount of a composition comprising nanoparticles comprising rapamycin or a derivative thereof and a carrier protein (*e.g*., albumin). The invention also provides compositions for use in inhibiting and/or reducing inflammation in the pulmonary artery of a patient having pulmonary hypertension comprising administering to the individual an effective amount of a composition comprising nanoparticles comprising taxane or a derivative thereof and a carrier protein (*e.g*., albumin). In some variations of any of the compositions for use, inflammation is inhibited and/or reduced by effecting neutrophil and T cell function. In some variations, the amount of a taxane (*e.g*., paclitaxel) or rapamycin administered is between about 30 mg/m² and about 250 mg/m².

### Carrier proteins

Provided herein are compositions comprising nanoparticles that comprise rapamycin and a carrier protein for use methods of treatment of pulmonary hypertension, methods of administration, and dosage regimes described herein. In some variations, rapamycin may be rapamycin or its derivatives or pharmaceutically acceptable salts and accordingly the invention contemplates and includes all these variations. In some variations, the carrier protein is albumin. In some variations, the carrier protein is human serum albumin.

Provided herein are also compositions comprising nanoparticles that comprise a taxane and a carrier protein for use methods of treatment of pulmonary hypertension, methods of administration, and dosage regimes described herein. In some variations, a taxane may be a taxane or its derivatives or pharmaceutically acceptable salts and accordingly the invention contemplates and includes all these variations. In some variations, the taxane is paclitaxel. In some variations, the carrier protein is albumin. In some variations, the carrier protein is human serum albumin.

Examples of suitable carrier proteins include proteins normally found in blood or plasma, which include, but are not limited to, albumin, immunoglobulin including IgA, lipoproteins, apolipoprotein B, α-acid glycoprotein, β-2-macroglobulin, thyroglobulin, transferin, fibronectin, factor VII, factor VIII, factor IX, factor X, and the like. In some variations, the carrier protein is a non-blood protein, such as casein, α-lactalbumin, or β-lactoglobulin. The carrier proteins may either be natural in origin or synthetically prepared. In some variations, the pharmaceutical acceptable carrier comprises albumin, such as human serum albumin (HSA). HSA is a highly soluble globular protein of Mᵣ 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Other albumins are contemplated, such as bovine serum albumin. Use of such non-human albumins could be appropriate, for example, in the context of use of these compositions in non-human mammals, such as the veterinary animals (including domestic pets and agricultural animals).

Human serum albumin (HSA) has multiple hydrophobic binding sites (a total of eight for fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, *e.g.,* Fehske et al., Biochem. Pharmcol., 30, 687-92 (1981), Vorum, Dan. Med. Bull., 46, 379-99 (1999), Kragh-Hansen, Dan. Med. Bull., 1441, 131-40 (1990), Curry et al., Nat. Struct. BioL, 5, 827-35 (1998), Sugio et al., Protein. Eng.,12, 439-46 (1999), He et al., Nature, 358, 209-15 (1992), and Carter et al., Adv. Protein. Chem., 45, 153-203 (1994)).

The carrier protein (*e.g*., albumin) in the composition generally serves as a carrier for rapamycin or derivative thereof and/or a taxane (*e.g*., paclitaxel) or derivative thereof, i.e., the carrier protein in the composition makes the rapamycin or derivative thereof and/or a taxane (*e.g*., paclitaxel) or derivative thereof more readily suspendable in an aqueous medium or helps maintain the suspension as compared to compositions not comprising a carrier protein. This can avoid the use of toxic solvents for solubilizing of rapamycin or a derivative thereof and/or a taxane (*e.g.,* paclitaxel) or a derivative thereof, and thereby can reduce one or more side effects of administration of rapamycin or a derivative thereof and/or a taxane (*e.g*., paclitaxel) or a derivative thereof into an individual (*e.g*., human). In some variations, the composition is substantially free (*e.g*. free) of organic solvents or surfactants. A composition is "substantially free of organic solvent" or "substantially free of surfactant" if the amount of organic solvent or surfactant in the composition is not sufficient to cause one or more side effect(s) in an individual when the composition is administered to the individual. In some variations, the nanoparticles in the composition have a solid core. In some variations, the nanoparticles in the composition have a core that is not aqueous (*i.e.*, other than aqueous core). In some variation, the nanoparticles in the composition are substantially free of lipids. In some variations, the nanoparticles in the composition are free of lipids. In some variations, the nanoparticles of the composition lack a polymeric matrix. In some variations, the nanoparticles of the composition are filter sterilizable. In some variations, the nanoparticles in the composition comprise at least one cross-linked carrier protein. In some variations, the nanoparticles in the composition comprise at least ten-percent of carrier protein that is cross-linked.

Rapamycin and/or the taxane (*e.g*., paclitaxel) is "stabilized" in an aqueous suspension if it remains suspended in an aqueous medium (*e.g*., without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (*e.g*., human). Stability of the suspension is generally (but not necessarily) evaluated at storage temperature, such as room temperature (*e.g.*, 20-25 °C) or refrigerated conditions (*e.g.*, 4 °C). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some variations, the composition comprises nanoparticles comprising (in various variations consisting essentially of) rapamycin and a carrier protein. In some variations, the composition comprises nanoparticles comprising (in various variations consisting essentially of) a taxane (*e.g.*, paclitaxel) and a carrier protein. When rapamycin and/or a taxane (*e.g.*, paclitaxel) is in a liquid form, the particles or nanoparticles are also referred to as droplets or nanodroplets. In some variations, rapamycin is coated with the carrier protein. In some variations, paclitaxel is coated with the carrier protein. Particles (such as nanoparticles) of poorly water soluble pharmaceutical agents have been disclosed in, for example, U.S. Pat. Nos. 5,916,596; 6,506,405; and 6,537,579 and also in U.S. Pat. App. Pub. No. 2005/0004002A1.

The amount of carrier protein in the composition described herein will vary depending on the rapamycin or derivative thereof, the taxane (*e.g*., paclitaxel) or derivative thereof, and other components in the composition. In some variations, the composition comprises a carrier protein in an amount that is sufficient to stabilize the rapamycin or the taxane (*e.g*., paclitaxel) in an aqueous suspension, for example, in the form of a stable colloidal suspension (*e.g*., a stable suspension of nanoparticles). In some variations, the carrier protein is in an amount that reduces the sedimentation rate of rapamycin or the taxane (*e.g*., paclitaxel) in an aqueous medium. The amount of the carrier protein also depends on the size and density of particles of rapamycin or the taxane (*e.g.*, paclitaxel).

In some variations of any of the aspects of the invention, the rapamycin or a derivative thereof is coated with a carrier protein, such as albumin (*e.g*., human serum albumin). In various variations, the composition comprises more than about any of 50%, 60%, 70%, 80%, 90%, 95%, or 99% of the rapamycin or derivative thereof in nanoparticle form. In some variations, the rapamycin or derivative thereof constitutes more than about any of 50%, 60%, 70%, 80%, 90%, 95%, or 99% of the nanoparticles by weight. In some variations, the nanoparticle has a non-polymeric matrix. In some variations, the nanoparticle has a non-polymeric matrix. In some variations, the rapamycin or derivative thereof is amorphous. In some variations, the rapamycin or derivative thereof is non-crystalline form. In some variations, the rapamycin or derivative thereof used for making the nanoparticle compositions is in anhydrous form. In some variations, the albumin to rapamycin weight ratio is about any of 18:1 or less, 15:1 or less, 14:1 or less, 13:1 or less, 12:1 or less, 11:1 or less, 10:1 or less, 9:1 or less, 8:1 or less, 7.5:1 or less, 7:1 or less, 6:1 or less, 5:1 or less, 4:1 or less, or 3:1 or less. In some variations, the albumin to rapamycin weight ratio is between about any of 18:1 to 1:18, 10:1 to 1:10, 9:1 to 1:1,8:1 to 1:1,7.5:1 to 1.1,7.1:1 to 1:1, 6:1 to 1:1,5:1 to 1:1,4:1 to 1:1, or 3:1 to 1:1. In some variations, the composition comprises a stable aqueous suspension of particles (*e.g*., nanoparticles) comprising rapamycin or a derivative thereof and albumin (*e.g*., particles of rapamycin or a derivative thereof coated with albumin).

In some variations of any of the aspects of the invention, the taxane or a derivative thereof is coated with a carrier protein, such as albumin (*e.g*., human serum albumin). In some variations, the taxane is paclitaxel. In various variations, the composition comprises more than about any of 50%, 60%, 70%, 80%, 90%, 95%, or 99% of the taxane or derivative thereof in nanoparticle form. In some variations, the taxane or derivative thereof constitutes more than about any of 50%, 60%, 70%, 80%, 90%, 95%, or 99% of the nanoparticles by weight. In some variations, the nanoparticle has a non-polymeric matrix. In some variations, the taxane or a derivative thereof is amorphous. In some variations, the taxane or a derivative thereof is non-crystalline form. In some variations, the taxane or a derivative thereof used for making the nanoparticle compositions is in anhydrous form. In some variations, the albumin to a taxane weight ratio is about any of 18:1 or less, 15:1 or less, 14:1 or less, 13:1 or less, 12:1 or less, 11:1 or less, 10:1 or less, 9:1 or less, 8:1 or less, 7.5:1 or less, 7:1 or less, 6:1 or less, 5:1 or less, 4:1 or less, or 3:1 or less. In some variations, the albumin to taxane weight ratio is between about any of 18:1 to 1:18, 10:1 to1:10, 9:1 to 1:1, 8:1 to 1:1, 7.5:1 to 1.1, 7.1:1 to 1:1, 6:1 to 1:1, 5:1 to 1:1, 4:1 to 1:1, or 3:1 to 1:1. In some variations, the composition comprises a stable aqueous suspension of particles (*e.g*., nanoparticles) comprising a taxane or a derivative thereof and albumin (*e.g*., particles of paclitaxel or a derivative thereof coated with albumin).

In some variations, the composition comprises nanoparticles of any shape (*e.g.*, a spherical or non-spherical shape) with an average or mean diameter of no greater than about 1000 nanometers (nm), such as no greater than about any of 900 nm, 800 nm, 700 nm, 600 nm, 500 nm, 400 nm, 300 nm, 200 nm, 100 nm, 90 nm, 80 nm, 70 nm, 60 nm, or 50 nm. In some variations, the average or mean diameter of the particles is no greater than about 200 nm. In some variations, the average or mean diameter of the particles is no greater than about 100 nm. In some variations, the average or mean diameter of the particles is between about 20 to about 400 nm. In some variations, the average or mean diameter of the particles is between about 40 to about 200 nm. In some variations, the composition comprises nanoparticles with a diameter of about any of 1000 nm, 900 nm, 800 nm, 700 nm, 600 nm, 500 nm, 400 nm, 300 nm, 200 nm, 100 nm, 90 nm, 80 nm, 70 nm, 60 nm, or 50 nm. In some variations, the composition comprises nanoparticles with a diameter of between about any of 50 nm and 75 nm, 50 nm and 100 nm, 75 nm and 100 nm, 100 nm and 125 nm, 125 nm and 150 nm, 100 nm and 150 nm, 150 nm and 175 nm, or 175 nm and 200 nm. In some variations, the diameter of about any of 50% or more, 65% or more, 75% or more, 80% or more, 90% or more, 95% or more, 98% or more, 99% or more, 99.5% or more, or 99.9% or more of the nanoparticles can fall within the range specified. In some variations, the particles are sterile-filterable.

The nanoparticles described herein may be present in a dry formulation (e.g., lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

In some variations, the nanoparticles do not comprise a blood-insoluble gas or do not comprise gas-filled microbubbles.

Also provided herein are pharmaceutical compositions for use in the methods of the invention, wherein the protein carrier is in an effective amount to reduce one or more side effects associated with administration of poorly water soluble pharmaceutical agent to a human compared to compositions without carrier protein. For example, the invention provides methods of reducing various side effects associated with administration of the poorly water soluble pharmaceutical agent, including, but not limited to, myelosuppression, neurotoxicity, hypersensitivity, inflammation, venous irritation, phlebitis, pain, skin irritation, neutropenic fever, anaphylactic reaction, hematologic toxicity, and cerebral or neurologic toxicity, and combinations thereof. In some variations, there is provided a method of reducing hypersensitivity reactions associated with administration of rapamycin or a derivative thereof and/or a taxane (*e.g*., paclitaxel) or a derivative thereof, including, for example, severe skin rashes, hives, flushing, dyspnea, tachycardia, pulmonary hypertension (*e.g*., lymphoma); chest pain; black, tarry stools; general feeling of illness, shortness of breath; swollen glands; weight loss; yellow skin and eyes, abdominal pain; unexplained anxiousness; bloody or cloudy urine; bone pain; chills; confusion; convulsions (seizures); cough; decreased urge to urinate; fast, slow, or irregular heartbeat; fever; frequent urge to urinate; increased thirst; loss of appetite; lower back or side pain; mood changes; muscle pain or cramps; nausea or vomiting; numbness or tingling around lips, hands, or feet; painful or difficult urination; rash; sore throat; sores or white spots on lips or in mouth; swelling of hands, ankles, feet, or lower legs; swollen glands; trouble breathing; unusual bleeding or bruising; unusual tiredness or weakness; weakness or heaviness of legs, skin ulcer or sores, weight gain, acne; constipation; diarrhea; difficulty in moving; headache; loss of energy or weakness; muscle pain or stiffness; pain; shaking or trembling; trouble sleeping; nosebleed; and/or swelling of the face. These side effects, however, are merely exemplary and other side effects, or combination of side effects, associated with rapamycin and/or a taxane (*e.g*., paclitaxel) can be reduced. The side effects may be immediate or delayed (such as not occurring for a few days, weeks, months, or years after treatment begins).

### Antimicrobial Agents in Compositions

In some variations, the compositions of the invention also include an antimicrobial agent (*e.g*., an agent in addition to the rapamycin or derivatives thereof or an agent in addition to the taxane (*e.g*., paclitaxel) or derivatives thereof) in an amount sufficient to significantly inhibit (*e.g*., delay, reduce, slow, and/or prevent) microbial growth in the composition for use in the methods of treatment, methods of administration, and dosage regimes described herein. Exemplary microbial agents and variations for the use of microbial agents are disclosed in U.S.S.N. 11/514,030, filed 8/30/2006 (such as those described in paragraphs [0036] to [0058]). In some variations, the antimicrobial agent is a chelating agent, such as EDTA, edetate, citrate, pentetate, tromethamine, sorbate, ascorbate, derivatives thereof, or mixtures thereof. In some variations, the antimicrobial agent is a polydentate chelating agent. In some variations, the antimicrobial agent is a non-chelating agent, such as any of sulfites, benzoic acid, benzyl alcohol, chlorobutanol, paraben, or derivatives thereof. In some variations, an antimicrobial other than rapamycin or derivatives thereof or a taxane or derivatives thereof discussed above is not contained or used in the methods of treatment, methods of administration, and dosage regimes described herein.

### Sugar Containing Compositions

In some variations, the compositions of the invention include a sugar for use in the methods of treatment described herein. In some variations, the compositions of the invention include both a sugar and an antimicrobial agent for use in the methods of treatment described herein. Exemplary sugars and variations for the use of sugars are disclosed in U.S.S.N. 11/514,030, filed 8/30/2006 (such as those described in paragraphs [0084] to [0090]). In some variations, the sugar serves as a reconstitution enhancer which causes a lyophilized composition to dissolve or suspend in water and/or aqueous solution more quickly than the lyophilized composition would dissolve without the sugar. In some variations, the composition is a liquid (*e.g*., aqueous) composition obtained by reconstituting or resuspending a dry composition. In some variations, the concentration of sugar in the composition is greater than about 50 mg/ml. In some variations, the sugar is in an amount that is effective to increase the stability of the rapamycin or derivative thereof in the composition as compared to a composition without the sugar. In some variations, the sugar is in an amount that is effective to increase the stability of the taxane (*e.g*., paclitaxel) or derivative thereof in the composition as compared to a composition without the sugar. In some variations, the sugar is in an amount that is effective to improve filterability of the composition as compared to a composition without the sugar.

The sugar-containing compositions described herein may further comprise one or more antimicrobial agents, such as the antimicrobial agents described herein or in U.S.S.N. 11/514,030, filed 8/30/2006. In addition to one or more sugars, other reconstitution enhancers

(such as those described in U.S. Pat. App. Publication No. 2005/0152979, which is hereby incorporated by reference in its entirety) can also be added to the compositions. In some variations, a sugar is not contained or used in the methods of treatment, methods of administration, and dosage regimes described herein.

### Stabilizing Agents in Compositions

In some variations, the compositions of the invention also include a stabilizing agent for use in the methods of treatment, methods of administration, and dosage regimes described herein. In some variations, the compositions of the invention include an antimicrobial agent and/or a sugar and/or a stabilizing agent for use in the methods of treatment, methods of administration, and dosage regimes described herein. Exemplary stabilizing agents and variations for the use of stabilizing agents are disclosed in U.S.S.N. 11/513,756, filed 8/30/2006 (such as those described in paragraphs [0038] to [0083] and [0107] to [0114]). The present invention in one of its variations provides for compositions and methods of preparation of rapamycin which retain the desirable therapeutic effects and remain physically and/or chemically stable upon exposure to certain conditions such as prolonged storage, elevated temperature, or dilution for parenteral administration. The present invention in another variation provides for compositions and methods of preparation of a taxane (*e.g*., paclitaxel) or derivatives thereof which retain the desirable therapeutic effects and remain physically and/or chemically stable upon exposure to certain conditions such as prolonged storage, elevated temperature, or dilution for parenteral administration. The stabilizing agent includes, for example, chelating agents (*e.g*., citrate, malic acid, edetate, or pentetate), sodium pyrophosphate, and sodium gluconate. In one variation, the invention provides pharmaceutical formulations of rapamycin or a derivative thereof comprising citrate, sodium pyrophosphate, EDTA, sodium gluconate, citrate and sodium chloride, and/or a derivative thereof. In another variation, the invention provides a composition of rapamycin, wherein the rapamycin used for preparing the formulation is in an anhydrous form prior to being incorporated into the composition. In some variations, the invention provides pharmaceutical formulations of a taxane (*e.g*., paclitaxel) or a derivative thereof comprising citrate, sodium pyrophosphate, EDTA, sodium gluconate, citrate and sodium chloride, and/or a derivative thereof. In another variation, the invention provides a composition of a taxane, wherein the taxane (*e.g*., paclitaxel) used for preparing the formulation is in an anhydrous form prior to being incorporated into the composition.

In some variations, a stabilizing agent is not contained or used in the methods of treatment, methods of administration, and dosage regimes described herein.

### Pharmaceutical Compositions and Formulations

The compositions described herein may be used in the preparation of a formulation, such as a pharmaceutical formulation, by combining the nanoparticle composition(s) described with a pharmaceutical acceptable carrier, excipients, stabilizing agents or other agent, which are known in the art, for use in the methods of treatment, methods of administration, and dosage regimes described herein. To increase stability by increasing the negative zeta potential of nanoparticles, certain negatively charged components may be added. Such negatively charged components include, but are not limited to bile salts, bile acids, glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid, and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine, stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearoylphosphatidylcholine (DSPC), hydrogenated soy phosphatidylcholine (HSPC), and other related compounds. Negatively charged surfactants or emulsifiers are also suitable as additives, *e.g*., sodium cholesteryl sulfate and the like.

In some variations, the composition is suitable for administration to a human. In some variations, the composition is suitable for administration to a mammal such as, in the veterinary context, domestic pets and agricultural animals. There are a wide variety of suitable formulations of the inventive composition (*see, e.g.,* U.S. Pat. Nos. 5,916,596 and 6,096,331, which are hereby incorporated by reference in their entireties). The following formulations and methods are merely exemplary and are in no way limiting. Formulations suitable for oral administration can comprise (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, (d) suitable emulsions, and (e) powders. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizing agents, and preservatives. The formulations can be presented in unit-dose or multi-dose sealed containers, such as ampules and vials, and can be stored in a freeze-dried (lyophilized) condition requiring only the addition of the sterile liquid excipient methods of treatment, methods of administration, and dosage regimes described herein (*i.e.*, water) for injection, immediately prior to use. Extemporaneous injection solutions and suspensions can be prepared from sterile powders, granules, and tablets of the kind previously described. Injectable formulations are preferred.

The invention also includes formulations of nanoparticle compositions comprising rapamycin or derivative thereof or a taxane (*e.g*., paclitaxel) or derivative thereof and a carrier suitable for administration by inhalation for use in the methods of the invention. Formulations suitable for aerosol administration comprise the inventive composition include aqueous and non-aqueous, isotonic sterile solutions, which can contain anti-oxidants, buffers, bacteriostats, and solutes, as well as aqueous and non-aqueous sterile suspensions that can include suspending agents, solubilizers, thickening agents, stabilizing agents, and preservatives, alone or in combination with other suitable components, which can be made into aerosol formulations to be administered via inhalation. These aerosol formulations can be placed into pressurized acceptable propellants, such as dichlorodifluoromethane, propane, nitrogen, and the like. They also can be formulated as pharmaceuticals for non-pressured preparations, such as in a nebulizer or an atomizer.

In some variations, the composition is formulated to have a pH in the range of about 4.5 to about 9.0, including for example pH ranges of any of about 5.0 to about 8.0, about 6.5 to about 7.5, and about 6.5 to about 7.0. In some variations, the pH of the composition is formulated to no less than about 6, including for example no less than about any of 6.5, 7, or 8 (*e.g*., about 8). The composition can also be made to be isotonic with blood by the addition of a suitable tonicity modifier, such as glycerol.

The nanoparticles of this invention can be enclosed in a hard or soft capsule, can be compressed into tablets, or can be incorporated with beverages or food or otherwise incorporated into the diet. Capsules can be formulated by mixing the nanoparticles with an inert pharmaceutical diluent and inserting the mixture into a hard gelatin capsule of the appropriate size. If soft capsules are desired, a slurry of the nanoparticles with an acceptable vegetable oil, light petroleum or other inert oil can be encapsulated by machine into a gelatin capsule.

In some variations of any of the formulations described herein, the formulation may be used to treat pulmonary hypertension. In some variations, the pulmonary hypertension is IPAH, FPAH, or APAH. In some variations, the amount of a taxane (*e.g*., paclitaxel) or rapamycin administered is between about 30 mg/m² and about 250 mg/m².

Also provided are unit dosage forms comprising the compositions and formulations described herein. In some variations of any of the unit dosage forms, the unit dosage form may be used to treat pulmonary hypertension. In some variations, the pulmonary hypertension is IPAH, FPAH, or APAH. These unit dosage forms can be stored in a suitable packaging in single or multiple unit dosages and may also be further sterilized and sealed. For example, the pharmaceutical composition (*e.g*., a dosage or unit dosage form of a pharmaceutical composition) may include (i) nanoparticles that comprise rapamycin or a derivative thereof and a carrier protein and (ii) a pharmaceutically acceptable carrier. In some variations, the pharmaceutical composition also includes one or more other compounds (or pharmaceutically acceptable salts thereof) that are useful for treating pulmonary hypertension. In various variations, the amount of rapamycin or a derivative thereof in the composition is included in any of the following ranges: about 5 to about 20, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of rapamycin or derivative thereof in the composition (*e.g*., a dosage or unit dosage form) is in the range of about 5 mg to about 500 mg, such as about 30 mg to about 300 mg or about 50 mg to about 200 mg, of the rapamycin or derivative thereof. In some variations, the carrier is suitable for parental administration (*e.g*., intravenous administration). In some variations, a taxane is not contained in the composition. In some variations, the rapamycin or derivative thereof is the only pharmaceutically active agent for the treatment of pulmonary hypertension that is contained in the composition.

In some variations, the invention features a dosage form (*e.g.*, a unit dosage form) for the treatment of pulmonary hypertension comprising (i) nanoparticles that comprise a carrier protein and rapamycin or a derivative thereof, wherein the amount of rapamycin or derivative thereof in the unit dosage from is in the range of about 5 mg to about 500 mg, and (ii) a pharmaceutically acceptable carrier. In some variations, the amount of the rapamycin or derivative thereof in the unit dosage form includes about 30 mg to about 300 mg.

Also provided are unit dosage forms comprising the compositions and formulations described herein. These unit dosage forms can be stored in a suitable packaging in single or multiple unit dosages and may also be further sterilized and sealed. For example, the pharmaceutical composition (*e.g*., a dosage or unit dosage form of a pharmaceutical composition) may include (i) nanoparticles that comprise a taxane (*e.g*., paclitaxel) or a derivative thereof and a carrier protein and (ii) a pharmaceutically acceptable carrier. In some variations, the pharmaceutical composition also includes one or more other compounds (or pharmaceutically acceptable salts thereof) that are useful for treating pulmonary hypertension. In various variations, the amount of a taxane (*e.g*., paclitaxel) or a derivative thereof in the composition is included in any of the following ranges: about 5 to about 50 mg, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of a taxane (*e.g.*, paclitaxel) or derivative thereof in the composition (*e.g.*, a dosage or unit dosage form) is in the range of about 5 mg to about 500 mg, such as about 30 mg to about 300 mg or about 50 mg to about 200 mg, of the taxane (*e.g*., paclitaxel) or derivative thereof. In some variations, the carrier is suitable for parental administration (*e.g*., intravenous administration). In some variations, a rapamycin is not contained in the composition. In some variations, the taxane (*e.g*., paclitaxel) or derivative thereof is the only pharmaceutically active agent for the treatment of pulmonary hypertension that is contained in the composition.

In some variations, the invention features a dosage form (*e.g.*, a unit dosage form) for the treatment of pulmonary hypertension comprising (i) nanoparticles that comprise a carrier protein and a taxane (*e.g*., paclitaxel) or a derivative thereof, wherein the amount of a taxane (*e.g*., paclitaxel) or derivative thereof in the unit dosage from is in the range of about 5 mg to about 500 mg, and (ii) a pharmaceutically acceptable carrier. In some variations, the amount of the taxane (*e.g*., paclitaxel) or derivative thereof in the unit dosage form includes about 30 mg to about 300 mg.

Also provided are articles of manufacture comprising the compositions, formulations, and unit dosages described herein in suitable packaging for use in the methods of treatment, methods of administration, and dosage regimes described herein. In some variations of any of the articles of manufacture, the articles of manufacture may be used to treat pulmonary hypertension. In some variations, the pulmonary hypertension is IPAH, FPAH, or APAH. Suitable packaging for compositions described herein are known in the art, and include, for example, vials (such as sealed vials), vessels (such as sealed vessels), ampules, bottles, jars, flexible packaging (*e.g*., sealed Mylar or plastic bags), and the like. These articles of manufacture may further be sterilized and/or sealed.

### Kits

The invention also provides kits comprising the compositions, formulations, unit dosages, and articles of manufacture described herein for use in the methods of treatment, methods of administration, and dosage regimes described herein. In some variations of any of the kits, the kits may be used to treat pulmonary hypertension. In some variations, the pulmonary hypertension is IPAH, FPAH, or APAH. Kits of the invention include one or more containers comprising rapamycin or a derivative thereof-containing nanoparticle compositions (formulations or unit dosage forms and/or articles of manufacture), and in some variations, further comprise instructions use in accordance with any of the methods of treatment described herein. In some variations, the kit comprises i) a composition comprising nanoparticles comprising a rapamycin and a carrier protein (such as albumin) and ii) instructions for administering the nanoparticles and the chemotherapeutic agents simultaneously and/or sequentially, for treatment of pulmonary hypertension. In some variations, the pulmonary hypertension is pulmonary arterial hypertension. In some variations, the pulmonary arterial hypertension is idiopathic pulmonary arterial hypertension. In various variations, the amount of rapamycin or a derivative thereof in the kit is included in any of the following ranges: about 5 mg to about 20 mg, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of rapamycin or a derivative thereof in the kit is in the range of about 5 mg to about 500 mg, such as about 30 mg to about 300 mg or about 50 mg to about 200 mg. In some variations, the kit includes one or more other compounds (*i*.*e*., one or more compounds other than a taxane) that are useful for treating pulmonary hypertension

Kits of the invention include one or more containers comprising a taxane (*e.g*., paclitaxel) or a derivative thereof-containing nanoparticle compositions (formulations or unit dosage forms and/or articles of manufacture), and in some variations, further comprise instructions for use in accordance with any of the methods of treatment described herein. In some variations, the kit comprises i) a composition comprising nanoparticles comprising a taxane (*e.g*., paclitaxel) and a carrier protein (such as albumin) and ii) instructions for administering the nanoparticles and the chemotherapeutic agents simultaneously and/or sequentially, for treatment of pulmonary hypertension. In some variations, the pulmonary hypertension is pulmonary arterial hypertension. In some variations, the pulmonary arterial hypertension is idiopathic pulmonary arterial hypertension. In various variations, the amount of a taxane (*e.g*., paclitaxel) or a derivative thereof in the kit is included in any of the following ranges: about 5 mg to about 20 mg, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some variations, the amount of a taxane (*e.g*., paclitaxel) or a derivative thereof in the kit is in the range of about 5 mg to about 500 mg, such as about 30 mg to about 300 mg or about 50 mg to about 200 mg. In some variations, the kit includes one or more other compounds (*i.e.*, one or more compounds other than a rapamycin) that are useful for treating pulmonary hypertension.

Instructions supplied in the kits of the invention are typically written instructions on a label or package insert (*e.g.*, a paper sheet included in the kit), but machine-readable instructions (*e.g*., instructions carried on a magnetic or optical storage disk) are also acceptable. The instructions relating to the use of the nanoparticle compositions generally include information as to dosage, dosing schedule, and route of administration for the intended treatment. The kit may further comprise a description of selecting an individual suitable or treatment.

The present invention also provides kits comprising compositions (or unit dosages forms and/or articles of manufacture) described herein and may further comprise instruction(s) on methods of using the composition, such as uses further described herein. In some variations, the kit of the invention comprises the packaging described above. In other variations, the kit of the invention comprises the packaging described above and a second packaging comprising a buffer. It may further include other materials desirable from a commercial and user standpoint, including other buffers, diluents, filters, needles, syringes, and package inserts with instructions for performing any methods described herein.

For combination therapies of the invention, the kit may contain instructions for administering the first and second therapies simultaneously and/or sequentially for the effective treatment of pulmonary hypertension. The first and second therapies can be present in separate containers or in a single container. It is understood that the kit may comprise one distinct composition or two or more compositions wherein one composition comprises a first therapy and one composition comprises a second therapy.

Kits may also be provided that contain sufficient dosages of rapamycin or a derivative thereof and/or a taxane (*e.g*., paclitaxel) or a derivative thereof as disclosed herein to provide effective treatment for an individual for an extended period, such as any of a week, 2 weeks, 3 weeks, 4 weeks, 6 weeks, 8 weeks, 3 months, 4 months, 5 months, 6 months, 7 months, 8 months, 9 months or more. Kits may also include multiple unit doses of rapamycin or a derivative thereof and/or a taxane (*e.g*., paclitaxel) or a derivative thereof compositions, pharmaceutical compositions, and formulations described herein and instructions for use and packaged in quantities sufficient for storage and use in pharmacies, for example, hospital pharmacies and compounding pharmacies. In some variations, the kit comprises a dry (*e.g.*, lyophilized) composition that can be reconstituted, resuspended, or rehydrated to form generally a stable aqueous suspension of nanoparticles comprising rapamycin or a derivative thereof and albumin (*e.g*., rapamycin or a derivative thereof coated with albumin). In some variations, the kit comprises a dry (*e.g*., lyophilized) composition that can be reconstituted, resuspended, or rehydrated to form generally a stable aqueous suspension of nanoparticles comprising a taxane (*e.g.*, paclitaxel) or a derivative thereof and albumin (*e.g.*, a taxane (*e.g.*, paclitaxel) or a derivative thereof coated with albumin).

The kits of the invention are in suitable packaging. Suitable packaging include, but is not limited to, vials, bottles, jars, flexible packaging (*e.g*., seled Mylar or plastic bags), and the like. Kits may optionally provide additional components such as buffers and interpretative information.

### Methods of Making the Compositions

Methods of making compositions containing carrier proteins and poorly water soluble pharmaceutical agents are known in the art. For example, nanoparticles containing poorly water soluble pharmaceutical agents and carrier proteins (*e.g*., albumin) can be prepared under conditions of high shear forces (*e.g*., sonication, high pressure homogenization, or the like). These methods are disclosed in, for example, U.S. Pat. Nos. 5,916,596; 6,506,405; and 6,537,579 and also in U.S. Pat. Pub. No. 2005/0004002A1, which are each hereby incorporated by reference in their entireties.

Briefly, the rapamycin or a derivative thereof and/or a taxane (*e.g*., paclitaxel) or a derivative thereof is dissolved in an organic solvent. Suitable organic solvents include, for example, ketones, esters, ethers, chlorinated solvents, and other solvents known in the art. For example, the organic solvent can be methylene chloride, chloroform/ethanol, or chloroform/t-butanol (for example with a ratio of about any of 1:9, 1:8, 1:7, 1:6, 1:5, 1:4, 1:3, 1:2, 1:1,2:1, 3:1, 4:1, 5:1, 6:1, 7:1, 8:1, or 9:1 or with a ratio of about any of 3:7, 5:7, 4:6, 5:5, 6:5, 8:5, 9:5, 9.5:5, 5:3, 7:3, 6:4, or 9.5:0.5). The solution is added to a carrier protein (*e.g*., human serum albumin). The mixture is subjected to high pressure homogenization (*e.g*., using an Avestin, APV Gaulin, Microfluidizer™ such as a Microfluidizer™ Processor M-110EH from Microfluidics, Stansted, or Ultra Turrax homogenizer). The emulsion may be cycled through the high pressure homogenizer for between about 2 to about 100 cycles, such as about 5 to about 50 cycles or about 8 to about 20 cycles (*e.g*., about any of 8, 10, 12, 14, 16, 18 or 20 cycles). The organic solvent can then be removed by evaporation utilizing suitable equipment known for this purpose, including, but not limited to, rotary evaporators, falling film evaporators, wiped film evaporators, spray driers, and the like that can be operated in batch mode or in continuous operation. The solvent may be removed at reduced pressure (such as at about any of 25 mm Hg, 30 mm Hg, 40 mm Hg, 50 mm Hg, 100 mm Hg, 200 mm Hg, or 300 mm Hg). The amount of time used to remove the solvent under reduced pressure may be adjusted based on the volume of the formulation. For example, for a formulation produced on a 300 mL scale, the solvent can be removed at about 1 to about 300 mm Hg (*e.g.*, about any of 5-100 mm Hg, 10-50 mm Hg, 20-40 mm Hg, or 25 mm Hg) for about 5 to about 60 minutes (*e.g*., about any of 7, 8, 9, 10, 11, 12, 13, 14, 15 16, 18, 20, 25, or 30 minutes).

If desired, human albumin solution may be added to the dispersion to adjust the human serum albumin to rapamycin and/or a taxane (*e.g*., paclitaxel) ratio or to adjust the concentration of rapamycin and/or a taxane (*e.g*., paclitaxel) in the dispersion. For example, human serum albumin solution (*e.g*., 25 % w/v) can be added to adjust the human serum albumin to rapamycin ratio to about any of 18:1, 15,:1 14:1, 13:1, 12:1, 11:1, 10:1, 9:1, 8:1, 7.5:1, 7:1, 6:1, 5:1, 4:1, or 3:1. For example, human serum albumin solution (*e.g.*, 25 % w/v) can be added to adjust the human serum albumin to a taxane (*e.g*., paclitaxel) ratio to about any of 18:1, 15,:1 14:1, 13:1, 12:1, 11:1, 10:1, 9:1, 8:1, 7.5:1, 7:1, 6:1, 5:1, 4:1, or 3:1. For example, human serum albumin solution (*e.g*., 25 % w/v) or another solution is added to adjust the concentration of rapamycin in the dispersion to about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml. For example, human serum albumin solution (*e.g*., 25 % w/v) or another solution is added to adjust the concentration of a taxane (e.g., paclitaxel) in the dispersion to about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml. The dispersion may be serially filtered through multiple filters, such as a combination of 1.2 µm and 0.8/0.2 µm filters; the combination of 1.2 µm, 0.8 µm, 0.45 µm, and 0.22 µm filters; or the combination of any other filters known in the art. The dispersion obtained can be further lyophilized. The nanoparticle compositions may be made using a batch process or a continuous process (*e.g*., the production of a composition on a large scale).

If desired, a second therapy (*e.g*., one or more compounds useful for treating pulmonary hypertension), an antimicrobial agent, sugar, and/or stabilizing agent can also be included in the composition. This additional agent can either be admixed with the rapamycin and/or the carrier protein during preparation of the rapamycin/carrier protein composition, or added after the rapamycin/carrier protein composition is prepared. For example, the agent can be added along with an aqueous medium used to reconstitute/suspend the rapamycin/carrier protein composition or added to an aqueous suspension of the carrier protein-associated rapamycin. In some variations, the agent is admixed with the rapamycin/carrier protein composition prior to lyophilization. This additional agent can either be admixed with the taxane (*e.g*., paclitaxel) and/or the carrier protein during preparation of the taxane (*e.g*., paclitaxel)/carrier protein composition, or added after the taxane (*e.g*., paclitaxel)/carrier protein composition is prepared. For example, the agent can be added along with an aqueous medium used to reconstitute/suspend the taxane (*e.g*., paclitaxel)/carrier protein composition or added to an aqueous suspension of the carrier protein-associated rapamycin. In some variations, the agent is admixed with the taxane (*e.g*., paclitaxel)/carrier protein composition prior to lyophilization. In some variations, the agent is added to the lyophilized pharmaceutical agent/carrier protein composition. In some variations when the addition of the agent changes the pH of the composition, the pH in the composition are generally (but not necessarily) adjusted to a desired pH. Exemplary pH values of the compositions include, for example, in the range of about 5 to about 8.5. In some variations, the pH of the composition is adjusted to no less than about 6, including for example no less than any of about 6.5, 7, or 8 (*e.g*., about 8).

Unless defined otherwise, the meanings of all technical and scientific terms used herein are those commonly understood by one of skill in the art to which this invention belongs. One of skill in the art will also appreciate that any methods and materials similar or equivalent to those described herein can also be used to practice or test the invention.

The following Examples are provided to illustrate, but not limit, the invention.

### EXAMPLES

The examples, which are intended to be purely exemplary of the invention and should therefore not be considered to limit the invention in any way, also describe and detail aspects and variations of the invention discussed above. The examples are not intended to represent that the experiments below are all or the only experiments performed. Efforts have been made to ensure accuracy with respect to numbers used (for example, amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1: Exemplary Methods for the Formation of Nanoparticle Compositions with Rapamycin and Albumin.

### Example 1-A

This example demonstrates the preparation of a pharmaceutical composition comprising rapamycin and albumin in which the rapamycin concentration was 8 mg/mL in the emulsion and the formulation was made on a 300 mL scale. Rapamycin (2400 mg) was dissolved in 12 mL of chloroform/t-butanol. The solution was then added into 288 mL of a human serum albumin solution (1-8% w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was removed at 40 °C at reduced pressure (10-200 mm of Hg). The dispersion was filtered through multiple filters. The size of the filtered formulation was 85-100 nm (Zₐᵥ, Malvern Zetasizer). The dispersion was further lyophilized (FTS Systems, Dura-Dry µP, Stone Ridge, New York) for 60 hours. The resulting cake was easily reconstitutable to the original dispersion by the addition of sterile water or 0.9 % (w/v) sterile saline. The particle size after reconstitution was the same as before lyophilization.

### Example 1-B

This example demonstrates the preparation of a pharmaceutical composition comprising rapamycin and albumin in which the rapamycin concentration was 8.3 mg/mL in the emulsion and the formulation was made on a 200 mL scale. Rapamycin (1660 mg) was dissolved in 8.5 mL of chloroform/ethanol. The solution was then added into 191.5 mL of a human serum albumin solution (6% w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (25 mm of Hg). The dispersion was serially filtered. The size of the 0.22 µm filtered formulation was 85 nm (Zₐᵥ, Malvern Zetasizer). The dispersion was further lyophilized (FTS Systems, Dura-Dry µP, Stone Ridge, New York) for 60 hours. The resulting cake was easily reconstitutable to the original dispersion by addition of 0.9 % (w/v) sterile saline. The particle size after reconstitution was the same as before lyophilization.

### Example 1-C

This example demonstrates the preparation of a pharmaceutical composition comprising rapamycin and albumin in which the rapamycin concentration was 16.2 mg/mL in the emulsion and the formulation was made on a 200 mL scale. Rapamycin (3240 mg) was dissolved in 16 mL of chloroform/ethanol. The solution was then added into 184 mL of a human serum albumin solution (6% w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (25 mm of Hg). At this stage, human serum albumin solution was added to the dispersion and the volume of the dispersion was made to 400 mL to adjust the human serum albumin to rapamycin ratio and to adjust the rapamycin concentration. The dispersion was serially filtered. The size of the 0.22 µm filtered formulation was 99 nm (Zₐᵥ, Malvern Zetasizer). The dispersion was further lyophilized (FTS Systems, Dura-Dry µP, Stone Ridge, New York) for 60 hours. The resulting cake was easily reconstitutable to the original dispersion by addition of 0.9 % (w/v) sterile saline. The particle size after reconstitution was the same as before lyophilization.

### Example 1-D

This example demonstrates the preparation of a pharmaceutical composition comprising rapamycin and albumin in which the rapamycin concentration was 8.2 mg/mL in the emulsion and the formulation was made on a 40 mL scale. Rapamycin (328 mg) was dissolved in 1.8 mL of chloroform/ethanol. The solution was then added into 38.2 mL of a human serum albumin solution (6% w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 108 nm (Zₐᵥ, Malvern Zetasizer). The liquid suspension was found to be stable at 4 °C and 25 °C at least for 48 hours.

### Example 1-E

This example demonstrates the preparation of a pharmaceutical composition comprising rapamycin and albumin in which the rapamycin concentration was 8.5 mg/mL in the emulsion and the formulation was made on a 30 mL scale. Rapamycin (255 mg) was dissolved in 1.35 mL of chloroform/ethanol. The solution was then added into 28.7 mL of a human serum albumin solution (6% w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 136 nm (Zₐᵥ, Malvern Zetasizer). The liquid suspension was found to be stable at 4 °C and 25 °C at least for 24 hours.

### Example 1-F

This example demonstrates the preparation of a pharmaceutical composition comprising rapamycin and albumin in which the rapamycin concentration was 9.2 mg/mL in the emulsion and the formulation was made on a 20 mL scale. Rapamycin (184 mg) was dissolved in 1.0 mL of chloroform/ethanol. The solution was then added into 19.0 mL of a human serum albumin solution (7 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 124 nm (Zₐᵥ, Malvern Zetasizer). The liquid suspension was found to be stable at 4 °C and 25 °C at least for 24 hours.

### Example 1-G

This example demonstrates the preparation of a pharmaceutical composition comprising rapamycin and albumin in which the rapamycin concentration was 8.4 mg/mL in the emulsion and the formulation was made on a 20 mL scale. Rapamycin (168 mg) was dissolved in 1.2 mL of chloroform/ethanol. The solution was then added into 18.8 mL of a human serum albumin solution (6 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 95 nm (Zₐᵥ, Malvern Zetasizer).

### Example 1-H

This example demonstrates the preparation of a pharmaceutical composition comprising rapamycin and albumin in which the rapamycin concentration was 8.2 mg/mL in the emulsion and the formulation was made on a 20 mL scale. Rapamycin (164 mg) was dissolved in 0.9 mL of chloroform/ethanol. The solution was then added into 19.1 mL of a human serum albumin solution (8 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 149 nm (Zₐᵥ, Malvern Zetasizer).

### Example 1-I

This example demonstrates the preparation of a pharmaceutical composition comprising rapamycin and albumin in which the rapamycin concentration was 6.6 mg/mL in the emulsion and the formulation was made on a 20 mL scale. Rapamycin (132 mg) was dissolved in 0.8 mL of chloroform/ethanol. The solution was then added into 19.2 mL of a human serum albumin solution (5 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 129 nm (Zₐᵥ, Malvern Zetasizer).

### Example 1-J

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 108 nm (Zₐᵥ, Malvern Zetasizer).

### Example 1-K

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (1 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 99 nm (Zₐᵥ, Malvern Zetasizer).

### Example 1-L

This example demonstrates the preparation of a pharmaceutical composition comprising rapamycin and albumin in which the rapamycin concentration was 5.0 mg/mL in the emulsion and the formulation was made on a 20 mL scale. Rapamycin (100 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 146 nm (Zₐᵥ, Malvern Zetasizer).

### Example 1-M

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The resulting dispersion was a white milky suspension. The dispersion was serially filtered. The size of the 0.22 µm filtered formulation was 129 nm (Zₐᵥ, Malvern Zetasizer).

### Example 1-N

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 166 nm (Zₐᵥ, Malvern Zetasizer).

### Example 1-O

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 90 nm (Zₐᵥ, Malvern Zetasizer).

### Example 1-P

This example demonstrates the preparation of a pharmaceutical composition comprising rapamycin and albumin in which the rapamycin concentration was 4.0 mg/mL in the emulsion and the formulation was made on a 20 mL scale. Rapamycin (80 mg) was dissolved in 0.8 mL of chloroform/ethanol. The solution was then added into 19.2 mL of a human serum albumin solution (3 % w/v). The mixture was homogenized for 5 minutes at 10,000 rpm (Vitris homogenizer model Tempest I.Q.) in order to form a crude emulsion, and then transferred into a high pressure homogenizer. The emulsification was performed at 20,000 psi while recycling the emulsion. The resulting system was transferred into a Rotavap, and the solvent was rapidly removed at 40 °C at reduced pressure (40 mm of Hg). The dispersion was serially filtered. The size of the 0.22 µm filtered formulation was 81 nm (Zₐᵥ, Malvern Zetasizer).

### Example 1-Q

This example demonstrates the preparation of a pharmaceutical composition comprising rapamycin and albumin. Rapamycin (30 mg) was dissolved in 2 ml chloroform/ethanol. The solution was then added into 27.0 ml of a human serum albumin solution (3% w/v). The mixture was homogenized for 5 minutes at low RPM (Vitris homogenizer model Tempest I.Q.) in order to form a crude emulsion, and then transferred into a high pressure homogenizer. The emulsification was performed at 9000-40,000 psi while recycling the emulsion for at least 5 cycles. The resulting system was transferred into a Rotavap, and the solvent was rapidly removed at 40 °C at reduced pressure (30 mm Hg) for 20-30 minutes. The resulting dispersion was translucent, and the typical average diameter of the resulting particles was in the range 50-220 nm (Z-average, Malvern Zetasizer). The dispersion was further lyophilized for 48 hours. The resulting cake was easily reconstituted to the original dispersion by addition of sterile water or saline. The particle size after reconstitution was the same as before lyophilization.

If desired, other compositions of the invention (e.g., compositions that contain rapamycin derivatives or carrier proteins other than human serum albumin) can be made using these methods or a variation of these methods. It should be recognized that the amounts, types, and proportions of drug, solvents, and proteins used in these examples are not limiting in any way.

### Example 2: Exemplary Methods for the Formation of Nanoparticle Compositions with Paclitaxel and Albumin.

This example provides formulations of paclitaxel/albumin. The compositions were prepared essentially as described in U.S. Pat. No. 5,439,686 and 5,916,596. Briefly, paclitaxel was dissolved in an organic solvent (such as methylene chloride or a chloroform/ethanol mixture), and the solution was added to a human serum albumin solution. The mixture was homogenized for 5 minutes at low RPM to form a crude emulsion, and then transferred into a high pressure homogenizer. The emulsification was performed at 9000 - 40,000 psi while recycling the emulsion for at least 5 cycles. The resulting system was transferred into a rotary evaporator, and the organic solvent was rapidly removed at 40 °C, at reduced pressure (30 mm Hg) for 20-30 minutes. The dispersion was then further lyophilized for 48 hours. The resulting cake was reconstituted to the original dispersion by addition of sterile water or saline, which may optionally contain additional antimicrobial agent(s).

### Example 3: Toxicology and Pharmacokinetic Studies of Nab-rapamycin

The overall toxicity of *Nab*-rapamycin was determined in a dose ranging study in Sprague Dawley rats. The dose levels of *Nab*-rapamycin used were 0, 15, 30, 45, 90 and 180 mg/kg with a q4dx3 schedule. The pharmacokinetics of *Nab*-rapamycin was also investigated in Sprague Dawley rats at dose levels of 1 (N=3), 15 (N=4), 30 (N=3), and 45 mg/kg (N=4). Blood samples were collected prior to dosing (baseline) and post-dosing at the following time points: 1, 5, 10, 15, 30 and 45 minutes, and 1, 4, 8, 24, 36 and 48 hours. Plasma samples were analyzed for rapamycin using LC/MS.

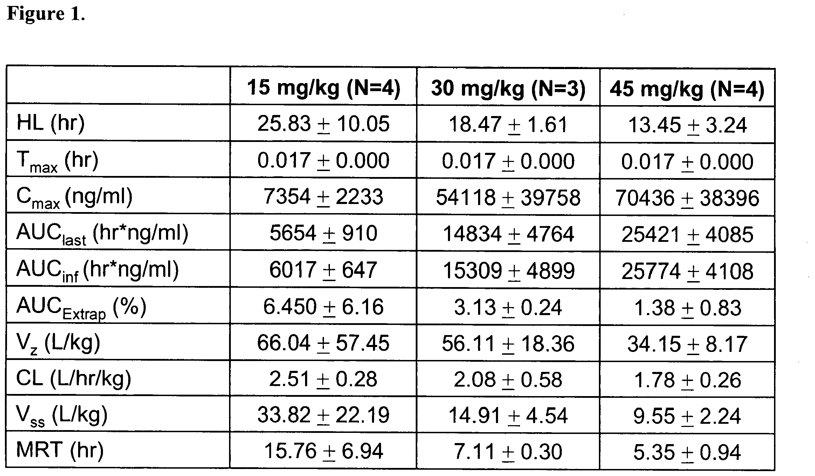

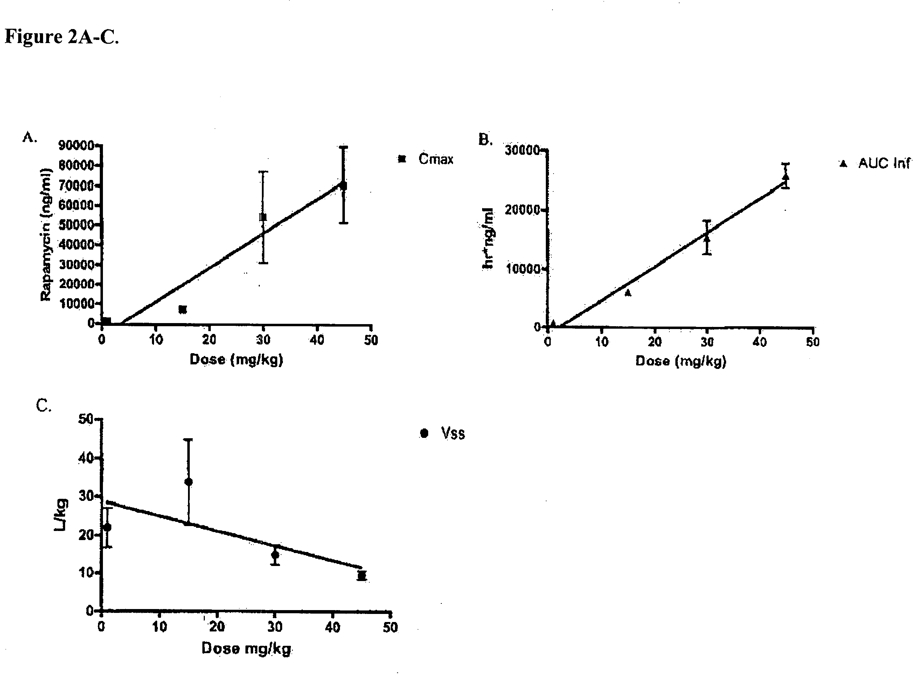

*Nab*-rapamycin was nontoxic at the highest dose of 180 mg/kg on a q4dx3 schedule. No changes in blood chemistry or CBC were observed. No hypercholesterolemia and hypertriglyceridemia were observed. As illustrated in Figures 1 and 2C, *Nab*-rapamycin exhibited linear pharmacokinetics with respect to dose and rapid extravascular distribution as demonstrated by large Vss and Vz. The Cmax and AUCinf of *Nab*-rapamycin were dose proportional (Figures 2A and 2B, respectively).

If desired, other compositions of the invention (e.g., compositions that contain rapamycin derivatives or carrier proteins other than human serum albumin) can be tested in these assays for toxicity and pharmacokinetics.

### Example 4: Pharmacokinetic Studies of Nab-paclitaxel

This example demonstrates preclinical pharmacokinetics and pharmacodynamics of a pharmaceutical composition comprising *Nab*-paclitaxel.

Several preclinical pharmacokinetic studies in mice and rats were conducted to evaluate the possible advantages of albumin-paclitaxel pharmaceutical compositions over cremophor-paclitaxel (Taxol) pharmaceutical compositions. These studies demonstrated: (1) that the pharmacokinetics of *Nab*-paclitaxel in rats was linear, whereas Taxol pharmacokinetics were non-linear with respect to dose, (2) pharmaceutical compositions comprising *Nab*-paclitaxel exhibited a lower plasma AUC and Cₘₐₓ, suggesting more rapid distribution of *Nab*-paclitaxel compositions to tissues compared with Taxol (excretion is similar), (3) pharmaceutical compositions comprising *Nab*-paclitaxel exhibited a lower Cₘₐₓ, which possibly accounts for the reduced toxicities associated with peak blood levels relative to Taxol, (4) the half-life of pharmaceutical compositions comprising *Nab*-paclitaxel exhibited was approximately 2-fold higher in rats and 4-fold higher in tumor bearing mice relative to Taxol, and (5) the metabolism of paclitaxel in pharmaceutical compositions comprising albumin and paclitaxel was slower than in Taxol pharmaceutical compositions. At 24 hours post-injection in rats, 44% of total radioactivity was still associated with paclitaxel for pharmaceutical compositions comprising albumin and paclitaxel, compared to only 22% for Taxol. The ultimate effect of the above pharmacodynamics, i.e., enhanced intra-cellular uptake, prolonged half-life and slower metabolism for pharmaceutical compositions comprising albumin and paclitaxel exhibited resulted in a tumor AUC 1.7-fold higher, tumor Cₘₐₓ 1.2-fold higher, and tumor half-life 1.7-fold longer than for Taxol in tumor bearing mice.

### Example 5: Pilot Study Response to Nab-rapamycin, Nab-paclitaxel, and the combination of Nab-rapamycin and Nab-paclitaxel in Pulmonary Hypertension Model

In rats, monocrotaline, a pyrrolizidine alkaloid, causes pulmonary vascular damage leading to pulmonary hypertension, right ventricular hypertrophy, and eventually heart failure (Hayashi 1967, Ghodse 1981, Meyrick 1980). Nine-week-old male Sprague-Dawley rats (N=40) weighing 325-425 grams receive a single subcutaneous injection of 60 mg/kg monocrotaline and are maintained in a normoxic environment. Monocrotaline (Sigma, St. Louis, MO) is dissolved in 1M HCl, neutralized with 0.5 M NaOH, and then diluted with distilled water to pH 7.0. Experimental rats receive a single subcutaneous injection of 60 mg/kg monocrotaline. The injection is given carefully to the gluteal region so that there is no tissue necrosis. All the rats are fed a commercial diet and maintained in a normoxic environment. Hemodynamic studies are performed 30 days post injection. Verification of pulmonary hypertension is done by echocardiographic and direct pulmonary pressure measurements. Post procedure care is provided adequately by principal investigator on daily basis and at more frequent interval if required. Long term post operative care record sheets are maintained until the final surgery and sacrifice of the rat. Veterinarian is consulted if the rats show any signs of pain and distress during the post injection period. Regarding signs of pain and distress certain things are monitored such as whether the rat is eating, dehydrated, lethargic or not. Limited movement, loss of motor responses, lack of sensory responses, muscle tenderness and pain with movement are further monitored. Signs of pulmonary hypertension and heart failure are noted regularly.

**Hemodynamic Evaluation:** Rats are anesthetized with sodium pentobarbital (80 mg/kg subcutaneously SC), ketamine (40 mg/kg, IM), and atropine (0.28 mg/kg, IP) followed by 2000 U heparin IP (Sigma Chemical). Anesthesia is maintained with 2.5-5.0 mg of pentobarbital SC every 30-60 minutes as needed. Blood temperature is maintained at 38-39°C with a heating pad. During surgery, 60% oxygen is delivered via a flow-by mask.

After obtaining stable anesthesia, the left carotid artery is isolated and an incision is made. A polyethylene catheter (PE 50) is inserted to record arterial pressure. Through the right jugular vein, a second catheter (PE 50) is advanced under X-ray guidance into the pulmonary artery to measure pulmonary artery pressure.

Baseline measurements (pressures, ECG, pulmonary flow, TEE) is obtained. Based upon the PA velocity profile, pulmonary artery pressure is estimated for the echo measurements. After baseline measurements, the rats receive *Nab*-rapamycin, *Nab*-paclitaxel, and the combination of *Nab*-rapamycin and *Nab*-paclitaxel to test the does-response range. The following increasing doses are used: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab*-paclitaxel (*e.g.,* between about 0.5-30 mg/kg, such as about 1, 2.5, 5, 7.5 or 10 mg/kg of *Nab*-rapamycin and/or *Nab*-paclitaxel). At each dose, data is recorded after 15-20 min stabilization.

At the end of experiments all animals are euthanized by cardiotomy under deep anesthesia (pentobarbital 5mg/kg, I.P). The hearts and lungs are removed and the left and right ventricular weights will be determined and pathological changes are verified to confirm RV hypertrophy.

### Example 6: Response to Nab-rapamycin, Nab-paclitaxel, and the combination of Nab-rapamycin and Nab-paclitaxel in Primary Chronic Pulmonary Hypertension Model

In rats, monocrotaline, a pyrrolizidine alkaloid, causes pulmonary vascular damage leading to pulmonary hypertension, right ventricular hypertrophy, and eventually heart failure (Hayashi 1967, Ghodse 1981, Meyrick 1980). Nine-week-old male Sprague-Dawley rats (N=40) weighing 325-425 g receive a single subcutaneous injection of 60 mg/kg monocrotaline and are maintained in a normoxic environment. Monocrotaline (Sigma, St. Louis, MO) is dissolved in 1M HCl, neutralized with 0.5 M NaOH, and then diluted with distilled water to pH 7.0. Experimental rats receive a single subcutaneous injection of 60 mg/kg monocrotaline. The injection is given carefully to the gluteal region so that there is no tissue necrosis. All the rats are fed a commercial diet and maintained in a normoxic environment. Hemodynamic studies are performed 30 days post injection. Verification of pulmonary hypertension is done by echocardiographic and direct pulmonary pressure measurements. Post procedure care is provided adequately by principal investigator on daily basis and at more frequent interval if required. Long term post operative care record sheets are maintained until the final surgery and sacrifice of the rat. Veterinarian is consulted if the rats show any signs of pain and distress during the post injection period. Regarding signs of pain and distress certain things are monitored such as whether the rat is eating, dehydrated, lethargic or not. Limited movement, loss of motor responses, lack of sensory responses, muscle tenderness and pain with movement are further monitored. Signs of pulmonary hypertension and heart failure are noted regularly.

At 31 to 34 days, daily evaluations will be preformed with a terminal evaluation on day 35. The hemodynamic evaluation and physiological responses to *Nab*-rapamycin, *Nab-*paclitaxel, and the combination of *Nab*-rapamycin and *Nab*-paclitaxel are compared to a control (*e.g*., saline, water, DMSO).

**Daily Evaluation:** Rats are anesthetized with sodium pentobarbital (80 mg/kg subcutaneously SC), ketamine (40 mg/kg, IM), and atropine (0.28 mg/kg, IP) followed by 2000 U heparin IP (Sigma Chemical). Anesthesia is maintained with 2.5-5.0 mg of pentobarbital SC every 30-60 minutes as needed. Blood temperature is maintained at 38-39°C with a heating pad. During surgery, 60% oxygen is delivered via a flow-by mask.

After obtaining stable anesthesia, a catheter (PE 90) is inserted into a vein. Baseline TEE is obtained. After baseline measurements, the rats are randomly be assigned to a control group or to receive either *Nab*-rapamycin, *Nab*-paclitaxel, or the combination of *Nab-*rapamycin and *Nab*-paclitaxel (N=10/group). After the initial randomization, the rats are daily observed. The following increasing doses are used: *Nab*-rapamycin (*e.g*., between about 0.5-30 mg/kg, such as about 1, 2.5, 5, 7.5 or 10 mg/kg), *Nab*-paclitaxel (*e.g.*, between about 0.5-30 mg/kg, such as about 1, 2.5, 5, 7.5 or 10 mg/kg), and *Nab*-rapamycin and *Nab*-paclitaxel (*e.g.,* between about 0.5-30 mg/kg, such as about 1, 2.5, 5, 7.5 or 10 mg/kg of *Nab*-rapamycin or *Nab-*paclitaxel) administered daily and weekly. At each vasodilator dose, data is recorded after 15-20 min stabilization. In the control group, data is obtained at equivalent time points. At the end of experiments all animals are allowed to recover and will be returned to its cage.

**Terminal Evaluation:** Rats are anesthetized with sodium pentobarbital (80 mg/kg subcutaneously SC), ketamine (40 mg/kg, IM), and atropine (0.28 mg/kg, IP) followed by 2000 U heparin IP (Sigma Chemical). Anesthesia is maintained with 2.5-5.0 mg of pentobarbital SC every 30-60 minutes as needed. Blood temperature is maintained at 38-39°C with a heating pad. During surgery, 60% oxygen is delivered via a flow-by mask.

After obtaining stable anesthesia, the left carotid artery is isolated and an incision made, and a polyethylene catheter (PE 50) is inserted to record arterial pressure. Through the right jugular vein, a second catheter (PE 50) is advanced under X-ray guidance into the pulmonary artery to measure pulmonary artery pressure.

Baseline measurements (pressures, ECG, pulmonary flow, TEE) are obtained. After baseline measurements, the rats receive no drugs (control group) or either *Nab*-rapamycin, *Nab*-paclitaxel, or the combination of *Nab*-rapamycin and *Nab*-paclitaxel (as previously assigned). The following increasing doses are used: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab*-paclitaxel (*e.g.,* between about 0.5-30 mg/kg, such as about 1, 2.5, 5, 7.5 or 10 mg/kg of *Nab*-rapamycin and/or *Nab*-paclitaxel) daily. At each vasodilator dose, data is recorded after 15-20 min stabilization. In the control group, data is obtained at equivalent time points.

At the end of experiments all animals are euthanized by cardiotomy under deep anesthesia (pentobarbital 5mg/kg, I.P). The hearts are removed and the left and right ventricular weights are determined and pathological changes are verified to confirm RV hypertrophy. The effects of each vasodilator on systemic and pulmonary pressures and pulmonary artery flow is calculated. Data are recorded as mean + SD. Data are analyzed by using an analysis of variance, followed by a Newmann-Keuls' teat when appropriate. Statistical significance is set at p<0.05.

### Example 7: Response to Nab-rapamycin, Nab-paclitaxel, and the combination of Nab-rapamycin and Nab-paclitaxel in Pulmonary Hypertension Model

**Study Sample:** Forty pathogen-free, 13-wk-old, male Sprague-Dawley rats (body weight, 350-400 g) are studied.

**Left Pneumonectomy:** On Day 0, rats are anesthetized with atropine sulfate (50 µg, intramuscular), ketamine hydrochloride (10 mg, intramuscular), and xylazine (3 mg, subcutaneous). After oral endotracheal intubation (with a 14-gauge catheter; Baxter, Deerfield, IL), anesthesia is maintained with halothane inhalation (0.5%) and rats are ventilated with a Harvard rodent ventilator (tidal volume, 3.0 ml; respiratory rate, 60/min; positive end-expiratory pressure [PEEP], 1 cm H2O) (Type 683; Harvard Apparatus, South Natick, MA). Left pneumonectomy is performed via left thoracotomy, using aseptic technique.

**Monocrotaline Administration:** On Day 7, rats are injected subcutaneously in the right hindlimb with monocrotaline (MCT, 60 mg/kg; Sigma, St. Louis, MO) (MCT is dissolved in distilled water, adjusted to pH 7.40 with 0.5 N HCl).

**Treatment Groups:** Rats are randomized to receive either *Nab*-rapamycin, *Nab-*paclitaxel, or the combination of *Nab*-rapamycin and *Nab*-paclitaxel, or vehicle by intravenous administration. Eight groups are studied: Group 1 receive *Nab*-rapamycin (*e.g*., between about 0.5-30 mg/kg, such as about 1, 2.5, 5, 7.5 or 10 mg/kg) from Day 15 to Day 35 (n = 4), Group 2 (early treatment group) receive *Nab*-rapamycin (*e.g*., between about 0.5-30 mg/kg, such as about 1, 2.5, 5, 7.5 or 10 mg/kg) from Day 5 to Day 14 (n = 4), Group 3 receive *Nab*-paclitaxel *(e.g.,* between about 0.5-30 mg/kg, such as about 1, 2.5, 5, 7.5 or 10 mg/kg) from Day 15 to Day 35 (n = 4), Group 4 (early treatment group) receive *Nab*-paclitaxel (*e.g*., between about 0.5-30 mg/kg, such as about 1,2.5,5,7.5 or 10 mg/kg) from Day 5 to Day 14 (n = 4), Group 5 receive *Nab-*rapamycin and *Nab*-paclitaxel (*e.g*., between about 0.5-30 mg/kg, such as about 1, 2.5, 5, 7.5 or 10 mg/kg) from Day 15 to Day 35 (n = 4), Group 6 (early treatment group) receive *Nab-*rapamycin and *Nab*-paclitaxel (*e.g*., between about 0.5-30 mg/kg, such as about 1, 2.5, 5, 7.5 or 10 mg/kg) from Day 5 to Day 14 (n = 4), Group 7 receive placebo (e.g., saline, water, DMSO) from Day 15 to Day 35 (n = 4), and Group 8 (early treatment group) receive placebo (e.g., saline, water, DMSO) from Day 5 to Day 14 (n = 4).

All animals receive humane care in compliance with the Principles of Laboratory Animal Care formulated by the National Society for Medical Research, and the Guide for the Care and Use of Laboratory Animals prepared by the National Academy of Science and published by the National Institutes of Health (NIH Publication No. 86-23, revised 1985).

**Hemodynamic Studies and Tissue Preparation:** On Day 35, rats are anesthetized by intramuscular injections of atropine sulfate (50 µg, intramuscular) and ketamine hydrochloride (10 mg, intramuscular) and are placed in the supine position. Anesthesia is maintained with inhalation halothane (0.5%, by hood). A carotid arterial catheter (PE-50, 0.58-mm i.d.) is placed after cutdown. A pulmonary artery catheter (PV 1, 0.28-mm i.d.) is inserted into the right internal jugular vein through an introducer. The pulmonary artery catheter is passed (under pressure wave guidance) through the right ventricle into the pulmonary artery. Right ventricular systolic pressure measurements are also obtained by percutaneous needle (27 gauge) puncture of the right ventricle. Mean arterial blood pressure, pulmonary artery blood pressure, and right ventricular systolic blood pressure are recorded. After exsanguination, the right lung, right ventricle, left ventricle plus septum, liver, spleen, kidney, testis, and thymus are collected for histology. Tissues are fixed in 10% neutral buffered formalin. The lungs are axially sectioned, processed, and embedded in paraffin wax. Five-micron sections are prepared and stained with elastin-van Gieson (EVG). The severity of neointimal formation is scored. Briefly, the absence of neointimal formation equals 0; the presence of neointimal proliferation causing less than 50% lumenal narrowing equals 1; lumenal narrowing greater than 50% equals 2. To facilitate comparisons across groups of rats, organ weights are presented per kilogram of body weight.

**Statistical Analysis:** Data are presented as means ± standard deviation. First, the data from normal rats are compared with data for Group PMV (the disease model), using the Student t test (statistical significance is indicated by p < 0.05). Next, Groups PMV, PMR5-35, PMR5-14, and PMR15-35 are analyzed by two-way analysis of variance (ANOVA) and multiple comparisons in order to determine the effects of early and late therapy. A value of p < 0.05 is considered significant.

### Example 8: Response to Nab-rapamycin, Nab-paclitaxel, and the combination of Nab-rapamycin and Nab-paclitaxel in Pulmonary Hypertension Model

Forty pathogen-free, 13-wk-old, male Sprague-Dawley rats (body weight, 350-400 g) are studied. The rats are randomly be assigned to a control group or to receive either *Nab-*rapamycin, *Nab*-paclitaxel, or the combination of *Nab*-rapamycin and *Nab*-paclitaxel (N=10/group). Monocrotaline, 100 mg/kg, is given by subcutaneous injection. The following doses are used: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab*-paclitaxel (*e.g*., between about 0.5-30 mg/kg, such as about 1, 2.5, 5, 7.5 or 10 mg/kg of *Nab*-rapamycin and/or *Nab*-paclitaxel) administered daily by gavage starting 1 day prior to monocrotaline injection.

After 28 days, hemodynamic measurements are made and animals are killed. Rats are anesthetized with isoflurane/oxygen, intubated orotracheally, and ventilated with room air/isoflurane. The heart is exposed through a midline chest incision, and a Millar catheter is inserted through the right ventricle into the pulmonary artery. Pulmonary artery pressure (PaP) is monitored and data are collected using PowerLab system and software (ADInstruments, Colorado Springs, CO, USA). The lungs are then flushed until clear with ice-cold saline at 20 cm H2O. The right lung and heart is removed and the right ventricle (RV) separated from the left ventricle (LV) and septum (S), and is weighed. The right lung is frozen in liquid nitrogen and stored at -80°C. The left lung is filled with 10% buffered formalin, removed, fixed overnight, and paraffin embedded. Sections (5 µm) are cut at two levels in the paraffin block and are stained with a standard hematoxylin-eosin stain, and additional staining is performed using Verhoeff-van Gieson stains to highlight elastic lamina. Neointimal formation is determined by computerized morphometry and is represented as lumen size to total vessel size, calculated by area within the internal elastic lamina/area within external elastic lamina 100 using image analysis software (Zeiss Axioplan 2 with SPOT digital camera and image analysis, Diagnostic Instruments Inc., Sterling Heights, MI, USA).

### Example 9: Prophylactic Treatment to Prevent Development of Pulmonary Hypertension Induced by Chronic Hypoxia

C57BL/6 mice are obtained and used at 6-8 weeks of age. The animals are fed standard mouse chow and are allowed to take food and water ad libidum. All experiments conform to the NIH guidelines to the care and use of experimental animals.

The mice are randomly be assigned to a control group or to receive either *Nab-*rapamycin, *Nab*-paclitaxel, or the combination of *Nab*-rapamycin and *Nab*-paclitaxel (N=10/group). The mice are exposed to CH (10% O₂) in a ventilation chamber as previously described in Adnot et al., (1999) J. Clin. Invest. 87:155-162 for between 14 and 56 days. The following doses are administered at the same time that the mice are exposed to chronic hypoxia and are developing pulmonary hypertension: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab*-paclitaxel (*e.g.,* between about 0.5-30 mg/kg, such as about 1, 2.5, 5, 7.5 or 10 mg/kg of *Nab*-rapamycin and/or *Nab*-paclitaxel) administered daily or weekly either intraperitoneally or intravenously.

At the end of experiments (4-12 weeks), all animals are euthanized by cardiotomy under deep anesthesia (pentobarbital 5mg/kg, I.P). The hearts are removed and the left and right ventricular weights are determined and pathological changes are verified to confirm RV hypertrophy. The effects of each vasodilator on systemic and pulmonary pressures and pulmonary artery flow are calculated. Data are recorded as mean + SD. Data are analyzed by using an analysis of variance, followed by a Newmann-Keuls' teat when appropriate. Statistical significance is set at p<0.05.

After, the right lung, right ventricle, left ventricle plus septum, liver, spleen, kidney, testis, and thymus are collected for histology. Tissues are fixed in 10% neutral buffered formalin. The lungs are axially sectioned, processed, and embedded in paraffin wax. Five-micron sections are prepared and stained with elastin-van Gieson (EVG). The severity of neointimal formation is scored. Briefly, the absence of neointimal formation equals 0; the presence of neointimal proliferation causing less than 50% lumenal narrowing equals 1; lumenal narrowing greater than 50% equals 2.

### Example 10: Prophylactic Treatment to Prevent Development of Pulmonary Hypertension Induced by MCT

C57BL/6 mice are obtained and used at 6-8 weeks of age. The animals are fed standard mouse chow and are allowed to take food and water ad libidum. All experiments conform to the NIH guidelines to the care and use of experimental animals.

The mice are randomly be assigned to a control group or to receive either *Nab-*rapamycin, *Nab*-paclitaxel, or the combination of *Nab*-rapamycin and *Nab*-paclitaxel (N=10/group). Monocrotaline (MCT, 60 mg/kg; Sigma, St. Louis, MO) (MCT is dissolved in distilled water, adjusted to pH 7.40 with 0.5 N HCl) is given weekly either subcutaneously or intraperitoneally for between 2 and 8 weeks. The following doses are administered at the same time that the mice begin receiving MCT and are developing pulmonary hypertension: *Nab-*rapamycin *(e.g.,* between about 0.5-30 mg/kg, such as about 1, 2.5, 5, 7.5 or 10 mg/kg), *Nab-*paclitaxel (*e.g.,* between about 0.5-30 mg/kg, such as about 1, 2.5, 5, 7.5 or 10 mg/kg), and *Nab-*rapamycin and *Nab*-paclitaxel (*e.g.*, between about 0.5-30 mg/kg, such as about 1, 2.5, 5, 7.5 or 10 mg/kg of *Nab*-rapamycin and/or *Nab*-paclitaxel) administered daily or weekly either intraperitoneally or intravenously.

At the end of experiments (4-12 weeks), all animals are euthanized by cardiotomy under deep anesthesia (pentobarbital 5mg/kg, I.P). The hearts are removed and the left and right ventricular weights are determined and pathological changes are verified to confirm RV hypertrophy. The effects of each vasodilator on systemic and pulmonary pressures and pulmonary artery flow are calculated. Data are recorded as mean + SD. Data are analyzed by using an analysis of variance, followed by a Newmann-Keuls' teat when appropriate. Statistical significance is set at p<0.05.

After, the right lung, right ventricle, left ventricle plus septum, liver, spleen, kidney, testis, and thymus are collected for histology. Tissues are fixed in 10% neutral buffered formalin. The lungs are axially sectioned, processed, and embedded in paraffin wax. Five-micron sections are prepared and stained with elastin-van Gieson (EVG). The severity of neointimal formation is scored. Briefly, the absence of neointimal formation equals 0; the presence of neointimal proliferation causing less than 50% lumenal narrowing equals 1; lumenal narrowing greater than 50% equals 2.

### Example 11: Reversal of Pulmonary Hypertension Induced by Chronic Hypoxia

C57BL/6 mice are obtained and used at 6-8 weeks of age. The animals are fed standard mouse chow and are allowed to take food and water ad libidum. All experiments conform to the NIH guidelines to the care and use of experimental animals.

The mice are randomly be assigned to a control group or to receive either *Nab-*rapamycin, *Nab*-paclitaxel, or the combination of *Nab*-rapamycin and *Nab*-paclitaxel (N=10/group). The mice are exposed to CH (10% O₂)) in a ventilation chamber as previously described in Adnot et al., (1999) J. Clin. Invest. 87:155-162 for between 14 and 56 days. The following doses are administered starting between 2 and 8 weeks after the mice are first exposed to chronic hypoxia conditions: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab*-paclitaxel (*e.g*., between about 0.5-30 mg/kg, such as about 1,2.5,5,7.5 or 10 mg/kg of *Nab*-rapamycin and/or *Nab*-paclitaxel) administered daily or weekly either intraperitoneally or intravenously.

At the end of experiments (4-12 weeks), all animals are euthanized by cardiotomy under deep anesthesia (pentobarbital 5mg/kg, I.P). The hearts are removed and the left and right ventricular weights are determined and pathological changes are verified to confirm RV hypertrophy. The effects of each vasodilator on systemic and pulmonary pressures and pulmonary artery flow are calculated. Data are recorded as mean + SD. Data are analyzed by using an analysis of variance, followed by a Newmann-Keuls' teat when appropriate. Statistical significance is set at p<0.05.

After, the right lung, right ventricle, left ventricle plus septum, liver, spleen, kidney, testis, and thymus are collected for histology. Tissues are fixed in 10% neutral buffered formalin. The lungs are axially sectioned, processed, and embedded in paraffin wax. Five-micron sections are prepared and stained with elastin-van Gieson (EVG). The severity of neointimal formation is scored. Briefly, the absence of neointimal formation equals 0; the presence of neointimal proliferation causing less than 50% lumenal narrowing equals 1; lumenal narrowing greater than 50% equals 2.

### Example 12: Reversal of Pulmonary Hypertension Induced by MCT

C57BL/6 mice are obtained and used at 6-8 weeks of age. The animals are fed standard mouse chow and are allowed to take food and water ad libidum. All experiments conform to the NIH guidelines to the care and use of experimental animals.

The mice are randomly be assigned to a control group or to receive either *Nab-*rapamycin, *Nab*-paclitaxel, or the combination of *Nab*-rapamycin and *Nab*-paclitaxel (N=10/group). Monocrotaline (MCT, 60 mg/kg; Sigma, St. Louis, MO) (MCT is dissolved in distilled water, adjusted to pH 7.40 with 0.5 N HCl) is given weekly either subcutaneously or intraperitoneally for between 2 and 8 weeks. The following doses are administered starting between 2 and 8 weeks after the mice first begin receiving MCT: *Nab*-rapamycin (*e.g*., between about 0.5-30 mg/kg, such as about 1, 2.5, 5, 7.5 or 10 mg/kg), *Nab*-paclitaxel (*e.g*., between about 0.5-30 mg/kg, such as about 1, 2.5, 5, 7.5 or 10 mg/kg), and *Nab*-rapamycin and *Nab-*paclitaxel (*e.g.,* between about 0.5-30 mg/kg, such as about 1, 2.5, 5, 7.5 or 10 mg/kg *of Nab-*rapamycin and/or *Nab*-paclitaxel) administered daily or weekly either intraperitoneally or intravenously.

At the end of experiments (4-12 weeks), all animals are euthanized by cardiotomy under deep anesthesia (pentobarbital 5mg/kg, I.P). The hearts are removed and the left and right ventricular weights are determined and pathological changes are verified to confirm RV hypertrophy. The effects of each vasodilator on systemic and pulmonary pressures and pulmonary artery flow are calculated. Data are recorded as mean + SD. Data are analyzed by using an analysis of variance, followed by a Newmann-Keuls' teat when appropriate. Statistical significance is set at p<0.05.

After, the right lung, right ventricle, left ventricle plus septum, liver, spleen, kidney, testis, and thymus are collected for histology. Tissues are fixed in 10% neutral buffered formalin. The lungs are axially sectioned, processed, and embedded in paraffin wax. Five-micron sections are prepared and stained with elastin-van Gieson (EVG). The severity of neointimal formation is scored. Briefly, the absence of neointimal formation equals 0; the presence of neointimal proliferation causing less than 50% lumenal narrowing equals 1; lumenal narrowing greater than 50% equals 2.

### Example 13: Clinical Use of Nab-rapamycin and Nab-paclitaxel for treatment of Severe Pulmonary Arterial Hypertension

In this study, *Nab*-rapamycin and *Nab*-paclitaxel are explored as a treatment for patients with severe PAH. 20-50 patients with severe PAH despite best available therapy are enrolled into the clinical trial. Target patients are those with severe PAH (NYHA class III or IV) despite best available therapy including prostacyclin, tracleer and/or sildenafil.

Patients are treated with *Nab*-rapamycin at a dose of 10-200 mg/m² (for example 10, 30, 50, 70, 100, 130, 160, or 200 mg/m²) given once per week for 3-16 weeks by IV infusion over 30 minutes. *Nab*-paclitaxel (Abraxane) is given at a dose of 10-100 mg/m² (for example 10, 30, 50, 70, or 100 mg/m²) by IV infusion over 30 minutes once per week for 3-16 weeks. A one week break is allowed after every 3 doses of *Nab*-rapamycin and *Nab*-paclitaxel. Dosing is escalated according to a standard 5x5 rule. If no Dose Limiting Toxicity (DLT) occurs after 5 patients are enrolled at Dose 1 and treated at Dose 1, then 5 patients additional patients are enrolled at next dose level and so on. When a patient in a group of 5 patients experiences a DLT, 5 additional patients will be enrolled at the same level, if no more than 2/10 DLT occurs, the next dose cohort will be opened. The dose of patients who experience a DLT is reduced to next lower dose. Dose Limiting Toxicity (DLT) is considered to be a Grade 3 or 4 non-hematologic toxicity or a Grade 4 hematologic toxicity.

Other than evaluating the safety profile of the drugs, the functional objectives of this study is to assess the efficacy of antiproliferative therapy with *Nab*-rapamycin and *Nab-*paclitaxel by Hemodynamic Monitoring (Pulmonary Vascular Resistance (PVR), Cardiac Output (CO), Mean Pulmonary Artery Pressure (mPAP), Pulmonary Capillary Occlusion Pressure (PCOP)), by a 6 Minute Walk Test, by measuring laboratory markers of ventricular dysfunction such as Brain Naturetic Peptide (BNP), and by Magnetic Resonance Imaging (MRI) to assess Right Ventricular Function.

### Example 14: Intrapulmonary Delivery of Nab-paclitaxel

This example demonstrates intrapulmonary delivery of a pharmaceutical composition comprising *Nab*-paclitaxel (ABI-007). The purpose of this study was to determine the time course of [³H]ABI-007 in blood and select tissues following intratracheal instillation to Sprague Dawley rats.

The target volume of the intratracheal dose formulation to be administered to the animals was calculated based on a dose volume of 1.5 mL per kg body weight. The dosing apparatus consisted of a Penn-Century microsprayer (Model 1A-1B; Penn-Century, Inc., Philadelphia, Pa.; purchased from DeLong Distributors, Long Branch, N.J.) attached to a 1-mL gas-tight, luer-lock syringe. The appropriate volume of dose preparation was drawn into the dosing apparatus, the filled apparatus was weighed and the weight-recorded. A catheter was placed in the trachea of the anesthetized animal, the microsprayer portion of the dosing apparatus was placed into the trachea through the catheter, and the dose was administered. After dose administration the empty dosing apparatus was reweighed and the administered dose was calculated as the difference in the weights of the dosing apparatus before and after dosing. The average dose for all animals was 4.7738+/-0.0060 (CV 1.5059) mg paclitaxel per kg body weight.

Blood samples of approximately 250 µL were collected from the indwelling jugular cannulas of the rats at the following predetermined post-dosing time points: 1, 5, 10, 15, 30, and 45 minutes (min), and 1, 4, 8, and 24 hours (h). The 24-h blood samples, as well as blood samples collected from animals sacrificed at 10 min, 45 min, and 2 h, were collected via cardiac puncture from anesthetized rats at sacrifice. All blood samples analyzed for total radioactivity were dispensed into pre-weighed sample tubes, and the sample tubes were reweighed, and the weight of each sample was calculated by subtraction. The blood samples collected from the jugular vein as well as the 250-µL aliquots of blood collected from each animal at sacrifice were assayed for total tritium content.

For all rats, the maximum concentration of tritium in blood was observed at 5 min (0.0833 hr) post dosing. The elimination half-life of tritium, determined over the time interval from 4 h to 24 h, ranged from 19.73 h to 43.02 h. It should be noted that this interval includes only three data points, which may account for the variability in this parameter. The apparent clearance of tritium from blood was on the order of 0.04 L/h. The results of these experiments are set forth below in Table 1.

**Table 1.**

| **Noncompartmental Analysis of Blood Tritium Concentration (mg-eq/L) vs. Time Profiles in Rats After Intratracheal Instillation of [³H]ABI-007** | |
|---|---|
| **Parameter** | **Mean +/- SD** |
| Cₘₐₓ (mg-eq/L) | 1.615 +/- 0.279 |
| Tₘₐₓ (hr) | 0.0833 +/- 0.0 |
| t ½ β (hr) | 33.02 +/- 1.99 |
| AUClast (mg-eq x hr/L) | 7.051 +/- 1.535 |
| CL/F (L/hr) | 0.0442 +/- 0.0070 |
| Fa (Bioavailability) | 1.229 +/- 0.268 |

The mean blood concentration of [³H]ABI-007-derived radioactivity after an intravenous dose to rats was analyzed as a function of time in order to evaluate the bioavailability of tritium derived from an intratracheal dose of [³H]ABI-007.

This analysis resulted in a 24-hour AUC (AUClast) of 6.1354 mg-eq x hr/L. Based on these data, radioactivity derived from the intratracheal dose of [³H]ABI-007 is highly bioavailable. These analyses are based on total radioactivity.

Tritium derived from [³H]ABI-007 is rapidly absorbed after intratracheal instillation. The average absorption and elimination half-lives (k01 half-life and k10 half-life, respectively) for tritium in blood after an intratracheal dose of [³H]ABI-007 (mean +/-SD) were 0.0155+/-0.0058 hr and 4.738+/-0.366 hr, respectively. The average apparent clearance of tritium from blood was 0.1235+/-0.0180 L/hr (see Table 1 above).

Tritium derived from [³H]ABI-007 was absorbed and distributed after intratracheal administration. The time course of tritium in blood was well described by a two-compartment model, with mean absorption and elimination half-lives of 0.0155 and 4.738 hr, respectively. Approximately 28% of the administered dose was recovered in the lung at 10 min after the intratracheal dose. A maximum of less than 1% of the dose was recovered in other tissues, excluding the gastrointestinal tract, at all time points examined.

Based on results from a previously conducted intravenous dose study with [³H]Capxol™, the bioavailability of tritium derived from the intratracheal dose was 1.229+/- 0.268 (mean +/-SD) for the three animals in this dose group. It should be noted, however, that this estimate of bioavailability is based on total radioactivity. Surprisingly, paclitaxel delivered by the pulmonary route using invention compositions with albumin was rapidly bioavailable indicating excellent transport across pulmonary endothelium. Based on these results, pulmonary delivery of *Nab*-paclitaxel may be suitable for the treatment of conditions such as pulmonary hypertension. No toxicity in the animals was noted, which was surprising since pulmonary delivery of cytotoxics is known to cause lung toxicities.

A fair amount of radioactivity was present in the gastrointestinal tract (including contents) at 24 hr post dosing (27% for the intratracheal dose). The presence of tritium in the gastrointestinal tract may be due to biliary excretion or clearance of tritium from the respiratory tract via mucociliary clearance with subsequent swallowing.

### Example 15: Pulmonary Delivery of Nab-paclitaxel

This example demonstrates an investigation of Aerotech II and Pari nebulizers for pulmonary delivery of pharmaceutical compositions comprising *Nab*-paclitaxel.

The study was carried out using the *Nab*-paclitaxel pharmaceutical composition ABI-007 under the following conditions: room temperature (20-23 °C), relative humidity (48-54%), ambient pressure (629 mmHg), nebulizer flowrate (10 L/min for Aerotech II; 7 L/min for Pari), total flowrate (28.3 L/min), nebulizer pressure drop (23 lb/in² for Aerotech II; 32 lb/in² for Pari), run time (15 to 60 seconds), sample volume (1.5 mL), ABI-007 paclitaxel concentration (5, 10, 15 and 20 mg/mL).

Both Aerotech II and Pari nebulizers provided acceptable overall efficiency (30%-60%) when ABI-007 was reconstituted at a concentration range of 5-15 mg/mL. The Pari nebulizer efficiency had higher nebulizer efficiency than the Aerotech II nebulizer. The Pari nebulizer efficiency decreased somewhat as ABI-007 concentration increased. Excellent fine particle fraction was observed (74%-96%). The Aerotech II nebulizer had higher fine particle fraction than the Pari nebulizer. The fine particle fraction was independent of concentration.

The Pari nebulizer delivered 100 mg of paclitaxel in less than 30 minutes using a 15 mg/mL solution of ABI-007. The Aerotech II nebulizer delivered 100 mg of paclitaxel in about 65 min using either a 10 mg/mL or 15 mg/mL solution of ABI-007. Performance stability was tested for both Aerotech II and Pari nebulizers. Aerosol concentration and efficiency of both nebulizers were stable until the drug was exhausted. At 15 mg/mL, the Pari nebulizer consumed the drug at twice the rate of the Aerotech II nebulizer and produced higher aerosol concentrations than that of the Aerotech II nebulizer.

In conclusion, the nanoparticle/albumin formulation of paclitaxel (ABI-007) shows excellent bioavailability in rats when administered by the pulmonary route. There were no overt signs of early toxicity at the administered dose. Pulmonary delivery of *Nab*-paclitaxel (ABI-007) may be achieved using conventional nebulizers to treat diseases such as pulmonary hypertension.

### Example 16: Intrapulmonary Delivery of Nab-rapamycin

This example describes intrapulmonary delivery of a pharmaceutical composition comprising *Nab*-rapamycin. The purpose of this study was to determine the pulmonary absorption of *Nab*-rapamycin in blood following intratracheal instillation to Sprague Dawley rats as compared to intravenous installation.

The target volume of the intratracheal dose formulation that was administered to the animals was calculated based on a dose volume of 1 mL per kg body. The intratracheal dosing apparatus consisted of a Penn-Century microsprayer (Model 1A-1B; Penn-Century, Inc., Philadelphia, Pa.; purchased from DeLong Distributors, Long Branch, N.J.) attached to a 1 mL gas-tight, luer-lock syringe. The appropriate volume of dose preparation was drawn into the dosing apparatus, the filled apparatus was weighed and the weight-recorded. A catheter was placed in the trachea of the anesthetized animal, the microsprayer portion of the dosing apparatus was placed into the trachea through the catheter, and the dose was administered. After dose administration the empty dosing apparatus was reweighed and the administered dose was calculated as the difference in the weights of the dosing apparatus before and after dosing.

250 µL samples were collected from the indwelling jugular cannulas of rats at the following predetermined post-dosing time points: 1, 5, 10, 15, 30, and 45 minutes (min) and 1, 4, 8, and 24 hours (h). All blood samples analyzed were dispensed into pre-weighed sample tubes, and the sample tubes were reweighed, and the weight of each sample was calculated by subtraction. The blood samples collected were assayed for total rapamycin concentration using LC/MS/MS.

Surprisingly, the results showed no significant difference in the blood concentration of rapamycin delivered via pulmonary route versus intravenously. The bioavailability of rapamycin delivered by the pulmonary route using a pharmaceutical composition comprising albumin was calculated to be 109%, indicating excellent transport across pulmonary endothelium. Based on these results, pulmonary delivery of *Nab*-rapamycin may be suitable for the treatment of conditions such as pulmonary hypertension.

### Example 17: Tissue Distribution After Intrapulmonary Delivery of Nab-rapamycin

This example demonstrates tissue distribution of *Nab*-rapamycin after intrapulmonary administration of a pharmaceutical composition comprising *Nab*-rapamycin in prepared in accordance with the present invention. The purpose of this study was to determine the pulmonary absorption of *Nab*-rapamycin in tissue following intratracheal instillation to Sprague Dawley rats as compared to intravenous installation.

The target volume of the intratracheal dose formulation that was administered to the animals was calculated based on a dose volume of 1 mL per kg body. The dosing apparatus consisted of a Penn-Century microsprayer (Model 1A-1B; Penn-Century, Inc., Philadelphia, Pa.; purchased from DeLong Distributors, Long Branch, N.J.) attached to a 1-mL gas-tight, luer-lock syringe. The appropriate volume of dose preparation was drawn into the dosing apparatus, the filled apparatus was weighed and the weight-recorded. A catheter was placed in the trachea of the anesthetized animal, the microsprayer portion of the dosing apparatus was placed into the trachea through the catheter, and the dose was administered. After dose administration the empty dosing apparatus was reweighed and the administered dose was calculated as the difference in the weights of the dosing apparatus before and after dosing.

Samples were collected from the brain, lung, and, liver of three rats per group per time point at 10 minutes, 45 minutes, 2 hours, and 24 hours. The samples were collected and analyzed for total rapamycin concentration using LC/MS/MS. The results indicated that rapamycin concentration is greater in lung tissue when delivered via pulmonary as compared to intravenous delivery. However, the total concentration of rapamycin in the brain was lower when delivered via intratracheal (IT) as compared to intravenous (IV). In the liver, there appears to be no difference in the concentration of rapamycin whether delivered IT or IV. Based on these results, pulmonary delivery of *Nab*-rapamycin may be suitable for the treatment of conditions such as pulmonary hypertension, wherein high local concentration of rapamycin would be beneficial.

### Example 18: Formulation for Inhalation of Nab-rapamycin and Nab-paclitaxel

*Nab*-rapamycin and *Nab*-paclitaxel are prepared using microparticle techniques to yield effective formulations for dry powder inhalers (DPI). Starting with paclitaxel or rapamycin, a dry formulation is prepared of appropriate particle size and release characteristics to ensure efficacious delivery in the respiratory system.

The formulation is prepared using sonication techniques, or homogenization in which the active drug, dissolved in solvent, is dispersed into an aqueous protein solution to form an emulsion of nanoparticles. This emulsion is then evaporated to remove solvents, leaving the active drug coated with protein in solution. This liquid sample containing the colloidal drug particles is measured by Malvern Zetasizer and gives a Z-average size of 260 nm. In a preferred embodiment, the range of sizes of these colloidal particles is about 50-1,000 nm, and more preferably about 70-400 nm.

In this liquid form, other excipients may be dissolved. Such excipients include (but are not limited to) mannitol 0.5-15% lactose 0.1-5%, and maltodextrin. At this stage, the resulting solution of active drug, protein, and excipient can be either spray-dried or lyophilized and milled to yield a dry powder. After spray-drying, the dry particle size is determined by Malvern Mastersizer as D(v.0.5) of about 1-10 µm. The preferred size range for these particles is 0.5-15 µm, with a more preferred range of 0.7-8 µm.

This spray dried powder is then mixed with an excipient carrier powder. Again, several carriers are available, including lactose, trehalose, Pharmatose 325 M, sucrose, mannitol, and the like. The size of the carrier powder is significantly larger than that of the formulated drug particles (∼63-90 µm for lactose, 40-100 µm for Pharmatose).

The efficacy of the dry powder formulation is demonstrated by testing with an Andersen eight-stage cascade impactor. High FPF results would indicate an efficacious and promising approach to aerosol delivery DPI formulations which may be used to treat conditions such as pulmonary hypertension.

### Example 19: Clinical Use of Nab-paclitaxel for treatment of Severe (NYHA III or IV) Pulmonary Arterial Hypertension

Patients with PAH in functional class III or IV on best available therapy have limited therapeutic options. Since implementation of a new organ allocation system, it has become more difficult for functional class IV patients to be transplanted. This study provides a therapeutic that may lead to better treatment options for patients with PAH.

Efficacy of anti-proliferative therapy with *Nab*-paclitaxel is assessed by: (i) hemodynamic parameters including: (a) Cardiac Output (CO), (b) Pulmonary Artery Pressures (PAP), (c) Pulmonary Artery Occlusion Pressure (PAOP), (d) Central Venous Pressure (CVP), and (e) Pulmonary Vascular Resistance (PVR); (ii) Six-Minute Walk Testing (6MWT); (iii) change in NYHA Functional Class; (iv) Magnetic Resonance Imaging (MRI) to assess right ventricular function; (v) laboratory markers of ventricular dysfunction: Brain Natriuretic Peptide (BNP); and (vi) laboratory markers of inflammation: C-Reactive Protein (CRP).

### Overview of Study Design and Method:

Patients with severe PAH despite best available therapy are recruited for participation in the clinical study. Target patients are those with severe PAH (NYHA class III or IV) despite best available therapy. Best available therapy includes a Prostacyclin (unless unwilling or unable to tolerate) and at least one oral agent (*e.g*., an endothelin receptor antagonist and/or phosphodiesterase type 5 inhibitor).

As a part of the initial screening, subjects participate in a physical examination, baseline laboratory testing, pulmonary function studies, chest X-ray, 6MWT, Borg dyspnea scoring (for baseline exercise capacity and assessment of NYHA/WHO functional class), and a cardiac MRI.

Subjects enrolled in the trial also undergo right heart catheterization (RHC) (using standard techniques and while the subject is in steady state) at baseline and after 16 weeks of therapy. RHC is performed following premedication and under local anesthesia with a heparin-bonded, thermodilution, Swan-Ganz catheter inserted percutaneously into the right internal jugular or a femoral vein depending on anatomic landmarks. The catheter is positioned in the pulmonary artery with pressure monitoring. The catheter tip position is evaluated by fluoroscopy or chest radiography. Fluoroscopy is performed according to standard clinical practice. A 20-gauge heparin-bonded cannula inserted percutaneously into a radial, brachial, or femoral artery is used for blood sampling and monitoring of systemic arterial pressure. Mean vascular pressure levels are determined by electronic integration of the pressure signals. Heart rate and vascular pressures are monitored continuously. Cardiac output is measured in triplicate by thermodilution or by the Fick method with measured oxygen consumption. Systemic and pulmonary vascular resistances are calculated using the mean cardiac index and mean vascular pressures.

The Treatment Stage of the trial is designed to test the safety, dosing, and potential efficacy of *Nab*-paclitaxel in patients with severe PAH. Anti-proliferative therapy includes *Nab*-paclitaxel (Abraxane) administered by IV infusion over 30 minutes once per week for 3 weeks. One cycle consists of 3 doses of Abraxane over three weeks and one week of rest. Patients undergo 4 cycles per 16 weeks. The initial group of subjects receives 30 mg/m² of *Nab-*paclitaxel.

Dosing is escalated according to a standard 3x3 rule. 3 patients are enrolled at Dose 1. If no Dose Limiting Toxicity (DLT) occurs, then 3 patients are enrolled at next dose level and so on. If a DLT occurs, then 3 additional patients are enrolled at that level. If no more than 2/6 of the same DLT occur, then the next dose cohort is opened. If an intolerable DLT thought to be secondary to *Nab*-paclitaxel occurs, then the individual patient has dose reduction to next lower dose level. The various dosing groups include 30 mg/m², 60 mg/m², 80 mg/m², and 100 mg/m².

A DLT includes any Grade 2 toxicity with the following exceptions, which are discussed below. It is anticipated patients will have a baseline anemia of chronic disease; therefore, a drop of 2g/dl below their baseline Hgb value is considered a DLT. It is anticipated this patient population would have baseline edema of varying severity requiring therapy related to right heart failure. Therefore, worsening edema resulting in weight gain of greater than 15 pounds despite a doubling of baseline diuretic therapy and not related to progression of underlying disease in the opinion of the investigator is considered a DLT. It is anticipated that this patient population may have baseline ascites requiring diuretic therapy related to right heart failure. Therefore, worsening ascites resulting in greater than 15 pound weight gain above baseline not responsive to doubling of baseline diuretic therapy or requiring multiple therapeutic paracentesis and not related to progression of underlying disease is considered DLT. It is anticipated that some patients receiving Prostacyclin therapy for PAH would have baseline diarrhea as a side effect of the medication. Therefore, an increase of 4-6 stools above the patient's baseline stools/day that is sustained for more than 10 days is considered a DLT. It is anticipated that this patient population may have baseline elevations in liver function tests (*e.g.,* ALT/AST, Bilirubin, and/or Alkaline Phosphatase) related to hepatic congestion from right heart failure. Therefore, a doubling of LFT values above baseline that is confirmed at 24 hour and 48 hour time points and not related to progression of underlying disease in the opinion of the investigator is considered a DLT. It is anticipated that this patient population would have severe dyspnea at baseline related to disease. In fact, this is part of what defines the patient population studied. Therefore, worsening dyspnea that worsens NYHA Class or requires ventilator support and not related to progression of underlying disease in the opinion of the investigator is considered a DLT. It is anticipated that this patient population would have hypoxia requiring oxygen therapy at rest as part of the disease process; therefore, increasing oxygen requirements of >2 lpm above baseline or need for initiating positive pressure ventilation is considered a DLT.

The risk of nausea and vomiting is intermediate (10-20%) with the use of *Nab-*paclitaxel based on the NIH Clinical Center Pharmacy Department antiemetic Guidelines 2003. Prophylaxis may be provided including administration of 8 mg of Ondansetron 30 minutes prior to drug infusion and q12 hours x 2 doses after infusion. Lorazepam or Promethazine may also be used as needed for breakthrough nausea/vomiting.

Subjects at weekly intervals undergo clinical testing and procedures including recording vital signs, neurotoxicity monitoring with West-Hand Esthesiometer and score sheet, adverse event monitoring, assessment of concomitant medications, standard chemistries, and venous blood drawn for CBC and, as necessary, to monitor coagulation profile if patient is on coumadin therapy.

Subjects at monthly intervals (at end of each drug cycle) undergo clinical testing and procedures including physical exam, recording vitals signs, assessment of NYHA/WHO functional class, venous blood drawn for coagulation panel, lipid panel, NT-pro BNP and CRP, 6MWT (with assessment of Borg dyspnea index), and echocardiogram with assessment of TRV LV and RV function.

Subjects at week 16 undergo clinical testing and procedures including pulmonary function tests, cardiac MRI, and right heart catheterization.

### Clinical and Laboratory Methods

Right Heart Catheterization: The pulmonary artery catheter is a triple lumen central venous catheter with two lumens devoted to the pressure transduction functions. The distal port transduces pressure from the pulmonary artery and from the left atrium, when in the "wedge" position. The central venous port transduces right atrial pressures. During catheter placement, right ventricular pressures are transduced. Warmed coils on the catheter and a thermister at the catheter tip allows for continuous, thermal dilution, cardiac output measurements, which complement mixed venous blood saturation for cardiac output calculation by the Fick method. Pulmonary artery systolic and diastolic pressures, right atrial pressure, pulmonary capillary wedge pressure, systemic artery systolic and diastolic pressures, cardiac output, and calculated systemic and pulmonary vascular resistances are obtained using this methodology. Vasoreactivity testing which involves recording the hemodynamic variables obtained in the right heart catheterization after the infusion of a vasodilating substance (*e.g.*, adenosine, inhaled NO, or flolan) are performed with each catheterization. These measurements are performed at baseline, and at the completion of the 16 weeks of *Nab*-paclitaxel therapy.

6MWT: The 6MWT is performed in accordance with standard practice. Briefly, patients are asked to walk with a trained exercise technician for six minutes along a premeasured path. A practice walk is performed first. Distance walked, Borg dyspnea index, NYHA functional class, and oxygen saturation are determined.

MRI: MRI of the cardiac muscle enhancement during first passage of intravenously injected gadolinium contrast is used to evaluate regional cardiac muscle perfusion. This is done by using T1-weighted MRI. The muscle is imaged on each heartbeat as the first pass of gadolinium contrast traverses the muscles vascular bed. Perfusion is related to signal intensity changes with this imaging technique. Patients are monitored in the scanner by two-way microphone contact, continuous ECG, intermittent blood pressure determination, and visually by a video monitor. Myocardial volumes are obtained at rest for determination of resting right and left ventricular ejection fraction. Right and left ventricular mass are measured. This redundancy in assessment of contractility with the determination made in the echocardiogram lab is important, because not all patients will have suitable echocardiogram windows and not all patients will be eligible for or tolerate (due to claustrophobia) MRI studies. The MRI also provides estimates of myocardial mass and quantitative right ventricular measurements not attainable by 2-D echocardiography. As with currently approved cardiac MRI procedures, the gradients are operated at slew rates that do not result in skeletal muscle stimulation, and the radiofrequency deposition is at or below FDA limits. Patients are asked to report sensations of muscle twitches or warmth during the scan. These measurements are performed at baseline and at the completion of therapy with *Nab*-paclitaxel.

MRI Perfusion and Delayed Enhancement with Gadolinium Contrast: Approximately 10-20 minutes after the administration of gadolinium, delayed enhancement images are obtained. In patients with coronary artery disease, these images are used to detect myocardial infarction at high resolution.

N-Terminal pro Brain Natriuretic Peptide (NT-proBNP): Reproducible, noninvasive parameters are useful in following patients with PAH. BNP is produced in the cardiac ventricles and is elevated in PPH/IPAH. BNP levels have recently been shown to be closely related to functional impairment in PPH/IPAH patients and parallel the extent of pulmonary hemodynamic changes and right heart failure. BNP levels longitudinally correlate with the functional assessments being made over the course of the study. Plasma NT-pro-BNP are measured by a sandwich immunoassay using polyclonal antibodies that recognize epitopes located in the N-terminal segment (1 to 76) of pro-BNP (1 to 108) (Elecsys analyzer, Roche Diagnostics, Manheim, Germany).

CRP: CRP is a marker of systemic inflammation associated with an increased risk of incident myocardial infarction, stroke, and systemic hypertension. PAH may be an endothelial-based disease, and inflammation has been shown to correlate with endothelial function.

Intended Use of The Samples/Specimens/Data: Samples and data collected under this protocol are used to develop the safety profile of *Nab*-paclitaxel in patients with severe PAH who are NYHA class III or IV despite best available therapy. They are also used to evaluate dosing regimen of *Nab*-paclitaxel in patients with severe PAH by monitoring hematologic data and symptom profile.

Subjects are included in the study if (1) age > 18yo with PAH, (2) right heart catheterization diagnosis of PAH: Mean Pulmonary Artery Pressure (mPAP) >25mmHg at rest, Pulmonary Capillary Occlusion Pressure (PCOP) or Left Ventricular End Diastolic Pressure (LVEDP) < 15 mmHg, and Pulmonary Vascular Resistance (PVR) > 3 mmHg/L/min, (3) patients must be IPAH, FPAH, or APAH, (4) echocardiographic evidence of Right Ventricular Dysfunction, (5) on standard and stable PAH therapy including: (a) a Prostacyclin (IV epoprostenol, IV or subcutaneous remodulin, inhaled iloprost) unless unwilling or unable to tolerate therapy, (b) phosphodiesterase type 5 inhibitor (sildenafil), (c) endothelin receptor antagonist (Ambrisenten) or (d) any combination of a-c, (6) NYHA class III or IV despite 3 months of stable therapy, (7) 6 Minute Walk Distance < 380m, (8) negative serum pregnancy test, and (9) female of childbearing age either surgically sterilized or using acceptable method of contraception.

Subjects are excluded in the study if (1) history of malignancy in 2 years prior to enrollment, (2) baseline Cytopenia's: (a) white blood cell count < 3,000, (b) hemoglobin < 7 , and (c) platelet < 80,000, (3) baseline liver disease: (a) porto-pulmonary hypertenion (Class 1.3.3) and (b) ALT/AST, Tbili, Alk phos > 5x ULN, (4) baseline renal disease: Cr > 2, (5) inability to attend scheduled clinic visits, (6) prior use of study drug within previous 6 months from enrollment, (7) previous lung transplant, (8) naïve to available standard PAH therapy, (9) pre-existing peripheral neuropathy, (10) concomitant enrollment in another investigational treatment protocol for PAH or taking any off label drug therapy for PAH, and (11) recent enrollment in or plans to enroll in pulmonary rehabilitation during the study period.

A complete set of vital signs (heart rate, blood pressure, respiratory rate, oxygen saturation on room air if>88% and baseline oxygen level, height, and weight), monitoring for adverse events including filament testing for neurotoxicity and assessment of concomitant medication use are taken every week of the trial on the day of drug infusion.

Laboratory monitoring includes a CBC with differential and Chem 20 drawn every week after initiation of study drug to monitor for hematologic, renal and liver adverse effect of drug therapy. At the end of the fourth cycle repeat CBC with differential, Chem 20, coagulation panel, lipid panel BNP and CRP are drawn. For patients on coumadin, coagulation labs are drawn at least once per week for four weeks to monitor for need to titrate dose.

Echocardiogram and 6MWT are performed every four weeks. At the end of the fourth cycle of drug therapy (16 weeks), repeat screening tests are performed including right heart catheterization, echocardiogram, full pulmonary function testing, 6MWT, CXR and cardiopulmonary MRI.

Patient monitoring procedures for Stage I of the *Nab*-paclitaxel are outlined in Table I below:

| TABLE 1: *Nab*-paclitaxel Monitoring | | | | | | | | | |
|---|---|---|---|---|---|---|---|---|---|
| Visit Number: | 1 | 2 | 3 | 4 | 5 | 6-9 | 10-13 | 14-17 | 18 |
| | | Cycle 1* | | | | Cycle 2* | Cycle 3* | Cycle 4* | |
| Time/Date | Screening Baseline | Wk 1 | Wk 2 | Wk 3 | Wk 4 | Wk 5-8 | Wk 9-12 | Wk 13-16 | End Stage I |
| Assessments | | | | | | | | | |
| Consent | X | | | | | | | | |
| History | X | | | | | | | | X |
| PEX | X | | | | X | X | X | X | X |
| Vitals | X | X | X | X | X | XXXX | XXXX | XXXX | X |
| Adverse Events Monitoring | | X | X | X | X | XXXX | XXXX | XXXX | |
| NYHA Functional Class | X | | | | X | X | X | X | X |
| Lab Eval | | | | | | | | | |
| CBC c diff | X | X | X | X | X | XXXX | XXXX | XXXX | X |
| Chem 20 | X | X | X | X | X | XXXX | XXXX | XXXX | X |
| Coags * | X | | | | X | X | X | X | X |
| Lipid panel | X | | | | X | X | X | X | X |
| BNP | X | | | | X | X | X | X | X |
| CRP | X | | | | X | X | X | X | X |
| GFR | X | | | | | | | | X |
| uHcG | X | | | | | | | | |
| Proteomics | X | | | | | X | | X | |
| | | | | | | | | | |
| RHC | X | | | | | | | | X |
| Echo | X | | | | X | X | X | X | X |
| PFTs | X | | | | | | | | X |
| 6 Minute Walk Test | X | | | | X | X | X | X | X |
| CXR | X | | | | | | | | X |
| MRI | X | | | | | | | | X |
| | | | | | | | | | |
| *Nab-*paclitaxel | | X | X | X | | XXX | XXX | XXX | X |

| | | | | | | | | | |
|---|---|---|---|---|---|---|---|---|---|
| * for patients on coumadin coags are drawn at least weekly as needed to titrate coumadin dose | | | | | | | | | |

In accordance with this invention as described herein and the example described above, an individual with pulmonary hypertension is administered nanoparticles comprising paclitaxel and albumin.

### Example 20: Clinical Use of Nab-rapamycin for treatment of Severe (NYHA III or IV) Pulmonary Arterial Hypertension

Patients with PAH in functional class III or IV on best available therapy have limited therapeutic options. Since implementation of a new organ allocation system, it has become more difficult for functional class IV patients to be transplanted. This study provides a therapeutic that may lead to better treatment options for patients with PAH.

Efficacy of anti-proliferative therapy with *Nab*-rapamycin is assessed by: (i) hemodynamic parameters including: (a) Cardiac Output (CO), (b) Pulmonary Artery Pressures (PAP), (c) Pulmonary Artery Occlusion Pressure (PAOP), (d) Central Venous Pressure (CVP), and (e) Pulmonary Vascular Resistance (PVR); (ii) Six-Minute Walk Testing (6MWT); (iii) change in NYHA Functional Class; (iv) Magnetic Resonance Imaging (MRI) to assess right ventricular function; (v) laboratory markers of ventricular dysfunction: Brain Natriuretic Peptide (BNP); and (vi) laboratory markers of inflammation: C-Reactive Protein (CRP).

### Overview of Study Design and Method:

Patients with severe PAH despite best available therapy are recruited for participation in the clinical study. Target patients are those with severe PAH (NYHA class III or IV) despite best available therapy. Best available therapy includes a Prostacyclin (unless unwilling or unable to tolerate) and at least one oral agent (*e.g.,* an endothelin receptor antagonist and/or phosphodiesterase type 5 inhibitor).

As a part of the initial screening, subjects participate in a physical examination, baseline laboratory testing, pulmonary function studies, chest X-ray, 6MWT, Borg dyspnea scoring (for baseline exercise capacity and assessment of NYHA/WHO functional class), and a cardiac MRI.

Subjects enrolled in the trial also undergo right heart catheterization (RHC) (using standard techniques and while the subject is in steady state) at baseline and after 16 weeks of therapy. RHC is performed following premedication and under local anesthesia with a heparin-bonded, thermodilution, Swan-Ganz catheter inserted percutaneously into the right internal jugular or a femoral vein depending on anatomic landmarks. The catheter is positioned in the pulmonary artery with pressure monitoring. The catheter tip position is evaluated by fluoroscopy or chest radiography. Fluoroscopy is performed according to standard clinical practice. A 20-gauge heparin-bonded cannula inserted percutaneously into a radial, brachial, or femoral artery is used for blood sampling and monitoring of systemic arterial pressure. Mean vascular pressure levels are determined by electronic integration of the pressure signals. Heart rate and vascular pressures are monitored continuously. Cardiac output is measured in triplicate by thermodilution or by the Fick method with measured oxygen consumption. Systemic and pulmonary vascular resistances are calculated using the mean cardiac index and mean vascular pressures.

The Treatment Stage of the trial is designed to test the safety, dosing, and potential efficacy of *Nab*-rapamycin in patients with severe PAH. Anti-proliferative therapy includes *Nab*-rapamycin administered by IV infusion over 30 minutes (alternatively, any of 1, 5, 10, 15, or 20 minutes) once per week for 3 weeks. One cycle consists of 3 doses of *Nab-*rapamycin over three weeks and one week of rest. Patients undergo 4 cycles per 16 weeks. The initial group of subjects receives 30 mg/m2 of *Nab*-rapamycin.

Dosing is escalated according to a standard 3x3 rule. 3 patients are enrolled at Dose 1. If no Dose Limiting Toxicity (DLT) occurs, then 3 patients are enrolled at next dose level and so on. If a DLT occurs, then 3 additional patients are enrolled at that level. If no more than 2/6 of the same DLT occur, then the next dose cohort is opened. If an intolerable DLT thought to be secondary to *Nab*-rapamycin occurs, then the individual patient has dose reduction to next lower dose level. The various dosing groups include dosages ranging from 30-250 mg/m² such as 30 mg/m², 60 mg/m², 80 mg/m², and 100 mg/m².

A DLT includes any Grade 2 toxicity with the exceptions described as in the previous example.

Prophylaxis may be provided including administration of 8 mg of Ondansetron 30 minutes prior to drug infusion and q12 hours x 2 doses after infusion. Lorazepam or Promethazine may also be used as needed for breakthrough nausea/vomiting.

Subjects at weekly or at other appropriate intervals undergo clinical testing and procedures including recording vital signs, neurotoxicity monitoring with West-Hand Esthesiometer and score sheet, adverse event monitoring, assessment of concomitant medications, standard chemistries, and venous blood drawn for CBC and, as necessary, to monitor coagulation profile if patient is on coumadin therapy.

Subjects at monthly or at other appropriate intervals (at end of each drug cycle) undergo clinical testing and procedures including physical exam, recording vitals signs, assessment of NYHA/WHO functional class, venous blood drawn for coagulation panel, lipid panel, NT-pro BNP and CRP, 6MWT (with assessment of Borg dyspnea index), and echocardiogram with assessment of TRV LV and RV function.

Subjects at week 16 or at other appropriate times for assessment undergo clinical testing and procedures including pulmonary function tests, cardiac MRI, and right heart catheterization.

### Clinical and Laboratory Methods

Right Heart Catheterization, 6MWT, MRI, NT-proBNP, and CRP are as described in the previous example.

Intended Use of The Samples/Specimens/Data: Samples and data collected under this protocol are used to develop the safety profile of *Nab*-rapamycin in patients with severe PAH who are NYHA class III or IV despite best available therapy. They are also used to evaluate dosing regimen of *Nab*-rapamycin in patients with severe PAH by monitoring hematologic data and symptom profile.

Subjects are included in the study if (1) age > 18yo with PAH, (2) right heart catheterization diagnosis of PAH: Mean Pulmonary Artery Pressure (mPAP)>25mmHg at rest, Pulmonary Capillary Occlusion Pressure (PCOP) or Left Ventricular End Diastolic Pressure (LVEDP) < 15 mmHg, and Pulmonary Vascular Resistance (PVR) > 3 mmHg/L/min, (3) patients must be IPAH, FPAH, or APAH, (4) echocardiographic evidence of Right Ventricular Dysfunction, (5) on standard and stable PAH therapy including: (a) a Prostacyclin (IV epoprostenol, IV or subcutaneous remodulin, inhaled iloprost) unless unwilling or unable to tolerate therapy, (b) phosphodiesterase type 5 inhibitor (sildenafil), (c) endothelin receptor antagonist (Ambrisenten) or (d) any combination of a-c, (6) NYHA class III or IV despite 3 months of stable therapy, (7) 6 Minute Walk Distance < 380m, (8) negative serum pregnancy test, and (9) female of childbearing age either surgically sterilized or using acceptable method of contraception. Inclusion criteria may vary from the above-described criteria (*i.e*., one or more criteria) as appropriate.

Subjects are excluded in the study if (1) history of malignancy in 2 years prior to enrollment, (2) baseline Cytopenia's: (a) white blood cell count < 3,000, (b) hemoglobin < 7 , and (c) platelet < 80,000, (3) baseline liver disease: (a) porto-pulmonary hypertenion (Class 1.3.3) and (b) ALT/AST, Tbili, Alk phos > 5x ULN, (4) baseline renal disease: Cr > 2, (5) inability to attend scheduled clinic visits, (6) prior use of study drug within previous 6 months from enrollment, (7) previous lung transplant, (8) naive to available standard PAH therapy, (9) pre-existing peripheral neuropathy, (10) concomitant enrollment in another investigational treatment protocol for PAH or taking any off label drug therapy for PAH, and (11) recent enrollment in or plans to enroll in pulmonary rehabilitation during the study period. Exclusion criteria may vary from the above-described criteria (*i.e.,* one or more criteria) as appropriate.

A complete set of vital signs (heart rate, blood pressure, respiratory rate, oxygen saturation on room air if>88% and baseline oxygen level, height, and weight), monitoring for adverse events including filament testing for neurotoxicity and assessment of concomitant medication use are taken every week of the trial on the day of drug infusion.

Laboratory monitoring include a CBC with differential and Chem 20 drawn every week after initiation of study drug to monitor for hematologic, renal and liver adverse effect of drug therapy. At the end of the fourth cycle repeat CBC with differential, Chem 20, coagulation panel, lipid panel BNP and CRP are drawn. For patients on coumadin, coagulation labs are drawn at least once per week for four weeks to monitor for need to titrate dose.

Echocardiogram and 6MWT are generally performed every four weeks. At the end of the fourth cycle of drug therapy (16 weeks or other period as appropriate), repeat screening tests are performed including right heart catheterization, echocardiogram, full pulmonary function testing, 6MWT, CXR and cardiopulmonary MRI.

Patient monitoring procedures for Stage I of the *Nab*-rapamycin are outlined in Table I below:

| TABLE 1: *Nab*-rapamycin Monitoring | | | | | | | | | |
|---|---|---|---|---|---|---|---|---|---|
| Visit Number: | 1 | 2 | 3 | 4 | 5 | 6-9 | 10-13 | 14-17 | 18 |
| | | | Cycle 1* | | | Cycle 2* | Cycle 3* | Cycle 4* | |
| Time/Date | Screening Baseline | Wk 1 | Wk 2 | Wk 3 | Wk 4 | Wk 5-8 | Wk 9-12 | Wk 13-16 | End Stage I |
| Assessments | | | | | | | | | |
| Consent | X | | | | | | | | |
| History | X | | | | | | | | X |
| PEX | X | | | | X | X | X | X | X |
| Vitals | X | X | X | X | X | XXXX | XXXX | XXXX | X |
| Adverse Events Monitoring | | X | X | X | X | XXXX | XXXX | XXXX | |
| NYHA Functional Class | X | | | | X | X | X | X | X |
| Lab Eval | | | | | | | | | |
| CBC c diff | X | X | X | X | X | XXXX | XXXX | XXXX | X |
| Chem 20 | X | X | X | X | X | XXXX | XXXX | XXXX | X |
| Coags * | X | | | | X | X | X | X | X |
| Lipid panel | X | | | | X | X | X | X | X |
| BNP | X | | | | X | X | X | X | X |
| CRP | X | | | | X | X | X | X | X |
| GFR | X | | | | | | | | X |
| uHcG | X | | | | | | | | |
| Proteomics | X | | | | | X | | X | |
| Testing | | | | | | | | | |
| RHC | X | | | | | | | | X |
| Echo | X | | | | X | X | X | X | X |
| PFTs | X | | | | | | | | X |
| 6 Minute Walk Test | X | | | | X | X | X | X | X |
| CXR | X | | | | | | | | X |
| MRI | X | | | | | | | | X |
| Intervention | | | | | | | | | |
| *Nab-*rapamycin | | X | X | X | | XXX | XXX | XXX | X |

| | | | | | | | | | |
|---|---|---|---|---|---|---|---|---|---|
| * for patients on coumadin coags are drawn at least weekly as needed to titrate coumadin dose | | | | | | | | | |

In accordance with this invention as described herein and the example described above, an individual with pulmonary hypertension is administered nanoparticles comprising rapamycin and albumin.

In accordance with this invention as described herein and the two previous examples, an individual with pulmonary hypertension is administered (a) nanoparticles comprising paclitaxel and albumin, and (b) nanoparticles comprising rapamycin and albumin.

The application discloses inter alia following items:
1. A method of treating pulmonary hypertension in an individual, comprising administering to the individual an effective amount of a composition comprising nanoparticles that comprise rapamycin or a derivative thereof and a carrier protein.
2. The method of item 1, wherein the pulmonary hypertension is pulmonary arterial hypertension.
3. The method of item 2, wherein the pulmonary arterial hypertension is idiopathic pulmonary arterial hypertension.
4. The method of item 1, wherein one or more symptoms of the pulmonary hypertension are ameliorated.
5. The method of item 1, wherein the pulmonary hypertension is delayed.
6. The method of item 1, wherein the amount of the rapamycin or derivative thereof in the effective amount of the composition is in the range of about 5 mg to about 500 mg.
7. The method of item 6, wherein the amount of the rapamycin or derivative thereof in the effective amount of the composition is about 30 mg to about 300 mg.
8. The method of item 1, wherein the rapamycin or derivative thereof is administered parenterally.
9. The method of item 8, wherein the rapamycin or derivative thereof is administered intravenously.
10. The method of item 1, wherein the rapamycin or derivative thereof is administered by inhalation.
11. The method of item 1, wherein the rapamycin or derivative thereof is rapamycin.
12. The method of item 1, wherein the rapamycin or derivative thereof is the only pharmaceutically active agent useful for treating pulmonary hypertension that is administered to the individual.
13. The method of item 1, wherein the composition comprises more than about 50% of the rapamycin or derivative thereof in nanoparticle form.
14. The method of item 1, wherein the carrier protein is albumin.
15. The method of item 14, wherein the albumin is human serum albumin.
16. The method of item 1, wherein the average diameter of the nanoparticles in the composition is no greater than about 200 nm.
17. The method of item 1, wherein the weight ratio of the carrier protein to the rapamycin or derivative thereof in the nanoparticles is less than about 18:1.
18. The method of item 1, wherein the individual is human.
19. A unit dosage form for treatment of pulmonary hypertension comprising (a) nanoparticles that comprise a carrier protein and rapamycin or a derivative thereof, wherein the amount of the rapamycin or derivative thereof in the unit dosage form is in the range of about 5 mg to about 500 mg, and (b) a pharmaceutically acceptable carrier.
20. The unit dosage form of item 19, wherein the amount of the rapamycin or derivative thereof in the unit dosage form is about 30 mg to about 300 mg.
21. The unit dosage form of item 19, wherein the carrier is suitable for parenteral administration.
22. The unit dosage form of item 21, wherein the carrier is suitable for intravenous administration.
23. The unit dosage form of item 19, wherein the carrier is suitable for administration by inhalation.
24. The unit dosage form of item 19, wherein the rapamycin or derivative thereof is rapamycin.
25. The unit dosage form of item 19, wherein the rapamycin or derivative thereof is the only pharmaceutically active agent useful for treating pulmonary hypertension that is contained in the unit dosage form.
26. The unit dosage form of item 19, wherein the composition comprises more than about 50% of the rapamycin or derivative thereof in nanoparticle form.
27. The unit dosage form of item 19, wherein the carrier protein is albumin.
28. The unit dosage form of item 27, wherein the albumin is human serum albumin.
29. The unit dosage form of item 19, wherein the average diameter of the nanoparticles in the composition is no greater than about 200 nm.
30. The unit dosage form of item 19, wherein the weight ratio of the carrier protein to the rapamycin or derivative thereof in the nanoparticles is less than about 18:1.
31. A kit comprising (a) nanoparticles that comprise a carrier protein and rapamycin or a derivative thereof, wherein the amount of the rapamycin or derivative thereof in the kit is in the range of about 5 mg to about 500 mg, and (b) instructions for using the kit in treating pulmonary hypertension.
32. The kit of item 31, wherein the amount of the rapamycin or derivative thereof in the kits is about 30 mg to about 300 mg.
33. The kit of item 31, wherein the carrier is suitable for parenteral administration.
34. The kit of item 33, wherein the carrier is suitable for intravenous administration.
35. The kit of item 31, wherein the carrier is suitable for administration by inhalation.
36. The kit of item 31, wherein the rapamycin or derivative thereof is rapamycin.
37. The kit of item 31, wherein the rapamycin or derivative thereof is the only pharmaceutically active agent useful for treating pulmonary hypertension that is contained in the kit.
38. The kit of item 31, wherein the composition comprises more than about 50% of the rapamycin or derivative thereof in nanoparticle form.
39. The kit of item 31, wherein the carrier protein is albumin.
40. The kit of item 39, wherein the albumin is human serum albumin.
41. The kit of item 31, wherein the average diameter of the nanoparticles in the composition is no greater than about 200 nm.
42. The kit of item 31, wherein the weight ratio of the carrier protein to the rapamycin or derivative thereof in the nanoparticles is less than about 18:1.
43. A method of treating pulmonary hypertension in an individual, comprising administering to the individual an effective amount of a composition comprising nanoparticles that comprise a taxane or a derivative thereof and a carrier protein.
44. The method of item 43, wherein the pulmonary hypertension is pulmonary arterial hypertension.
45. The method of item 44, wherein the pulmonary arterial hypertension is idiopathic pulmonary arterial hypertension.
46. The method of item 43, wherein one or more symptoms of the pulmonary hypertension are ameliorated.
47. The method of item 43, wherein the pulmonary hypertension is delayed.
48. The method of item 43, wherein the amount of the taxane or derivative thereof in the effective amount of the composition is in the range of about 5 mg to about 500 mg.
49. The method of item 48, wherein the amount of the taxane or derivative thereof in the effective amount of the composition is about 30 mg to about 300 mg.
50. The method of item 43, wherein the taxane or derivative thereof is administered parenterally.
51. The method of item 50, wherein the taxane or derivative thereof is administered intravenously.
52. The method of item 43, wherein the taxane or derivative thereof is administered by inhalation.
53. The method of item 43, wherein the taxane or derivative thereof is paclitaxel.
54. The method of item 43, wherein the taxane or derivative thereof is the only pharmaceutically active agent useful for treating pulmonary hypertension that is administered to the individual.
55. The method of item 43, wherein the composition comprises more than about 50% of the taxane or derivative thereof in nanoparticle form.
56. The method of item 43, wherein the carrier protein is albumin.
57. The method of item 56, wherein the albumin is human serum albumin.
58. The method of item 43, wherein the average diameter of the nanoparticles in the composition is no greater than about 200 nm.
59. The method of item 43, wherein the weight ratio of the carrier protein to the taxane or derivative thereof in the nanoparticles is less than about 18:1.
60. The method of item 43, wherein the individual is human.
61. A unit dosage form for treatment of pulmonary hypertension comprising (a) nanoparticles that comprise a carrier protein and a taxane or a derivative thereof, wherein the amount of the taxane or derivative thereof in the unit dosage form is in the range of about 5 mg to about 500 mg, and (b) a pharmaceutically acceptable carrier.
62. The unit dosage form of item 61, wherein the amount of the taxane or derivative thereof in the unit dosage form is about 30 mg to about 300 mg.
63. The unit dosage form of item 61, wherein the carrier is suitable for parenteral administration.
64. The unit dosage form of item 63, wherein the carrier is suitable for intravenous administration.
65. The unit dosage form of item 61, wherein the carrier is suitable for administration by inhalation.
66. The unit dosage form of item 61, wherein the taxane or derivative thereof is paclitaxel.
67. The unit dosage form of item 61, wherein the taxane or derivative thereof is the only pharmaceutically active agent useful for treating pulmonary hypertension that is contained in the unit dosage form.
68. The unit dosage form of item 61, wherein the composition comprises more than about 50% of the taxane or derivative thereof in nanoparticle form.
69. The unit dosage form of item 61, wherein the carrier protein is albumin.
70. The unit dosage form of item 69, wherein the albumin is human serum albumin.
71. The unit dosage form of item 61, wherein the average diameter of the nanoparticles in the composition is no greater than about 200 nm.
72. The unit dosage form of item 61, wherein the weight ratio of the carrier protein to the taxane or derivative thereof in the nanoparticles is less than about 18:1.
73. A kit comprising (a) nanoparticles that comprise a carrier protein and a taxane or a derivative thereof, wherein the amount of the taxane or derivative thereof in the kit is in the range of about 5 mg to about 500 mg, and (b) instructions for using the kit in treating pulmonary hypertension.
74. The kit of item 73, wherein the amount of the taxane or derivative thereof in the kits is about 30 mg to about 300 mg.
75. The kit of item 73, wherein the carrier is suitable for parenteral administration.
76. The kit of item 75, wherein the carrier is suitable for intravenous administration.
77. The kit of item 73, wherein the carrier is suitable for administration by inhalation.
78. The kit of item 73, wherein the taxane or derivative thereof is paclitaxel.
79. The kit of item 73, wherein the taxane or derivative thereof is the only pharmaceutically active agent useful for treating pulmonary hypertension that is contained in the kit.
80. The kit of item 73, wherein the composition comprises more than about 50% of the taxane or derivative thereof in nanoparticle form.
81. The kit of item 73, wherein the carrier protein is albumin.
82. The kit of item 81, wherein the albumin is human serum albumin.
83. The kit of item 73, wherein the average diameter of the nanoparticles in the composition is no greater than about 200 nm.
84. The kit of item 73, wherein the weight ratio of the carrier protein to the paclitaxel or derivative thereof in the nanoparticles is less than about 18:1.
85. A method of treating pulmonary hypertension in an individual, comprising administering to the individual an effective amount of a composition comprising nanoparticles that comprise rapamycin or a derivative thereof and a carrier protein and a composition comprising nanoparticles that comprise a taxane or a derivative thereof and a carrier protein.
86. The method of item 85, wherein the pulmonary hypertension is pulmonary arterial hypertension.
87. The method of item 86, wherein the pulmonary arterial hypertension is idiopathic pulmonary arterial hypertension.
88. The method of item 85, wherein one or more symptoms of the pulmonary hypertension are ameliorated.
89. The method of item 85, wherein the pulmonary hypertension is delayed.
90. The method of item 85, wherein the amount of the rapamycin or derivative thereof in the effective amount of the composition is in the range of about 5 mg to about 500 mg.
91. The method of item 90, wherein the amount of the rapamycin or derivative thereof in the effective amount of the composition is about 30 mg to about 300 mg.
92. The method of item 85, wherein the amount of the taxane or derivative thereof in the effective amount of the composition is in the range of about 5 mg to about 500 mg.
93. The method of item 92, wherein the amount of the taxane or derivative thereof in the effective amount of the composition is about 30 mg to about 300 mg.
94. The method of item 85, wherein the compositions are administered parenterally.
95. The method of item 94, wherein the compositions are administered intravenously.
96. The method of item 85, wherein the compositions are administered by inhalation.
97. The method of item 85, wherein the rapamycin or derivative thereof is rapamycin and the taxane or derivative thereof is paclitaxel.
98. The method of item 85, wherein the carrier protein is albumin.
99. The method of item 98, wherein the albumin is human serum albumin.
100. The method of item 85, wherein the average diameter of the nanoparticles in the compositions is no greater than about 200 nm.
101. The method of item 85, wherein the individual is human.

## Claims

1. A composition comprising nanoparticles that comprise rapamycin or a derivative thereof and a carrier protein, for use in a method of treating pulmonary hypertension in an individual.

2. The composition for use of claim 1, wherein the pulmonary hypertension is pulmonary arterial hypertension.

3. The composition for use of claim 2, wherein the pulmonary arterial hypertension is idiopathic pulmonary arterial hypertension.

4. The composition for use of any one of claims 1 to 3, wherein the amount of the rapamycin or derivative thereof in an effective amount of the composition is in the range of about 5 mg to about 500 mg.

5. The composition for use of claim 4, wherein the amount of the rapamycin or derivative thereof in the effective amount of the composition is about 30 mg to about 300 mg.

6. The composition for use of any one of claims 1 to 5, wherein the method comprises administering rapamycin or derivative thereof parenterally.

7. The composition for use of claim 6, wherein the method comprises administering rapamycin or derivative thereof intravenously.

8. The composition for use of any one of claims 1 to 5, wherein the method comprises administering rapamycin or derivative thereof by inhalation.

9. The composition for use of any one of claims 1 to 8, wherein the carrier protein is albumin.

10. The composition for use of claim 9, wherein the albumin is human serum albumin.

11. The composition for use of any one of claims 1 to 10, wherein the average diameter of the nanoparticles in the composition is no greater than about 200 nm.

12. The composition for use of any one of claims 1 to 11, wherein the individual is a human.

13. The composition for use of any one of claims 1 to 12, wherein the method further comprises administering a composition comprising nanoparticles that comprise a taxane or a derivative thereof and a carrier protein.

14. A unit dosage form for use in a method of treating pulmonary hypertension, wherein said unit dosage form comprises (a) nanoparticles that comprise an albumin and rapamycin or a derivative thereof, wherein the amount of the rapamycin or derivative thereof in the unit dosage form is in the range of about 5 mg to about 500 mg, and (b) a pharmaceutically acceptable carrier.

15. A unit dosage form for use in a method of treating pulmonary hypertension, wherein said unit dosage form comprises (a) nanoparticles that comprise a carrier protein and a taxane or a derivative thereof, wherein the amount of the taxane or derivative thereof in the unit dosage form is in the range of about 5 mg to about 500 mg, and (b) a pharmaceutically acceptable carrier.
